# EUROPÄISCHE PATENTSCHRIFT

(11) **EP 0 664 811 B1**
(45) Veröffentlichungstag und Bekanntmachung des Hinweises auf die Patenterteilung: **13.08.1997**
(21) Anmeldenummer: 93922949.8
(22) Anmeldetag: 13.10.1993
(51) Int. Cl.: C07J 53/00, A61K 31/56

(54) **GESTAGEN WIRKSAME 19,11-ÜBERBRÜCKTE 4-ESTRENE**
GESTAGENICALLY ACTIVE 19,11-BRIDGED 4-ESTRENES
4-ESTRENES PONTES EN POSITIONS 19,11 A ACTION GESTAGENE

(30) Priorität: 13.10.1992 DE 4235220
(43) Veröffentlichungstag der Anmeldung: 02.08.1995
(73) Patentinhaber: SCHERING AKTIENGESELLSCHAFT, 13342 Berlin (DE)
(72) Erfinder: OTTOW, Eckhard, D-12203 Berlin (DE); SCHWEDE, Wolfgang, D-13467 Berlin (DE); HALFBRODT, Wolfgang, D-13505 Berlin (DE); FRITZEMEIER, Karl-Heinrich, D-13505 Berlin (DE); KRATTENMACHER, Rolf, D-13467 Berlin (DE)
(86) Internationale Anmeldenummer: EP9302823
(87) Internationale Veröffentlichungsnummer: WO9409025

(56) Entgegenhaltungen:
- EP-A- 0 283 428
- DE-A- 3 717 169
- US-A- 3 465 010
- STEROIDS. Bd. 30, Nr. 4 , 1977 , SAN FRANCISCO US Seiten 481 - 510 A. J. V D BROEK ET AL 'Strategy in Drug Research. Synthesis and Study of the Progestational and Ovulation Inhibitory Activity of a Series of 11- -Substituted-1 7-a-Ethynyl-4-Estren-17- -ols'

## Beschreibung

Die vorliegende Erfindung betrifft 19,11-überbrückte 4-Estrene der allgemeinen Formel I 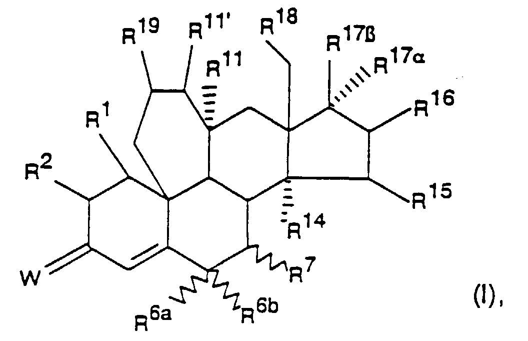 worin
- W: für ein Sauerstoffatom, die Hydroxyiminogruppe >N~OH oder zwei Wasserstoffatome steht,
- R¹ und R²: je für ein Wasserstoffatom oder gemeinsam für eine zusätzliche Bindung oder für eine α-ständige Methylenbrücke stehen,
- R^{6a} und R^{6b}: je für ein Wasserstoffatom oder gemeinsam für eine Methylengruppe oder gemeinsam mit dem Kohlenstoffatom 6 für einen Dreiring stehen, wobei R⁷ in diesen Fällen ein Wasserstoffatom bedeutet oder
- R^{6a}: für ein Wasserstoffatom oder ein Fluor-, Chlor-, Brom- oder Iodatom oder für einen gerad- oder verzweigtkettigen gesättigten α- oder β-ständigen Alkylrest mit bis zu 4 Kohlenstoffatomen, wobei dann R^{6b} und R⁷ je ein Wasserstoffatom oder gemeinsam eine zusätzliche Bindung darstellen, steht, oder
- R^{6b} und R⁷: gemeinsam für eine α- oder β-ständige Methylenbrücke, wobei R^{6a} dann ein Wasserstoffatom ist, stehen,
- R⁷: für einen gerad- oder verzweigtkettigen gesättigten α- oder β-ständigen Alkylrest mit bis zu 4 Kohlenstoffatomen oder für eine Thiogruppe -SR²⁰, worin R²⁰ ein Wasserstoffatom oder eine Alkanoylgruppe mit 1 bis 4 Kohlenstoffatomen darstellt, steht, wobei R^{6a} und R^{6b} dann je ein Wasserstoffatom bedeuten,
- R¹⁴, R¹⁵ und R¹⁶: je für ein Wasserstoffatom oder
- R¹⁴: für ein α-ständiges Wasserstoffatom sowie R¹⁵ und R¹⁶ gemeinsam für eine zusätzliche Bindung oder für eine α- oder β-ständige Methylenbrücke, oder R¹⁴ und R¹⁵ je für ein Wasserstoffatom sowie R¹⁶ für eine α- oder β-ständige C₁-C₄-Alkylgruppe oder R¹⁶ gemeinsam mit R^{17α} für eine α-ständige Methylenbrücke und R^{17β} für eine Gruppe 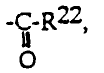
- R¹⁶: für ein Wasserstoffatom sowie R¹⁴ und R¹⁵ gemeinsam für eine zusätzliche Bindung,
- R¹¹, R^{11'} und R¹⁹: je für ein Wasserstoffatom oder R¹¹ für ein α-ständiges Wasserstoffatom und R^{11'} und R¹⁹ gemeinsam für eine zusätzliche Bindung oder R¹⁹ für ein Wasserstoffatom und R¹¹ und R^{11'} gemeinsam für eine zusätzliche Bindung stehen,
- R^{17β}/R^{17α}: -OR²¹ / -(CH₂)ₙ-A
-OR²¹ / -(CH₂)ₘ-C≡C-B
-OR²¹ / -(CH₂)ₚ-CH=CH-(CH₂)ₖ-D
-OR²¹ / -HC=C=CEG
-OR²¹/ -CF₃ 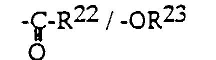 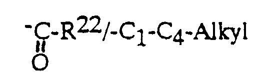 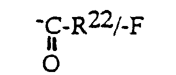
- oder R^{17β}/R^{17α}: gemeinsam 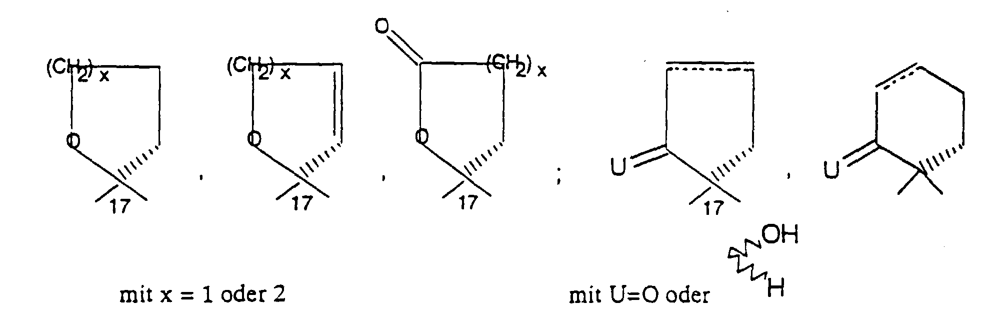 bedeuten, mit
- R²¹ und R²³: in der Bedeutung eines Wasserstoffatoms, einer C₁-C₄-Alkyl- oder einer C₁-C₄-Alkanoylgruppe,
- R²²: in der Bedeutung einer C₁-C₃-Alkylgruppe,
- A: in der Bedeutung eines Wasserstoffatoms, der Cyanogruppe, von -COOR²⁴ oder -OR²⁵ wobei R²⁴ für C₁-C₄-Alkyl und R²⁵ für Wasserstoff, C₁-C₄-Alkyl- oder C₁-C₄-Alkanoyl stehen,
- B: in der Bedeutung eines Wasserstoffatoms, einer C₁-C₄-Alkylgruppe, einer C₂- oder C₃-Alkinylgruppe, eines Fluor-, Chlor-, Brom- oder Jodatoms, einer Hydroxyalkyl-, Alkoxyalkyl- oder Alkanoyloxyalkylgruppe mit jeweils 1 bis 4 Kohlenstoffatomen im Alkyl-, Alkoxy- oder Alkanoyloxyteil,
- D: in der Bedeutung eines Wasserstoffatoms, einer Hydroxy-, C₁-C₄-Alkoxy- oder C₁-C₄-Alkanoyloxygruppe,
- E und G: in der Bedeutung von Wasserstoff oder C₁-C₃-Alkyl,
- n: in der Bedeutung 0, 1, 2, 3 oder 4,
- m: in der Bedeutung 0, 1 oder 2,
- p: in der Bedeutung 0 oder 1,
- k: in der Bedeutung 0, 1, 2 oder 3 und
- R¹⁸: für ein Wasserstoffatom oder eine Methylgruppe steht.

Bevorzugt gemäß vorliegender Erfindung sind solche Verbindungen der allgemeinen Formel I, in welchen
- W: für ein Sauerstoffatom oder zwei Wasserstoffatome,
- R^{6a} und R^{6b}: je für ein Wasserstoffatom oder gemeinsam mit dem Kohlenstoffatom 6 für einen Dreiring, oder
- R^{6a}: für ein Chlor- oder Bromatom oder für einen geradkettigen gesättigten α- oder β-ständigen C₁-C₄-Alkylrest, oder
- R^{6b} und R⁷: gemeinsam für eine β-ständige Methylenbrücke oder gemeinsam für eine zusätzliche Doppelbindung, oder
- R⁷: für einen gerad- oder verzweigtkettigen gesättigten α- oder β-ständigen Alkylrest mit bis zu 4 Kohlenstoffatomen,
- R¹⁴, R¹⁵ und R¹⁶: je für ein Wasserstoffatom oder
- R¹⁴: für ein α-ständiges Wasserstoffatom sowie R¹⁵ und R¹⁶ gemeinsam für eine zusätzliche Bindung oder für eine β-ständige Methylenbrücke stehen,
- R^{17β}/R^{17α}: -OH / -CH₃,
-OC(O)CH₃ / -CH₃;
-OH / -C≡CH,
-OC(O)CH₃ / -C≡CH;
-OH / -C≡C-CH_{3,}
-OC(O)CH₃ / -C≡C-CH₃;
-C(O)CH₃ / -OC(O)CH₃ 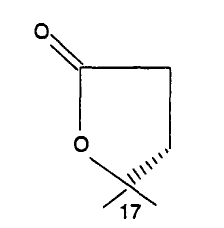 bedeuten,
sowie die anderen Substituenten alle die in Formel I angegebenen Bedeutungen haben können.

Die nachstehend genannten Verbindungen sind erfindungsgemäß insbesondere bevorzugt:
17-(Acetyloxy)-9,11α-dihydro-6'H-benzo[10,9,11]-19-norpregn-4-en-3,20-dion;
17-(Acetyloxy)-9,11α-dihydro-6'H-benzo[10,9,11]-19-norpregna-4,6-dien-3,20-dion;
17-(Acetyloxy)-9,11α-dihydro-6-methyl-6'H-benzo[10,9,11]-19-norpregna-4,6-dien-3,20-dion;
17-(Acetyloxy)-9,11α-dihydro-6α-methyl-6'H-benzo[10,9,11]-19-norpregn-4-en-3,20-dion;
17-(Acetyloxy)-6-chlor-9,11α-dihydro-6'H-benzo[10,9,11]-19-norpregna-4,6-dien-3,20-dion;
17-(Acetyloxy)-6-chlor-1β,2β,9,11α-tetrahydro-3'H-cyclopropa[1,2][6"H]benzo-[10,9,11]-19-norpregna-4,6-dien-3,20-dion;
17-(Acetyloxy)-4',5',9,11α-tetrahydro-6'H-benzo[10,9,11]-19-norpregn-4-en-3,20-dion;
17-(Acetyloxy)-6-methyl-4',5',9,11α-tetrahydro-6'H-benzo[10,9,11]-19-norpregna-4,6-dien-3,20-dion;
17-(Acetyloxy)-6-chlor-1β,2β,4",5",9,11α-hexahydro-3'H-cyclopropa[1,2][6"H]benzo-[10,9,11]-19-norpregna-4,6-dien-3,20-dion;
17-(Acetyloxy)-5',6'-dihydro-9H-benzo[10,9,11]-19-norpregn-4-en-3,20-dion;
17-(Acetyloxy)-5',6'-dihydro-6-methyl-9H-benzo[10,9,11]-19-norpregna-4,6-dien-3,20-dion;
17-(Acetyloxy)-9,11α-dihydro-6'H-benzo[10,9,11]-18a-homo-19-norpregn-4-en-3,20-dion;
17-(Acetyloxy)-9,11α-dihydro-6-methyl-6'H-benzo[10,9,11]-18a-homo-19-norpregna-4,6-dien-3,20-dion;
9,11α-Dihydro-17-methyl-6'H-benzo[10,9,11]-19-norpregn-4-en-3,20-dion;
3',9,11α,16β-Tetrahydrocyclopropa[16,17][6H]benzo[10,9,11]-19-norpregn-4-en-3,20-dion;
9,11α-Dihydro-17-methyl-6'H-benzo[10,9,11]-18a-homo-19-norpregn-4-en-3,20-dion;
3',9,11α,16β-Tetrahydrocyclopropa[16,17][6H]benzo[10,9,11]-18a-homo-19-norpregn-4-en-3,20-dion;
9,11α-Dihydo-17β-hydroxy-17α-methyl-6'H-benzo[10,9,11]estr-4-en-3-on;
9,11α-Dihydro-17α-ethinyl-17β-hydroxy-6'H-benzo[10,9,11]estr-4-en-3-on;
9,11α-Dihydo-17β-hydroxy-17α-(1-propinyl)-6'H-benzo[10,9,11]estr-4-en-3-on;
9,11α-Dihydro-17β-hydroxy-17α-(1,3-pentadiinyl)-6'H-benzo[10,9,11]estr-4-en-3-on;
(Z)-9,11α-Dihydro-17β-hydroxy-17α-(3-hydroxy-1-propenyl)-6'H-benzo[10,9,11]estr-4-en-3-on;
9,11α-Dihydo-17β-hydroxy-17α-(3-hydroxypropyl)-6'H-benzo[10,9,11]estr-4-en-3-on;
17β-Hydroxy-17α-methyl-4',5',9,11α-tetrahydro-6'H-benzo[10,9,11]estr-4-en-3-on;
17α-Ethinyl-17β-hydroxy-4',5',9,11α-tetrahydro-6'H-benzo[10,9,11]estr-4-en-3-on;
17β-Hydroxy-17α-(1-propinyl)-4',5',9,11α-tetrahydro-6'H-benzo[10,9,11]estr-4-en-3-on
5',6'-Dihydro-17α-ethinyl-17β-hydroxy-9H-benzo[10,9,11]estr-4-en-3-on;
5',6'-Dihydro-17β-hydroxy-17α-(1-propinyl)-9H-benzo[10,9,11]estr-4-en-3-on;
9,11α-Dihydro-17α-ethinyl-17β-hydroxy-6'H-benzo[10,9,11]-18a-homoestr-4-en-3-on;
9,11α-Dihydro-17β-hydroxy-17α-(1-propinyl)-6'H-benzo[10,9,11]-18a-homoestr-4-en-3-on;
17α-(1-Butinyl)-9,11α-dihydro-17β-hydroxy-6'H-benzo[10,9,11]-18a-homoestr-4-en-3-on;
9,11α-Dihydro-17α-(1,2-propadienyl)-17β-hydroxy-6'H-benzo[10,9,11]-18a-homoestr-4-en-3-on;
17α-Ethinyl-17β-hydroxy-4',5',9,11α-tetrahydro-6'H-benzo[10,9,11]18a-homoestr-4-en-3-on;
17β-Hydroxy-17α-(1-propinyl)-4',5',9,11α-tetrahydro-6'H-benzo[10,9,11]-18a-homoestr-4-en-3-on;
5',6'-Dihydro-17α-ethinyl-17β-hydroxy-9H-benzo[10,9,11]-18a-homoestr-4-en-3-on;
5',6'-Dihydro-17β-hydroxy-17α-(1-propinyl)-9H-benzo[10,9,11]-18a-homoestr-4-en-3-on;
9,11α-Dihydro-17α-ethinyl-17β-hydroxy-6'H-benzo[10,9,11]estra-4,15-dien-3-on;
9,11α-Dihydro-17β-hydroxy-17α-(1-propinyl)-6'H-benzo[10,9,11]estra-4,15-dien-3-on;
17α-Ethinyl-17β-hydroxy-4',5',9,11α-tetrahydro-6'H-benzo[10,9,11]estra-4,15-dien-3-on;
5',6'-Dihydro-17α-ethinyl-17β-hydroxy-9H-benzo[10,9,11]estra-4,15-dien-3-on;
5',6'-Dihydro-17β-hydroxy-17α-(1-propinyl)-9H-benzo[10,9,11]estra-4,15-dien-3-on;
9,11α-Dihydro-17α-ethinyl-17β-hydroxy-6'H-benzo[10,9,11]-18a-homoestra-4,15-dien-3-on;
9,11α-Dihydo-17β-hydroxy-17α-(1-propinyl)-6'H-benzo[10,9,11]-18a-homoestra-4,15-dien-3-on;
17α-Ethinyl-17β-hydroxy-4',5',9,11α-tetrahydro-6'H-benzo[10,9,11]-18a-homoestra-4,15-dien-3-on;
5',6'-Dihydro-17α-ethinyl-17β-hydroxy-9H-benzo[10,9,11]-18a-homoestra-4,15-dien-3-on;
5',6'-Dihydro-17β-hydroxy-17α-(1-propinyl)-9H-benzo[10,9,11]-18a-homoestra-4,15-dien-3-on;
4",5",9,11α-Tetrahydrospiro[6'H-benzo[10,9,11]estr-4-en-17β,2"(3"H)-furan]-3-on;
3",4",9,11α-Tetrahydrospiro[6'H-benzo[10,9,11]estr-4-en-17β,2"(5"H)-furan]-3,5"-dion;
3"",4"",6α,7α,9,11α,15α,16α-Octahydrospiro[3'H,3"H-dicyclopropa[6,7:15,16]-[6H]benzo[10,9,11]estr-4-en-17β,2""(5""H)-furan]-3,5""-dion;
3"",4"",9',11'α,15'α,16'α-Hexahydrospiro[cyclopropan-1,6'-[3H]cyclopropa-[15,16][6H]benzo[10,9,11]estr-4-en-17'β,2""(5"'H)-furan]-3',5""-dion;
3"",4"",9',11'α,15'α,16'α-Hexahydrospiro[cyclopropan-1,6'-[3H]cyclopropa[15,16]-[6H]benzo[10,9,11]estra-1,4-dien-17'β,2""(5""H)-furan]-3',5""-dion;
3"",4"',4"",5"',6α,7α,9,11α,15α,16α-Decahydrospiro[3'H,3"H-dicyclopropa[6,7:15,16][6H]benzo[10,9,11]estr-4-en-17β',2""(5""H)-furan]-3,5""-dion;
3",4",9,11α-Tetrahydrospiro[6'H-benzo[10,9,11]-18a-homoestr-4-en-17β,2"(5")-furan]-3,5"-dion;
3"",4"",6α,7α,9,11α,15α,16α-Octahydrospiro[3'H,3"H-dicyclopropa[6,7:15,16][6H]-benzo[10,9,11]-18a-homoestr-4-en-17β,2""(5""H)-furan]-3,5""-dion;
9,11α-Dihydro-17α-ethinyl-6'H-benzo[10,9,11]estr-4-en-17β-ol;
17α-Ethinyl-4',5',9,11α-tetrahydro-6'H-benzo[10,9,11]estr-4-en-17β-ol;
5',6'-Dihydro-17α-ethinyl-9H-benzo[10,9,11]estr-4-en-17β-ol;
9,11α-Dihydro-17α-ethinyl-6'H-benzo[10,9,11]estra-4,15-dien-17β-ol;
9,11α-Dihydro-17α-ethinyl-6'H-benzo[10,9,11]-18a-homoestr-4-en-17β-ol;
9,11α-Dihydro-17α-ethinyl-6'H-benzo[10,9,11]-18a-homoestra-4,15-dien-17β-ol.

19,11β-überbrückte Steroide als den vorliegend beschriebenen Verbindungen strukturell nächstkommende Spezies gehen erstmals aus der DE-A 37 08 942 (EP-A 0 283 428) hervor. Die bekannten Verbindungen weisen im Unterschied zu den hier in Frage stehenden Verbindungen aber keine Etheno- oder Ethanobrücke zwischen C11 und C19 auf; vielmehr sind dort die beiden Kohlenstoffatome durch zwei benachbarte Kohlenstoffatome eines im allgemeinen substituierten Phenylenringes überbrückt. Sowohl die bekannten als auch die hier beschriebenen Verbindungen zeichnen sich durch eine außerordentlich hohe Affinität zum Gestagenrezeptor aus.
Im Gestagenrezeptor-Bindungstest auf gestagene Wirkung unter Verwendung von Cytosol aus Kaninchenuterushomogenat und von ³H-Progesteron als Bezugssubstanz zeigen die neuen Verbindungen eine sehr starke Affinität zum Gestagenrezeptor und sind im Schwangerschaftserhaltungs-Test an der Ratte nach subkutaner Applikation stark wirksam.

In der folgenden Tabelle werden die Kompetitionsfaktoren (K_{F}) im Gestagenrezeptor-Bindungstest angegeben. Der Kompetitionsfaktor K_{F} als Maß für die Bindungsstärke ist definiert als das Verhältnis der Konzentration der Prüfsubstanz zur Konzentration des Standards (Progesteron), bei der beide Verbindungen eine gleich große Verdrängung von ³H-Progesteron vom Progesteron-Rezeptorkomplex zeigen, so daß ein niedriger K_{F}-Wert große Bindungsstärke (hohe Affinität) anzeigt (Tabelle 1):

**Tabelle 1**

| Gestagenrezeptor-Bindungstest | |
|---|---|
| Verbindung | K_{F} (Gestagen) |
| A | 0,6 |
| B | 0,7 |
| C | 0,4 |
| D | 0,6 |
| E | 0,5 |
| F | 0,5 |
| G | 0,6 |
| H | 0,7 |
| I | 0,3 |
| K | 0,3 |
| L | 0,4 |

A: 9,11α-Dihydro-17α-ethinyl-17β-hydroxy-6'H-benzo[10,9,11]estr-4-en-3-on;
B: 9,11α-Dihydro-17β-hydroxy-17α-(1-propinyl)-6'H-benzo[10,9,11]estr-4-en-3-on;
C: 9,11α-Dihydro-17α-ethinyl-17β-hydroxy-6'H-benzo[10,9,11]estra-4,15-dien-3-on;
D: 9,11α-Dihydro-17β-hydroxy-17α-(1-propinyl)-6'H-benzo[10,9,11]estra-4,15-dien-3-on;
E: 9,11α-Dihydro-17α-ethinyl-17β-hydroxy-6'H-benzo[10,9,11]-18a-homoestr-4-en-3-on;
F: 9,11α-Dihydro-17β-hydroxy-17α-(1-propinyl)-6'H-benzo[10,9,11]-18a-homoestr-4-en-3-on;
G: 9,11α-Dihydro-17α-ethinyl-17β-hydroxy-6'H-benzo[10,9,11]-18a-homoestra-4,15-dien-3-on;
H: 9,11α-Dihydro-17β-hydroxy-17α-(1-propinyl)-6'H-benzo[10,9,11]-18a-homoestra-4,15-dien-3-on;
I: 17-(Acetyloxy)-9,11α-dihydro-6'H-benzo[10,9,11]-19-norpregn-4-en-3,20-dion;
K: 17-(Acetyloxy)-9,11α-dihydro-6'H-benzo[10,9,11]-19-norpregna-4,6-dien-3,20-dion;
L: 17-(Acetyloxy)-9,11α-dihydro-6-methyl-6'H-benzo[10,9,11]-19-norpregna-4,6-dien-3,20-dion.

Während diese starke Bindung aber bei den bekannten Verbindungen in erster Linie zu ausgeprägter kompetitiver progesteron-antagonistischer Wirksamkeit führt und diese Verbindungen somit in erster Linie zur Auslösung von Aborten, gegen hormonelle Unregelmäßigkeiten, zur Menstruationsauslösung und zur Geburtseinleitung eingesetzt werden können, zeichnen sich die erfindungsgemäßen Verbindungen überraschenderweise durch starke agonistische, d.h. gestagene Wirksamkeit aus.

Die gestagene Wirkung wurde im bekannten Schwangerschaftserhaltungstest bei der Ratte nach subkutaner Applikation der Verbindungen ermittelt. Die Ergebnisse sind in Tabelle 2 zusammengefaßt:

**Tabelle 2**

| Schwangerschaftserhaltungstest an der Ratte nach s.c. Applikation | |
|---|---|
| Verbindung | voll wirksam bei [mg/Tag/Tier] |
| A | 0,1 |
| B | 0,1 |
| C | 0,03 |
| D | 0,03 |
| E | 0,03 |
| F | 0,1 |
| G | 0,03 |
| H | 0,1 |

Die erfindungsgemäßen Verbindungen der allgemeinen Formel I verfügen somit über sehr starke gestagene Wirksamkeit bei nur schwacher androgener oder sogar schwach antiandrogener Aktivität (Dissoziation).

Aufgrund ihrer gestagenen Wirksamkeit können die neuen Verbindungen der allgemeinen Formel I allein oder in Kombination mit Estrogen in Präparaten zur Kontrazeption verwendet werden.

Die Dosierung der erfindungsgemäßen Verbindungen in Kontrazeptionspräparaten soll vorzugsweise 0,01 bis 2 mg pro Tag betragen.
Die gestagenen und estrogenen Wirkstoffkomponenten werden in Kontrazeptionspräparaten vorzugsweise zusammen oral appliziert. Die tägliche Dosis wird vorzugsweise einmalig verabfolgt.

Als Estrogene kommen vorzugsweise synthetische Estrogene wie Ethinylestradiol, 14α,17α-Ethano-1,3,5(10)-estratrien-3,17β-diol (WO 88/01275) oder 14α,17α-Ethano-1,3,5(10)-estratrien-3,16α,17β-triol (WO 91/08219) in Betracht.

Das Estrogen wird in einer Menge verabfolgt, die der von 0,01 bis 0,05 mg Ethinylestradiol entspricht.

Die neuen Verbindungen der allgemeinen Formel I können auch in Präparaten zur Behandlung gynäkologischer Störungen und zur Substitutionstherapie eingesetzt werden. Wegen ihres günstigen Wirkungsprofils sind die erfindungsgemäßen Verbindungen besonders gut geeignet zur Behandlung praemenstrueller Beschwerden, wie Kopfschmerzen, depressive Verstimmungen, Wasserretention und Mastodynie. Die Tagesdosis bei der Behandlung praemenstrueller Beschwerden liegt bei etwa 1 bis 20 mg.

Die Formulierung der pharmazeutischen Präparate auf Basis der neuen Verbindungen erfolgt in an sich bekannter Weise, indem man den Wirkstoff, gegebenenfalls in Kombination mit einem Estrogen, mit den in der Galenik gebräuchlichen Trägersubstanzen, Verdünnungsmitteln, gegebenenfalls Geschmackskorrigentien usw., verarbeitet und in die gewünschte Applikationsform überführt.

Für die bevorzugte orale Applikation kommen insbesondere Tabletten, Dragees, Kapseln, Pillen, Suspensionen oder Lösungen infrage.

Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Es ist auch möglich, die erfindungsgemäßen Substanzen in ein transdermales System einzuarbeiten und sie damit transdermal zu applizieren.

Schließlich können die neuen Verbindungen auch als gestagene Komponente in den neuerdings bekannt gewordenen Zusammensetzungen für die weibliche Fertilitätskontrolle, die sich durch die zusätzliche Verwendung eines kompetitiven Progesteronantagonisten auszeichnen, zum Einsatz kommen (H. B. Croxatto und A. M. Salvatierra in Female Contraception and Male Fertility Regulation, ed. by Runnebaum, Rabe & Kiesel - Vol. 2, Advances in Gynecological and Obstetric Research Series, Parthenon Publishing Group - 1991, Seite 245).

Die Dosierung liegt im bereits angegebenen Bereich, die Formulierung kann wie bei konventionellen OC-Präparaten erfolgen. Die Applikation des zusätzlichen, kompetitiven Progesteronantagonisten kann dabei auch sequentiell vorgenommen werden.

Die neuen Verbindungen der allgemeinen Formel I werden erfindungsgemäß hergestellt, indem eine Verbindung der allgemeinen Formel II 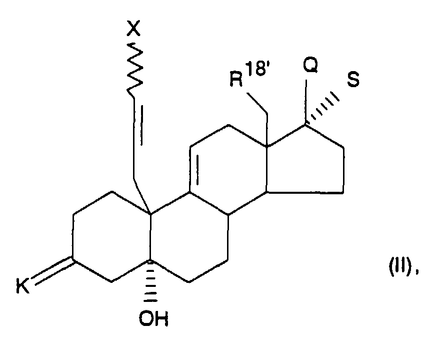 worin
K für eine Ketoschutzgruppe oder eine geschützte Hydroxygruppe und ein Wasserstoffatom,
X für ein syn- oder antiständiges Chlor- oder Bromatom,
R¹⁸ für ein Wasserstoffatom oder eine Methylgruppe sowie
Q für eine β-ständige Hydroxygruppe und S für ein α-ständiges Wasserstoffatom oder Q und S gemeinsam für ein Ketosauerstoffatom oder außerdem
Q und S für eine der in Formel I genannten R^{17β}/R^{17α}-Substituentenkombinationen einschließlich der Spiroverbindungen, wobei darin vorhandene Hydroxygruppen und/oder Ketogruppen gegebenenfalls geschützt sind,
stehen,
a) durch radikalische Cyclisierung in eine Verbindung der allgemeinen Formel III 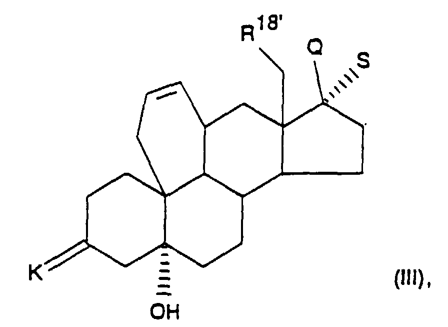 worin K, R¹⁸ sowie Q und S die in Formel II angegebene Bedeutung haben, überführt,
b) anschließend, wenn Q für eine Hydroxygruppe steht, diese gewünschtenfalls oxidiert,
c) wenn R¹¹, R^{11'} und R¹⁹ letztendlich Wasserstoffatome sein sollen, die 19,11β-Ethenobrücke hydriert, oder
d) wenn R¹⁹ letzendlich für ein Wasserstoffatom sowie R¹¹ und R^{11'} gemeinsam für eine zusätzliche Bindung stehen sollen, die Doppelbindung in der 19,11β-Ethenobrücke in die 11-Position (exoständig) isomerisiert,
e) gewünschtenfalls in den D-Ring eine 15,16-Doppelbindung eingeführt und diese
f) gewünschtenfalls in die 14,15-Position isomerisiert oder
g) durch Methylenierung in die entsprechende 15β,16β-Methylenverbindung sowie
h) wenn Q und S gemeinsam ein Ketosauerstoffatom bedeuten, durch nucleophile Addition des Substituenten R^{17α} oder eines reaktiven Vorläufers von R^{17α} und gegebenenfalls Veretherung oder Veresterung der 17β-Hydroxygruppe mit einem den entsprechenden Rest R²¹ liefernden Reagenz oder das 17α-Hydroxy-17β-alkanoylsubstitutionsmuster aufgebaut und die 17α-Hydroxygruppe gegebenenfalls mit einem den entsprechenden Rest R²³ liefernden Reagenz verethert oder verestert,
i) gegebenenfalls durch teilweises oder vollständiges Hydrieren einer ungesättigten C₁₇-Seitenkette und
j) gewünschtenfalls durch Oxidation der entsprechenden 17-(3-Hydroxypropyl)- bzw. 17-(4-Hydroxybutyl)-Verbindung zur Bildung des 17-Spirolaktons oder
k) gewünschtenfalls durch Ringschlußreaktion der entsprechenden (Z)-17α-(3-Hydroxyprop-1-enyl)- bzw. (Z)-17α-(4-Hydroxybut-1-enyl)-17β-hydroxyverbindung oder der entsprechenden in der Seitenkette gesättigten Verbindungen zur Bildung des Spiroethers und
l) durch Säurebehandlung in einem mit Wasser mischbaren Lösungsmittel in das Δ⁴-3-Keto-System, wobei weitere vorhandene Schutzgruppen ebenfalls abgespalten werden, und somit in eine Verbindung der allgemeinen Formel I überführt, und diese Verbindung der allgemeinen Formel I gegebenenfalls
m) durch Einführung einer 1,2- und/oder 6,7-Doppelbindung und gegebenenfalls Methylenierung einer oder beider Doppelbindung(en),
n) durch Einführung eines gerad- oder verzweigtkettigen α- oder β-ständigen Alkylrestes oder einer Thiogruppe -SR²⁰ in 7-Position,
o) durch Epoxidierung der 6,7-Doppelbindung und Öffnung des Epoxides mit Halogenwasserstoff (Hal = F, Cl, Br, I) und Eliminierung der gebildeten 7α-Hydroxygruppe,
p) durch 6α-Hydroxymethylierung und anschließende Wasserabspaltung zur 6-Methylenverbindung,
q) durch Isomerisierung der exo-ständigen Doppelbindung der 6-Methylengruppe oder durch direkte Einführung einer 6-Alkylgruppe (6-Alkyl-4,6-dien-3-on),
r) durch Hydrierung der 6-Methylengruppe
in eine Verbindung der allgemeinen Formel I
worin
R¹, R², R¹⁴, R¹⁵, R¹⁶, R^{17α}, R^{17β}, R¹⁸, R¹¹ und R¹⁹ die letztendlich gewünschte Bedeutung haben,
und R^{6a} eine α-Methylgruppe und R^{6b} und R⁷ je ein Wasserstoffatom oder gemeinsam eine zusätzliche Bindung darstellen,
umgewandelt,
oder,
s) wenn R^{6a} letztendlich ein gerad- oder verzweigtkettiger gesättigter α- oder β-ständiger Alkylrest mit bis zu 4 Kohlenstoffatomen sein soll,
   durch Ketalisierung unter gleichzeitiger Isomerisierung der 4(5)-Doppelbindung nach 5(6), Epoxidierung der 5(6)-Doppelbindung und nucleophile Öffnung des 5,6α-Epoxids mit geschützter 3-Ketogruppe mit einem/einer gerad- oder verzweigtkettigen gesättigten Alkylmagnesiumhalogenid oder Alkyllithiumverbindung mit bis zu 4 Kohlenstoffatomen im Alkylrest und Spaltung der 3-Ketoschutzgruppe in der gebildeten 5α-Hydroxy-6β-Alkylverbindung unter milden sauren Bedingungen zur entsprechenden 3-Keto-5α-hydroxy-6β-alkyl-Verbindung und basische Eliminierung der 5α-Hydroxygruppe in die entsprechende 3-Keto-4-en-Verbindung der allgemeinen Formel I mit β-ständiger 6-Alkylgruppe oder durch Abspaltung der 3-Ketoschutzgruppe unter drastischeren Bedingungen in die entsprechende 3-Keto-4-en-Verbindung der allgemeinen Formel I mit α-ständiger 6-Alkylgruppe überführt und
t) gegebenenfalls eine der vorstehend erhaltenen 3-Keto-Verbindungen mit Hydroxylaminhydrochlorid in Gegenwart von tertiären Aminen bei einer Temperatur zwischen -20° und +40°C in die 3-Hydroxyiminoverbindung (W = 〉 N~OH; bedeutet syn- oder antiständiges OH) oder
u) gegebenenfalls in das 3-Thioketal, vorzugsweise das 3-(1',3'-Ethylendithio)-Ketal, umgewandelt und dieses reduktiv zur Verbindung der allgemeinen Formel I, worin W für zwei Wasserstoffatome steht,
gespalten wird.

Die Syntheseroute für die die neuartige Überbrückung enthaltenden Verbindungen ist in Schema 1 gezeigt:

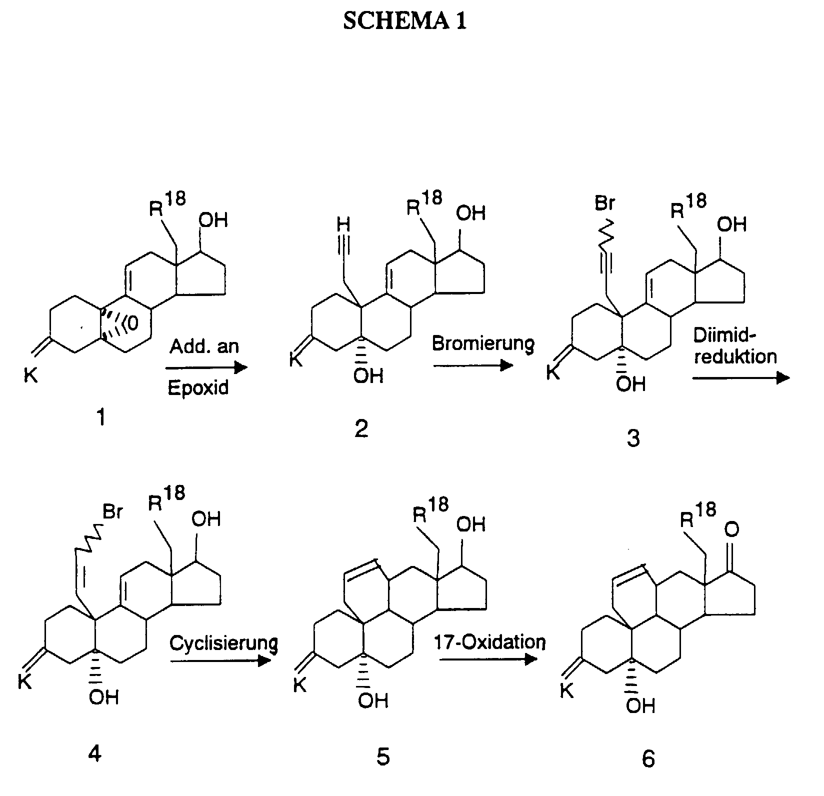

Gemäß Schema 1 wird das z.B. in den Europäischen Patentanmeldungen 0110434 und 0127864 beschriebene Epoxid 1, in dem R¹⁸ für ein Wasserstoffatom oder eine Methylgruppe steht und K für eine Ketalschutzgruppe, durch Öffnung mit Propargylmagnesiumhalogeniden (Darstellung siehe "Synthesis of Acetylenes, Allenes and Cumulenes", L. Brandsma und H.D. Verkruijsse, S. 16, Elsevier Scientific Publishing Company, Amsterdam, Oxford, New York (1981)) in eine Verbindung der Formel **2** überführt. K ist eine gängige Ketalschutzgruppe, beispielsweise die Ethylendioxy- oder die 2,2-Dimethylpropylen-1,3-dioxygruppe. Auch andere gängige Ketoschutzgruppen kommen in Betracht. K kann auch eine geschützte Hydroxygruppe und ein Wasserstoffatom bedeuten, wobei die Hydroxygruppe dann beispielsweise als Methoxymethyl-, Methoxyethyl-, Tetrahydroxypyranyl- oder Silylether geschützt ist. Durch Abspaltung der Schutzgruppe und Oxidation der freien Hydroxygruppe gelangt man zur Ketogruppe.

Verbindung **2** wird dann nach einem bekannten Verfahren am terminalen Ende der Dreifachbindung bromiert (H. Hofmeister, K. Annen, H. Laurent und R. Wiechert, Angew. Chem. **96**, S. 720 (1984)). Anschließend wird die erhaltene Verbindung **3** durch Hydrierung bzw. Hydrid-Übertragung in das Vinylhalogenid **4** überführt. Vorzugsweise wird die Reaktion durch Diimid-Reduktion durchgeführt.

Die radikalische Cyclisierung von Verbindung **4** erfolgt analog zu der bereits mehrfach beschriebenen Cyclisierung von entsprechenden Arylhalogenierungen (siehe z.B. E. Ottow, G. Neef und R. Wiechert; Angw. Chem. **101**, S. 776 (1989)). Von den möglichen Verfahren zur Generierung der intermediären Radikale kommen auch hier insbesondere zwei zur Anwendung:
Die Reaktion mit Trialkylstannanen, vorzugsweise mit Tributylzinnhydrid, in geeigneten Lösungsmitteln, wie z.B. Toluol, oder die Umsetzung mit Lithium in flüssigem Ammoniak, gemischt mit einem organischen Lösungsmittel wie z.B. Tetrahydrofuran bei Temperaturen zwischen -78° und -33°C.

Verbindung **5** kann dann in bekannter Weise durch Oxidation der 17-Hydroxyfunktion in **6** überführt werden. Die Verbindungen **5** und **6** sind Ausgangsprodukte bei der Darstellung von Verbindungen der allgemeinen Formel I.

Sind R¹¹, R^{11'} und R¹⁹ je ein Wasserstoffatom, so kann die in **5** oder **6** vorhandene Doppelbindung nach bekannten Verfahren hydriert werden.

Soll R¹⁹ ein Wasserstoffatom sein sowie R¹¹ und R^{11'} gemeinsam für eine zusätzliche Doppelbindung stehen, so wird die Isomerisierung der ursprünglichen Doppelbindung (R¹¹ = H, R^{11'} und R¹⁹ bilden gemeinsam eine zusätzliche Bindung) durch Erwärmen der Verbindung **5** oder **6** in Ethanol mit 5%-Palladium-Kohle-Katalysator, der entweder mit Wasserstoff oder durch Erwärmen mit einer geringen Menge Cyclohexen vorbehandelt wurde, erzielt. Die Isomerisierung kann aber auch erreicht werden durch Umsetzung mit Palladium-Kohle-Katalysator in z.B. Ethanol oder Tetrahydrofuran/Ethanol-Gemischen unter einer Atmosphäre Wasserstoffdruck bei Raumtemperatur oder durch Kochen dieses Reaktionsgemisches mit Cyclohexen anstelle von Wasserstoff.

Die nächsten Schritte beinhalten dann eventuell gewünschte Funktionalisierungen im D-Ring: Die Einführung einer 15,16-Doppelbindung (R¹⁵ und R¹⁶ bilden eine gemeinsame zusätzliche Bindung) erfolgt z.B. durch eine modifizierte Saegusa-Oxidation (I. Minami, K. Takahashi, I. Shimizu, T. Kimura, J. Tsuji; Tetrahydron **42** (1986) S. 2971; EP-A 0299913) der entsprechenden Enolverbindungen des 17-Ketons.

Gegebenenfalls kann die Doppelbindung nach Position 14 isomerisiert werden. Hierzu behandelt man die 15,16-en-Verbindungen mit Kieselgel/Triethylamin (S. Scholz et al., Lieb. Ann.Chem. 1989, S. 151).

Für Beispiele in denen R¹⁵ und R¹⁶ gemeinsam eine β-ständige Methylengruppe darstellen erfolgt die Einführung dieser Gruppe z.B. durch Umsetzung der entsprechenden 15,16-en-17-on-Verbindung mit Dimethylsulfoxoniummethylid (siehe z.B. Deutsche Auslegeschrift 11 83 500, Deutsche Offenlegungsschrift 29 22 500, EP-A 0-0 19690, US 4 291 029 A, EJ. Corey, M. Chaykovsky, J. Am. Chem. Soc. **84**, S. 867 (1962)).

Nach erfolgter D-Ringmodifikation gelten die weiteren Schritte zunächst der Einführung der Reste R^{17α} und R^{17β} am C-17-Atom. Diese Einführung erfolgt analog zu literaturbekannten Verfahren (z.B. J. Fried, J.A. Edwards, "Organic Reactions in Steroid Chemistry", van Nostrand Reinhold Company, 1972, Vol. 1 und 2; "Terpenoids and Steroids", Specialist Periodical Report, The Chemical Society, London, Vol. 1-2) in den meisten Fällen durch nucleophile Addition des Substituenten R^{17α} oder eines reaktiven Vorläufers von R^{17α} an das C-17-Atom.
Im Falle eines leicht enolisierbaren 17-Ketons, wie z.B. der 14,15-en Verbindungen, werden Nucleophile unter Zusatz von Cersalzen eingeführt (T. Imamoto, N. Takiyana, K. Nakamura, Y. Sugiura, Tet. Lett. **25**, 4233 (1984)).

Die Einführung des Substituenten C≡C-B als R^{17α} mit den genannten Bedeutungen für B erfolgt mit Hilfe der metallierten Verbindungen, die auch in situ gebildet und mit dem 17-Keton zur Reaktion gebracht werden können. Die Bildung der metallierten Verbindungen erfolgt zum Beispiel durch Reaktion der Acetylene mit Alkalimetallen, insbesondere Kalium, Natrium oder Lithium, in Gegenwart eines Alkohols oder in Gegenwart von Ammoniak. Das Alkalimetall kann aber auch in Form von z.B. Methyl- oder Butyllithium zur Einwirkung kommen. Verbindungen in denen B = Brom oder Jod ist, werden aus den 17-Ethinyl- Verbindungen in bekannter Weise dargestellt (siehe z.B. H. Hofmeister, K. Annen, H. Laurent und R. Wiechert, Angew. Chem. **96**, 720 (1984)).

Die Einführung von 3-Hydroxy-1-propin in 17-Stellung erfolgt durch Umsetzung des 17-Ketons mit dem Dianion des Propargylalkohols (3-Hydroxypropin), z.B. dem in situ generierten Dikaliumsalz des Propargylalkohols oder mit entsprechenden, an der Hydroxyfunktion geschützten Derivaten, wie z.B. der Lithiumverbindung des 3-[(Tetrahydro-2H-pyran-2-yl)oxy]-1-propin.
Die Hydroxypropyl- und Hydroxypropenyl-Verbindungen können aus den Hydroxypropinyl-Derivaten dargestellt werden. Die Darstellung der Hydroxypropylkette erfolgt z.B. durch Hydrierung bei Raumtemperatur und Normaldruck in Lösungsmitteln wie Methanol, Ethanol, Tetrahydrofuran oder Ethylacetat unter Zusatz von Edelmetall-Katalysatoren wie Platin oder Palladium.

Die Darstellung von Verbindungen mit Z-konfigurierter Doppelbindung in der Seitenkette erfolgt durch Hydrieren der acetylenischen Dreifachbindung mit einem desaktivierten Edelmetallkatalysator, z.B. 10% Palladium auf Bariumsulfat in Gegenwart eines Amins oder 5% Palladium auf Calciumcarbonat unter Zusatz von Blei(II)-Acetat. Die Hydrierung wird nach der Aufnahme eines Äquivalents Wasserstoff abgebrochen.

Verbindungen mit E-konfigurierter Doppelbindung in der Seitenkette entstehen durch Reduktion der Dreifachbindung, z.B. mit Natrium in flüssigem Ammoniak (K.N. Cambell, L.T. Eby, J. Am. Chem. Soc. **63** (1941), S. 216), mit Natriumamid in flüssigem Ammoniak oder mit Lithium in niedermolekularen Aminen (R.A. Benkeser et al., J. Am. Chem. Soc. **77** (1955), S. 3378).
Die Einführung der Hydroxyalkene und Hydroxyalkane kann auch direkt durch Umsetzung des 17-Ketons mit metallierten Derivaten erfolgen (E.J. Corey, R.H. Wollenberg, J. Org. Chem. **40**, 2265 (1975); H.P. On. W. Lewis, G. Zweifel, Synthesis 1981, S. 999; G. Gohiez, A. Alexakis, J.F. Normant, Tet. Lett. 1978, S. 3013; P.E. Eaton et al., J.Org. Chem. **37**, 1947). Die Einführung homologer Hydroxyalkin-, Hydroxyalken- und Hydroxyalkangruppen ist in entsprechender Weise möglich.

Produkte in denen R^{17α}/R^{β} für 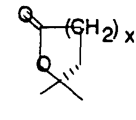 mit X = 1 oder 2 steht, lassen sich aus den 17-(3-Hydroxypropyl)- bzw. 17-(4-Hydroxybutyl)-Verbindungen durch Oxidation in bekannter Weise, z.B. mit dem Jones-Reagenz, Braunstein, Pyridiniumdichromat, Pyridiniumchlorochromat, Chromsäure-Pyridin oder dem Fetizon-Reagenz darstellen.

Produkte in denen R^{17α}/R^{17β} für 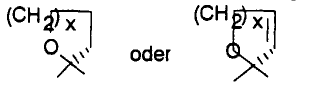 mit x = 1 oder 2 steht, können durch Ringschlußreaktion der entsprechenden (Z)-17α-(3-Hydroxyprop-1-enyl) bzw. (Z)-17α-(4-Hydroxy-1-butenyl)-17β-hydroxy-Verbindungen oder den entsprechend in der Seitenkette gesättigten Verbindungen dargestellt werden. Verbindungen mit einem gesättigten Spiroether können durch Hydrierung der ungesättigten Spiroether an Platin- oder Palladium-Katalysatoren dargestellt werden.

17α-1,2-Alkdienyl-substituierte Steroide sind beispielsweise durch Umsetzung der gegebenenfalls als Tetrahydropyranyl-Ether, α-Alkoxyethylether, Alkyl- oder Arylsulfonate geschützten 17α-(3-Hydroxy-1-alkin)-substituierten Verbindungen mit komplexen Hydriden in aliphatischen oder alicyclischen Ethern (siehe z.B. A. Burger, J.-P. Roussel, C. Hetru, J. A. Hoffmann und B. Luu, Tetrahedron **45**, 155 (1989); A. Claesson, L.-I. Olsson und C. Bogentoft, Acta Chem. Scand. **27**, 2941 (1973); L.-I. Olsson und A. Claesson, Acta Chem. Scand. **B31**, 614 (1977)) aber auch nach anderen literaturbekannten Verfahren (siehe z.B. DE-AS 19 58 5333; DE-OS 16 68 679) zugänglich.

Die Einführung einer Trifluormethylgruppe gelingt durch Umsetzung des 17-Ketons mit Trifluormethyltrimethylsilan in Gegenwart von Tetrabutylammoniumfluorid (siehe R. Krshnamurti, D. R. Bellew und G. K. S. Prahash, J. Org. Chem., 56, 984 (1991).

Der Aufbau der 17-Cyanmethylseitenkette erfolgt aus dem 17-Keton entweder direkt durch Addition von Acetonitril oder durch Spaltung des Spiroepoxides mit HCN gemäß K. Ponsold et al., Z. Chem. **18**, (1978) 259-260.

Die Synthese 16.17-α-Methylen-17β-alkanoyl-substituierter Verbindungen erfolgt nach literaturbekannten Verfahren. So lassen sich beispielsweise ausgehend von den 17-Ketonen Δ¹⁶-17-Perfluorsulfonyloxyverbindungen darstellen, die in Gegenwart von Übergangsmetallkatalysatoren mit Alkoxyvinylzinn- oder Zinkverbindungen gekuppelt werden können (siehe z.B. M. Kosugi, T. Sumiya, Y. Obara, M. Suzuki, H. Sano und T. Migita, Bull. Chem. Soc. Jpn. **60**, 767 (1987); P. G. Ciattini, E. Morera und G. Ortar, Tetrahedron Lett. **31**, 1889 (1990)). Saure Hydrolyse der Kupplungsprodukte ergibt die Δ¹⁶-17-Acetylverbindungen. Diese Enone lassen sich nach den oben für die Cyclopropanierung der Δ¹⁵-17-Ketone angegebenen Verfahren mit Trimethylsulfoxoniumjodid zu 16,17α-Methylen-17β-acetylverbindungen umsetzen, oder aber durch konjugierte Addition von Alkylkupferverbindungen in die 16α-Alkyl-progesteronderivate überführen.

Verbindungen, in denen R^{17α} ein Alkylrest und R^{17β} ein Alkanoylrest ist, lassen sich beispielsweise aus Δ¹⁶-17-Alkanoylverbindungen oder17α-Hydroxy-17β-alkanoylverbindungen darstellen, indem man durch Reduktion mit Lithium in flüssigem Ammoniak gemischt mit Tetrahydrofuran 17-Enolatanionen erzeugt, die sich mit Alkylhalogeniden zu den gewünschten Verbindungen alkylieren lassen (siehe z.B. M. J. Weiss, R. E. Schaub, G. R. Allen, Jr., J. F. Poletto, C. Pidacks, R. B. Conrow und C. J. Coscia, Tetrahedron **20**, 357 (1964).

Die Darstellung von Derivaten, in denen R^{17α} und R^{17β} gemeinsam für 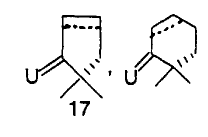 stehen, erfolgt ausgehend vom 17-Keton nach literaturbekannten Methoden (z.B. EP-A-0 444 3951, 1991; und EP-A-0 154 429; 1989). Hierzu werden beispielsweise durch reduktive Allylierung der oben erwähnten Δ¹⁶-17-Acetylverbindung die entsprechende 17α-(2-Propenyl)verbindungen (Allylierung mit Allylbromid) dargestellt. Die endständige Doppelbindung wird dann entweder über Hydroborierung, z.B. mit 9.BBN (9-Borabicyclo nonan), oxidative Aufarbeitung und Weiteroxidation zum entsprechenden C₃-Aldehyd oder aber durch ozonolytischen Abbau in den C₂-Aldehyd überführt. Die 6-Ring- bzw. 5-Ringspiroketone können dann via Aldolreaktion dargestellt werden. Hierbei entstehen zunächst die α,β-ungesättigten Ketone, die gegebenenfalls nach bekannten Methoden zu den gesättigten Ketonen reduziert werden können.

Die Einführung eines Hydroxyprogesteronsubstitutionsmusters (17β = Acetyl, 17α = Hydroxy) oder der Aufbau der entsprechenden homologen 17α-Hydroxy-17β-alkanoyl-Verbindungen erfolgt nach literaturbekannten Verfahren. Besonders hervorzuheben ist hierbei der Weg über eine 17β-Cyano-17α-hydroxy-Verbindung (Cyanhydrinmethode; siehe u.a. DE 39 31 064 A1 (1989); DDR-Patent 147 669 (1981); DE 21 10 140 (1971); Jap. Patent 57062296-300 (1982); J.C. Gase und L. Nedelec, Tet. Lett. 1971, S. 2005; J.N.M. Batist, N.C.M.E. Barendse, A.F. Marx, Steroids 1990, S. 109).

Hierbei wird das 17-Keton durch Umsetzung mit zum Beispiel Acetoncyanhydrin (2-Hydroxy-2-methylpropannitril) in geeigneten Lösungsmittelsystemen, z.B. Ethanol oder Methanol und Dichlormethan bei einem geeigneten (zumeist leicht basischen) pH-Wert (wird durch Zugabe von KCN oder NaCN bzw. KOH oder NaOH eingestellt) umgesetzt. Unter diesen Reaktionsbedingungen kann ein Auskristallisieren der 17β-Cyanoverbindung erreicht werden. Die 17α-Hydroxyfunktion wird dann geschützt und anschließend läßt man die Cyanogruppe mit C₁-C₄-Alkyllithium-, z.B. mit Methyllithium oder C₁-C₄-Alkylmagnesiumhalogeniden-, z.B. Methylmagnesiumhalogeniden, reagieren, um dann nach saurer Spaltung zur 17α-Hydroxy-17β-alkanoyl-Verbindung zu gelangen. Ausgehend von den 17α-Hydroxy-17β-alkanoyl-Verbindungen können dann in bekannter Weise die 17α-Alkanoyloxy-Derivate erhalten werden.

Des weiteren besonders hervorzuheben sind die Überführung von 17α-Ethinyl-17β-nitrooxy-verbindungen (siehe H. Hofmeister, K. Annen, H. Laurent und R. Wiechert, Chem. Ber. **111**, 3086 (1978)) bzw. von aus 17α-Ethinyl-17β-hydroxyverbindungen durch Umsetzung mit Phenylsulfenylchlorid generierten Allensulfoxiden (siehe V. VanRheenen und K. P. Shephard, J. Org. Chem. **44**, 1582 (1979)) in die 17α-Hydroxy-17β-acetylverbindungen.

17β-Acetyl-17α-fluorverbindungen können z.B. aus den entsprechenden 17β-Acetyl-17α-hydroxyverbindungen durch Umsetzung mit DAST (Diethylaminoschwefeltrifuorid) in geeigneten Lösungsmitteln wie beispielsweise Trichlormethan dargestellt werden.

Die sich anschließende Freisetzung der 3-Ketofunktion unter Wasserabspaltung und Ausbildung der 4(5)-Doppelbindung erfolgt durch Behandlung mit Säure oder einem sauren Ionenaustauscher. Die saure Behandlung erfolgt in an sich bekannter Weise, indem man das entsprechende 5α-Hydroxy-3-Ketal in einem mit Wasser mischbaren Lösungsmittel, wie wäßrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis vorhandene Schutzgruppen entfernt sind. Die Umsetzung, die bei Temperaturen von 0 bis 100° C abläuft, kann auch mit einem sauren Ionenaustauscher vorgenommen weden. Der Verlauf der Umsetzung kann mit analytischen Methoden, beispielsweise durch Dünnschichtchromatographie entnommener Proben, verfolgt werden.

Die nächsten Schritte gelten in aller Regel dem Aufbau der Reste R^{6a}, R^{6b}, R⁷ und R¹, R².
Für Endverbindungen in denen eine 1,2- und eine 6,7-Doppelbindung vorliegt oder für entsprechende Zwischenprodukte in denen beide Doppelbindungen nebeneinander erwünscht sind, kann, ausgehend vom 3-Keton (W = Sauerstoff), prinzipiell über eine 2,6-Dibromierung und anschließende Eliminierung die Einführung beider Doppelbindungen gleichzeitig erfolgen (siehe z.B. Deutsche Auslegeschrift 11 19 266). Häufig ist es jedoch notwendig aufgrund der anderen Funktionalitäten im Molekül die beiden Doppelbindungen nacheinander einzuführen. Hierbei findet in aller Regel zunächst die Einführung einer 6,7-Doppelbindung statt. Diese Einführung gelingt über eine Dienoletherbromierung und anschließende Bromwasserstoffabspaltung (siehe z.B. J. Fried, J.A. Edwards, Organic Reactions in Steroid Chemistry, von Nostrand Reinhold Company 1972, S. 265-374).

Die Dienoletherbromierung kann z.B. analog der Vorschrift in Steroids 1, 233 erfolgen. Die Bromwasserstoffabspaltung gelingt durch Erhitzen der 6-Bromverbindung mit basischen Mitteln, wie z.B. LiBr oder LiCO₃ in aprotischen Lösungsmitteln wie Dimethylformamid bei Temperaturen von 50-120°C oder aber indem die 6-Bromverbindungen in Collidin oder Lutidin erhitzt werden.

Die Einführung einer 1,2-Doppelbindung kann je nach gewünschter Endverbindung direkt nach Einführung der 6,7-Doppelbindung oder auch auf einer späteren Zwischenstufe erfolgen. Diese Dehydrierung gelingt vorzugsweise auf chemischem oder auch mikrobiologischem Weg nach literaturbekannten Verfahren (z.B. DE 34 02 329 A1 und EP-A-0 150 157).
Die chemische Dehydrierung erfolgt beispielsweise durch Erwärmen mit Selendioxid, 2,3-Dichlor-5,6-dicyanobenzochinon, Chloranil, Thalliumtriacetat oder Bleitetraacetat in geeigneten Lösungsmitteln wie z.B. Dioxan, tert.-Butanol, Tetrahydrofuran, Toluol, Benzol bzw. Gemischen dieser Lösungsmittel.

Die Einführung der 1,2-Doppelbindung kann aber auch durch eine modifizierte Saegusa-Oxidation (I. Minami, K. Takahashi, I. Shimizu, T. Kimura, J. Tsuji, Tetrahedron **42** (1986), S. 2971; EP-A-0 299 913) der entsprechenden Enolverbindungen des 3-Ketons erfolgen.

Verbindungen, die eine 1,2α-Methylenfunktion tragen, werden aus den 1,2-ungesättigten Verbindungen durch Umsetzung mit Dimethylsulfoxoniummethylid analog zur Darstellung der 15,16β-Methylenverbindung (sie oben) dargestellt. Hierbei ist eine selektive Einführung der 1,2-Methylenfunktion auch in Gegenwart der 4,6-dien-3-on-Einheit möglich (siehe z.B. Deutsche Auslegeschrift 11 83 500).

Für Verbindungen mit einer 6,7-Methylenfunktion erfolgt die Einführung ebenfalls aus dem Dienon durch Umsetzung mit Dimethylsulfoxoniummethylid, wobei hier allerdings ein Gemisch der α- und β-Isomeren auftritt (das Verhältnis ist abhängig von den verwendeten Substraten und liegt bei ca. 1:1), die z.B. über Säulenchromatographie getrennt werden können.

Verbindungen mit R⁷ gleich Alkyl oder SR²⁰, wobei R²⁰ die genannten Bedeutungen hat, werden aus den 4,6-Dien-3-on-Verbindungen durch 1,6-Addition nach bekannten Methoden hergestellt (J. Fried, J.A. Edwards: "Organic Reactions in Steroid Chemistry", von Nostrand Reinhold Company 1972, Seite 75 bis 82; und A. Hosomi, H. Sakurai, J. Am. Chem. Soc. **99** (1977), S. 1673).

Die Einführung der 7-Alkylfunktionen erfolgt hierbei in der Regel über die Dialkylkupferlithium-Verbindungen. Die Einführung einer SR²⁰-Gruppe erfolgt durch 1,6-Addition von Thioessigsäure. Hierbei entstehen gewöhnlich Gemische der Stereoisomeren, wobei eine Beeinflussung dieser Reaktion im Falle der Thioessigsäureaddition durch Zusatz von Lewissäuren, wie z.B. Bortrifluoretherat in Tetrahydrofuran als Lösungsmittel, zu einer drastischen Erhöhung der 7α-Isomeren führt.

Verbindungen, in denen R^{6a} ein Chloratom darstellt und R^{6b} und R⁷ gemeinsam eine zusätzliche Bindung bilden, werden ebenfalls ausgehend von den 4,6-Dien-3-on-Verbindungen dargestellt. Hierzu wird zunächst die 6,7-Doppelbindung unter Verwendung organischer Persäuren, wie z.B. meta-Chlorperbenzoesäure in Methylenchlorid, gegebenenfalls in Gegenwart von Natriumhydrogencarbonatlösung (siehe W. Adam et al., J. Org. Chem. **38** (1973), S. 2269) epoxidiert. Die Öffnung dieses Epoxides und die Eliminierung der primär gebildeten 7α-Hydroxygruppe erfolgt z.B. durch Umsetzung mit Chlorwassestoffgas in Eisessig (siehe u.a. DE-A-11 58 966 und DE-A 40 06 165).
Die beiden Reaktionen können aber auch nacheinander durchgeführt werden, indem das Epoxid zunächst mit z.B. Alkalimetallhalogeniden (z.B. LiCl) in Lösungsmitteln wie Essigsäure nucleophil geöffnet wird und die gebildete 7α-Hydroxygruppe dann nach Überführung in eine Fluchtgruppe (z.B. das Mesylat oder Tosylat) eliminiert wird.

Die Einführung einer 6-Methylengruppe kann z.B. ausgehend von einem 3-Amino-3,5-dien-Derivat durch Umsetzung mit Formalin in alkoholischer Lösung unter Bildung einer 6α-Hydroxymethylgruppe und anschließender saurer Wasserabspaltung z.B. mit Salzsäure in Dioxan/Wasser erfolgen. Die Wasserabspaltung kann aber auch in der Weise erfolgen, daß zunächst eine Fluchtgruppe eingeführt und dann eliminiert wird. Als Fluchtgruppen sind z.B. das Mesylat, Tosylat oder Benzoat geeignet (siehe DE-A-34 02 329 A1; EP-A-o 150 157; US-Patent 4,584,288 (86); K. Nickisch et al., J. Med. Chem. **34**, 2464 (1991)).

Eine weitere Möglichkeit zur Einführung der 6-Methylenverbindungen besteht in der direkten Umsetzung der 4(5)-ungesättigten 3-Ketone mit Acetalen des Formaldehyds in Gegenwart von Natriumacetat mit z.B. Phosphoroxychlorid oder Phosphorpentachlorid in geeigneten Lösungsmitteln wie Chloroform (siehe z.B. K. Annen, H. Hofmeister, H. Laurent und R. Wiechert, Synthesis 1982, S. 34).

Die 6-Methylenverbindungen können zur Darstellung von Verbindungen der allgemeinen Formel I, in denen R^{6a} gleich Methyl ist und R^{6b} und R⁷ eine gemeinsame zusätzliche Bindung bilden, genutzt werden.
Hierzu kann man z.B. ein von D. Burn et al. in Tetrahydron **21** (1965), S. 1619 beschriebenes Verfahren nutzen, bei dem eine Isomerisierung der Doppelbindung durch Erwärmen der 6-Methylenverbindungen in Ethanol mit 5%-Palladium-Kohle-Katalysator, der entweder mit Wasserstoff oder durch Erwärmen mit einer geringen Menge Cyclohexen vorbehandelt wurde, erzielt wird. Die Isomerisierung kann auch mit einem nicht vorbehandelten Katalysator erfolgen, wenn zur Reaktionsmischung eine geringe Menge Cyclohexen zugesetzt wird. Das Auftreten geringer Anteile hydrierter Produkte kann durch Zugabe eines Überschusses an Natriumacetat verhindert werden.
Die Darstellung von 6-Methyl-4,6-dien-3-on-Derivaten kann aber auch direkt erfolgen (siehe K. Annen, H. Hofmeister, H. Laurent und R. Wiechert, Lieb. Ann. 1983, S. 712).

Verbindungen in denen R^{6a} eine α-Methylfunktion darstellt, können aus den 6-Methylenverbindungen durch Hydrierung unter geeigneten Bedingungen dargestellt werden. Die besten Ergebnisse (selektive Hydrierung der exo-Methylenfunktion) werden durch Transfer-Hydrierung erreicht (E.A. Brande, R.P. Linstead und P.W.D. Mitchell, J. Chem. Soc. 3578 (1954). Erhitzt man die 6-Methylenderivate in einem geeigneten Lösungsmittel, wie z.B. Ethanol, in Gegenwart eines Wasserstoffdonators, wie z.B. Cyclohexen, so kommt man in sehr guten Ausbeuten zu 6α-Methylderivaten. Geringe Anteile an 6β-Methylverbindungen können sauer isomerisiert werden (siehe z.B. D. Burn, D.N. Kirk und V. Petrow, Tetrahedron, (1965), S. 1619).

Auch die gezielte Darstellung von 6β-Alkylverbindungen ist möglich. Hierfür werden die 4(5)-ungesättigten 3-Ketone z.B. mit Ethylenglycol, Trimethylorthoformiat in Dichlormethan in Gegenwart katalytischer Mengen einer Säure, (z.B. p-Toluolsulfonsäure) zu den entsprechenden 3-Ketalen umgesetzt. Während dieser Ketalisierung isomerisiert die Doppelbindung in die Position 5(6). Eine selektive Epoxidierung dieser 5(6)-Doppelbindung gelingt z.B. durch Verwendung organischer Persäuren in geeigneten Lösungsmitteln wie etwa Dichlormethan. Alternativ hierzu kann die Epoxidierung auch mit Wasserstoffperoxid in Gegenwart von z.B. Hexachloraceton oder 3-Nitrotrifluoracetophenon erfolgen. Die gebildeten 5,6α-Epoxide können dann unter Verwendung von z.B. Alkylmagnesiumhalogeniden oder Alkyllithiumverbindungen axial geöffnet werden. Man gelangt so zu 5α-Hydroxy-6β-Alkylverbindungen. Die Spaltung der 3-Ketoschutzgruppe kann unter Erhalt der 5α-Hydroxyfunktion durch Behandeln unter milden sauren Bedingungen (Essigsäure oder 4 n Salzsäure bei 0°C) erfolgen.Basische Eliminierung der 5α-Hydroxyfunktion mit z.B. verdünnter wäßriger Natronlauge ergibt die 3-Keto-4-en-Verbindungen mit einer β-ständigen 6-Alkylgruppe. Alternativ hierzu ergibt die Ketalspaltung unter drastischeren Bedingungen (wäßrige Salzsäure oder eine andere starke Säure) die entsprechenden 6α-Alkylverbindungen.

Die erhaltenen Verbindungen der allgemeinen Formel I mit W in der Bedeutung eines Sauerstoffatoms können gewünschtenfalls durch Umsetzung mit Hydroxylaminhydrochlorid in Gegenwart von tert. Aminen bei Temperaturen zwischen -20 und +40°C in die Oxyme überführt werden (allgemeine Formel I mit X in der Bedeutung von 〉N~OH, wobei die Hydroxygruppe syn- oder antiständig sein kann).

Die Entfernung der 3-Oxogruppe zu einem Endprodukt der allgemeinen Formel I mit W in der Bedeutung von zwei Wasserstoffatomen kann beispielsweise nach der in DE-A 28 05 490 angegebenen Vorschrift durch reduktive Spaltung des Thioketals erfolgen.

Zum Gegenstand der vorliegenden Erfindung gehören auch die Zwischenverbindungen der allgemeinen Formel III' 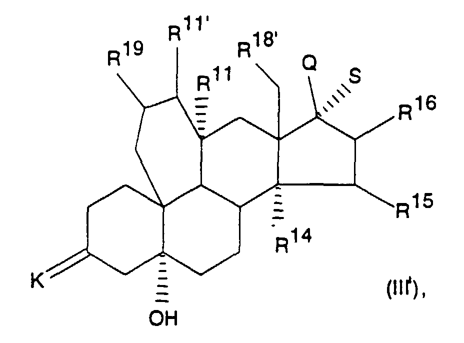 worin
K für eine Ketoschutzgruppe oder eine geschützte Hydroxygruppe und ein Wasserstoffatom,
R^{11'} und R¹⁹ gemeinsam für eine zusätzliche Bindung und R¹¹ für ein α-ständiges Wasserstoffatom oder R¹⁹ für ein Wasserstoffatom und R¹¹ und R^{11'} gemeinsam für eine zusätzliche Bindung oder R¹¹, R^{11'} und R¹⁹ je für ein Wasserstoffatom,
R¹⁴, R¹⁵ und R¹⁶ für die hierfür in Formel I angegebenen Substituenten,
R¹⁸ für ein Wasserstoffatom oder eine Methylgruppe sowie
Q für eine β-ständige Hydroxygruppe und S für ein α-ständiges Wasserstofftom oder Q und S gemeinsam für ein Ketosauerstoffatom oder außerdem
Q und S für eine der in Formel I genannten R^{17β}/R^{17α}-Substituentenkombinationen einschließlich der Spiroverbindungen, wobei darin vorhandene Hydroxy- und /oder Ketogruppen gegebenenfalls geschützt sind, stehen.

Diese Verbindungen haben bereits die Cyclisierungsreaktion durchlaufen und können in 17-Stellung noch die ursprüngliche Hydroxygruppe, bereits die Ketogruppe oder das endgültige R^{17α}/R^{17β}-Substituentenmuster aufweisen.

Die nachfolgenden Beispiele dienen der näheren Erläuterung der vorliegenden Erfindung:

### Beispiele:

### Generelle Anmerkungen:

I) Alle Versuche werden unter Schutzgasatmosphäre (Argon) durchgeführt
II) Falls nicht anders angegeben, erfolgt die Aufarbeitung der Experimente wie folgt: Die Reaktionslösung wird entweder auf gesättigte wäßrige Natriumchloridlösung (A), gesättigte wäßrige Natriumhydrogencarbonatlösung (B) oder gesättigte wäßrige Ammoniumchloridlösung (C) gegossen. Anschließend wird mehrmals mit Ethylacetat extrahiert. Die vereinigten organischen Phasen werden entweder mit gesättigter wäßriger Ammoniumchloridlösung (D) oder gesättigter wäßriger Natriumhydrogencarbonatlösung (E) sowie mit gesättigter wäßriger Natriumchloridlösung (F) gewaschen und über Natriumsulfat getrocknet. Danach wird filtriert und im Vakuum eingeengt.
III) Falls nicht anders angegeben, werden die erhaltenen Rohprodukte durch Säulenchromatographie an Kieselgel mit einem Gemisch aus Hexan/Ethylacetat gereinigt.
IV) Allgemeine Vorschriften:
   1) Spaltung von C-3-Ketalen gegebenenfalls mit Eliminierung einer 5α-Hydroxygruppe sowie Spaltung einer eventuell vorhandenen Tetrahydropyranyl-Schutzgruppe in der 17α-Seitenkette:
      5 mmol Ausgangsmaterial und 5 ml 4 normale Salzsäure werden in 60 ml Aceton gelöst. Man läßt eine Stunde bei Raumtemperatur sowie 30 Minuten bei 40°C nachrühren. Anschließende wäßrige Aufarbeitung (A,E,F) und Aufreinigung ergibt das entsprechende 3-Keton.
   2) Addition einer Ethinyl-, Propinyl- oder 1-Butinylseitenkette an C-17:
      100 ml absolutes Tetrahydrofuran werden bei 0°C über 30 Minuten mit Ethin- oder Propingas gesättigt; für die Addition der 1-Butinylseitenkette leitet man 5 g 1-Butin in 100 ml Tetrahydrofuran ein. Anschließend addiert man 31 ml einer 1,6 molaren Lösung vom n-Butyllithium in Hexan und läßt 30 Minuten bei 0°C nachrühren. Danach wird eine Lösung des entsprechenden Ausgangsmaterials (5 mmol) in absolutem Tetrahydrofuran addiert. Man läßt eine Stunde bei 0°C nachrühren und arbeitet dann wäßrig (C,F) auf.
   3) Oxidation mit Chromtrioxid/Pyridin:
      Zu 10 ml Pyridin in 80 ml Dichlormethan werden bei 0°C 30 mmol Chromtrioxid addiert. Man rührt 30 Minuten bei 0°C nach und addiert dann bei 0°C 5 mmol des Ausgangsmaterials in 15 ml Dichlormethan. Anschließend läßt man eine Stunde bei 0°C nachrühren. Danach wird die Reaktionslösung abdekantiert und der Rückstand dreimal mit Dichlormethan gewaschen. Die vereinigten organischen Phasen werden zweimal mit 5%iger wäßriger Natriumhydroxidlösung und einmal mit gesättigter wäßriger Natriumchloridlösung gewaschen und über Natriumsulfat getrocknet. Danach filtriert man und engt im Vakuum ein.
   4) Einführung einer 15(16)-Doppelbindung aus einem gesättigten 17-Keton via Silylenolether-Bildung und anschließende Saegusa-Oxidation
      a) Darstellung des Silylenolethers:
         Aus 15 mmol Diisopropylamin in 100 ml absolutem Tetrahydrofuran und 9 ml einer 1,5 molaren Lösung von n-Butyllithium in Hexan wird bei -30°C Lithiumdiisopropylamid hergestellt. Danach addiert man eine Lösung von 5 mmol des jeweiligen 17-Ketons in 50 ml absolutem Tetrahydrofuran und läßt eine Stunde bei -30°C nachrühren. Anschließend werden 17 mmol Trimethylchlorsilan addiert. Danach läßt man das Reaktionsgemisch auf Raumtemparatur kommen und rührt eine Stunde nach. Durch wäßrige Aufarbeitung (B,D,F) wird der entsprechende Silylenolether erhalten.
      b) Einführung der 15(16)-Doppelbindung:
         5 mmol des unter a) beschriebenen Silylenolethers werden in 60 ml Acetonitril gelöst. Man addiert 5,5 mmol Palladium(II)-acetat und läßt 8 Stunden bei Raumtemperatur nachrühren. Anschließend wird die Reaktionslösung über Celite filtriert und im Vakuum eingeengt.

### Beispiel 1

### 9,11α-Dihydro-17β-hydroxy-17α-methyl-6'H-benzo[10,9,11]estr-4-en-3-on

### a) 3,3-[2,2-Dimethyl-1,3-propandiylbis(oxy)]-19-ethinyl-5α-androst-9(11)-en-5,17β-diol

Zu einer Suspension von 48,6 g Magnesiumspänen in 700 ml absolutem Diethylether werden 3 g Quecksilber(II)-chlorid gegeben. Man rührt 30 Minuten nach und kühlt dann auf 0°C. Anschließend werden zunächst 7,5 ml 3-Brompropin addiert. Nach dem Anspringen der Reaktion (Temperaturanstieg) wird auf -5°C gekühlt. Dann werden weitere 67,5 ml 3-Brompropin mit einer solchen Geschwindigkeit hinzugetropft, daß die Innentemperatur nicht 0°C übersteigt. Nach vollständiger Zugabe wird 30 Minuten bei 0°C nachgerührt und dann eine Lösung von 50 g 3,3-[2,2-Dimethyl-1,3-propandiylbis-(oxy)]-5,10α-epoxy-5α-estr-9(11)-en-17β-ol in 300 ml absolutem Tetrahydrofuran langsam hinzugetropft. Man läßt eine Stunde bei 0°C nachrühren und dekantiert dann überschüssiges Magnesium ab. Anschließend addiert man vorsichtig 500 ml gesättigte wäßrige Ammoniumchloridlösung und läßt eine Stunde bei Raumtemperatur rühren (starke Gasentwicklung). Danach wird wäßrig aufgearbeitet (F). Nach Umkristallisation des Rohproduktes aus Diisopropylether werden 45,2 g 1.a) erhalten.

¹H-NMR (CDCl₃): δ= 5,42 ppm m (1H,H-11); 4,40 s (1H,OH); 3,75 dd (J=14, 7.5Hz, 1H,H-17); 3,40-3,60 m (4H,Ketal); 1,93 t (J=1.5 Hz,1H,Ethin); 0,99 s (3H,Me-Ketal); 0,92 s (3H, Me-Ketal); 0,76 s (3H,C-18)

### b) 19-(Bromethinyl)-3,3-[2,2-dimethyl-1,3-propandiylbis(oxy)]-5α-androst-9(11)-en-5,17β-diol

45 g der unter 1a) beschriebenen Substanz werden in 750 ml Aceton gelöst. Man addiert 1,85 g Silbernitrat und 23,3 g N-Bromsuccinimid. Anschließend wird 20 Minuten bei Raumtemperatur nachgerührt. Wäßrige Aufarbeitung (B,F) ergibt 52,5 g 1b), welches ohne Reinigung in die Folgestufe eingesetzt wird.

### c) 19-(2-Bromethenyl)-3,3-[2,2-dimethyl-1,3-propandiylbis(oxy)]-5α-androst-9(11)-en-5,17β-diol

52,5 g der unter 1b) beschriebenen Substanz werden in 1000 ml eines Gemisches aus Tetrahydrofuran und Wasser (1:1) gelöst. Man addiert 79 g p-Toluolsulfonsäurehydrazid und 52 g Natriumacetat. Anschließend wird 4 Stunden unter Rückfluß gekocht. Wäßrige Aufarbeitung (B,F) sowie Aufreinigung ergibt 36,9 g 1c) als weißen Schaum.
¹H-NMR (CDCl₃): δ= 6,18 ppm m (1H, Vinyl); 5,85 m (1H, Vinyl); 5,43 m (1H, H-11); 4,40 s (1H,OH); 3,73 dd (J=14, 7.5 Hz,1H,H-17); 3,48-3,58 m (4H, Ketal); 1,00 s (3H, Me-Ketal); 0,98 s (3H, Me-Ketal); 0,70 s (3H,C-18)

### d) 9,11α-Dihydro-3,3-[2,2-dimethyl-1,3-propandiylbis(oxy)]-6'H-benzo[10,9,11]-5α-estran-5,17β-diol

### Darstellung unter Verwendung von Tributylzinnhydrid:

20 g der unter 1c) beschriebenen Substanz werden in 500 ml absolutem Toluol gelöst. Man addiert 12 ml Tributylzinnhydrid und 25 mg Azobisisobutyronitril und kocht eine Stunde unter Rückfluß bei gleichzeitiger Bestrahlung mit einer UV-Lampe. Nach vollständiger Umsetzung wird die Reaktionslösung im Vakuum eingeengt und der Rückstand durch Umkristallisation aus Diisopropylether gereinigt. Man erhält 9,1 g 1d) als weiße Kristalle. Durch Säulenchromatographie der Mutterlauge an Kieselgel mit einem Gemisch aus Hexan/Ethylacetat werden weitere 3,84 g 1d) als weißer Schaum erhalten.
¹H-NMR (CDCl₃): δ= 5,50 ppm dbr (J=10 Hz,1H, Bügel); 5,47 m (1H, Bügel); 4,37 s (1H, OH); 3,50-3,62 m (5H, Ketal und H-17); 2,47 m (1H,H-11); 0,99 s (3H, Me-Ketal); 0,98 s (3H, Me-Ketal); 0,80 s (3H, C-18)

### Darstellung unter Birchbedingungen:

Zu 400 ml kondensiertem Ammoniak werden bei -78°C langsam 3,5 g Lithium addiert. Nach vollständiger Auflösung wird eine Lösung von 5 g der unter 1c) beschriebenen Substanz in 600 ml absolutem Tetrahydrofuran hinzugetropft. Nach vollständiger Zugabe wird 15 Minuten bei -40°C nachgerührt. Danach quencht man die Reaktionslösung durch Zugabe von Wasser. Man läßt den Ammoniak über Nacht abziehen und arbeitet wäßrig (F) auf. Nach Aufreinigung werden neben 1,9 g 1d) 1,6 g 3,3-[2,2-Dimethyl-1,3-propandiylbis(oxy)]-19-ethenyl-5α-androst-9(11)-en-5,17β-diol erhalten.

### 1e) 9,11α-Dihydro-3,3-[2,2-dimethyl-1,3-propandiylbis(oxy)]-5-hydroxy-6'H-benzo-[10,9,11]-5α-estran-17-on

### 1. Darstellung aus 1d)

Gemäß der allgemeinen Vorschrift 3) werden aus 12,9 g der unter 1d) beschriebenen Substanz, 18,5 g Chromtrioxid und 62 ml Pyridin in 450 ml Dichlormethan 11,34 g 1e) als weißer Schaum erhalten.

### 2. Alternative Darstellung von 1e)

### 1e1) 3,3-[2'2-Dimethyl-1,3-propandiylbis(oxy)]-19-ethinyl-5-hydroxy-5α-androst-9(11)-en-17-on

Gemäß der allgemeinen Vorschrift 3 werden 5 g der unter 1a) beschriebenen Substanz umgesetzt. Man erhält 4,82 g 1e1) als weißen Schaum.
¹H-NMR (CDCl₃): δ= 5,43 ppm dbr (J=5.5 Hz, 1H, H-11); 4,40 s (1H, OH); 3,40-3,60 m (4H, Ketal); 1,92 t (J=1.5 Hz, 1H, Ethin); 1,02 s (3H, C-18); 0,95 s (3H, Me-Ketal); 0,91 s (3H, Me-Ketal)

### 1e2) 19-(2-Bromethinyl)-3,3-[2,2-dimethyl-1,3-propandiylbis(oxy)]-5-hydroxy-5α-androst-9(11)-en-17-on

Analog zu 1b) werden 4,82 g der unter 1e1) beschriebenen Substanz mit 5,5 g N-Bromsuccinimid und 200 mg Silbernitrat in 100 ml Aceton umgesetzt. Man erhält 5,7 g 1e2), welches als Rohprodukt in die Folgestufe eingesetzt wird.

### 1e3) 19-(2-Bromethenyl)-3,3-[2,2-dimethyl-1,3-propandiylbis(oxy)]-5-hydroxy-5α-androst-9(11)-en-17-on

Analog zu Beispiel 1c) werden 5,7 g der unter 1e2) beschriebenen Substanz mit 9 g p-Toluolsulfonsäurehydrazid und 6 g Natriumacetat in 100 ml eines Gemisches aus Tetrahydrofuran und Wasser (1:1) umgesetzt. Man erhält nach Aufreinigung 3 g 1e3) als weißen Schaum.
¹H-NMR (CDCl₃): δ= 6,19 m (1H, Vinyl); 5,83 m (1H, Vinyl); 5,33 dbr (J=5.5 Hz, 1H, H-11); 4,40 s (1H, OH); 3,45-3,60 m (4H, Ketal); 1,00 s (3H, C-18); 0,95 s (3H, Me-Ketal); 0,82 s (3H, Me-Ketal)

### 1e) 9,11α-Dihydro-3,3-[2,2-dimethyl-1,3-propandiylbis(oxy)]-5-hydroxy-6'H-benzo-[10,9,11]-5α-estran-17-on

Analog zu 1d) werden 3 g der unter 1e3) beschriebenen Substanz mit 3 ml Tributylzinnhydrid und 25 mg Azobisisobutyronitril in 100 ml absolutem Toluol umgesetzt. Man erhält nach Aufreinigung 2,39 g 1e) als weißen Schaum.
¹H-NMR (CDCl₃): δ= 5,57 ppm dbr (J=10 Hz,1H, Bügel); 5,49 m (1H, Bügel); 4,40 s (1H, OH); 3,50-3,60 m (4H, Ketal); 2,53 m (1H, H-11); 1,00 s (3H, Me-Ketal); 0,98 s (3H, Me-Ketal); 0,92 s (3H, C-18)

### 1f) 9,11α-Dihydro-3,3-[2,2-dimethyl-1,3-propandiylbis(oxy)]-17α-methyl-6'H-benzo-[10,9,11]-5α-estran-5,17β-diol

Zu 13 ml einer 1,6 molaren Lösung von Methyllithium in Diethylether wird bei 0°C unter Argon eine Lösung von 882 mg der unter 1e) hergestellten Verbindung in 20 ml absolutem Tetrahydrofuran addiert. Man rührt 2 Stunden bei 0°C nach und arbeitet danach wäßrig (C,F) auf. Man erhält 845 mg 1f) als weißen Schaum, der ohne Reinigung in die Folgestufe eingesetzt wird.

### 1g) 9,11α-Dihydro-17β-hydroxy-17α-methyl-6'H-benzo[10,9,11]estr-4-en-3-on

Gemäß der allgemeinen Vorschrift 1) werden 839 mg 1f) mit 4 normaler Salzsäure in Aceton umgesetzt. Man erhält nach Aufreinigung 594 mg 1g) als weißen Schaum.
¹H-NMR (CDCl₃): δ= 5,81 ppm sbr (1H,H-4); 5,61 dbr (J=10 Hz,1H, Bügel); 5,50 m (1H, Bügel); 2,69 m (1H, H-11); 1,23 s (3H, Methyl); 0,95 s (3H,C-19)

### Beispiel 2

### 9,11α-Dihydro-17α-ethinyl-17β-hydroxy-6'H-benzo[10,9,11]estr-4-en-3-on

### a) 9,11α-Dihydro-3,3-[2,2-dimethyl-1,3-propandiylbis(oxy)]-17α-ethinyl-6'H-benzo-[10,9,11]-5α-estran-5,17β-diol

Gemäß der allgemeinen Vorschrift 2) werden 1,24 g der unter le) beschriebenen Substanz und 18,9 ml einer 1,6 molaren Lösung von n-Butyllithium in Hexan sowie Ethingas in absolutem Tetrahydrofuran umgesetzt. Man erhält nach Aufreinigung 1,20 g 2a) als weißen Schaum.
¹H-NMR (CDCl₃): δ= 5,55 ppm dbr (J=10 Hz, 1H, Bügel); 5,48 m (1H, Bügel); 4,40 s (1H, OH); 3,50-3,62 m (4H, Ketal); 2,59 s (1H, Ethin); 2,52 m (1H, H-11); 0,98 s (3H, Me-Ketal); 0,97 s (3H, Me-Ketal); 0,90 s (3H, C-18)

### b) 9,11α-Dihydro-17α-ethinyl-17β-hydroxy-6'H-benzo[10,9,11]estr-4-en-3-on

Gemäß der allgemeinen Vorschrift 1) werden 1,2 g 2a) mit 3 ml 4 normaler Salzsäure in Aceton umgesetzt. Man erhält nach Kristallisation des Rohproduktes aus Ethylacetat 776 mg 2b) als weiße Kristalle.
¹H-NMR (CDCl₃): δ= 5,81 ppm sbr (1H, H-4); 5,62 dbr (J=10 Hz, 1H, Bügel); 5,56 m (1H, Bügel); 2,67 m (1H, H-11); 2,59 s (1H, Ethin); 0,91 s (3H, C-18)

### Beispiel 3

### 9,11α-Dihydro-17β-hydroxy-17α-(1-propinyl)-6'H-benzo[10,9,11]estr-4-en-3-on

### a) 9,11α-Dihydro-3,3-[2,2-dimethyl-1,3-propandiylbis(oxy)]-17α-(1-propinyl)-6'H-benzo[10,9,11]-5α-estran-5,17β-diol

Gemäß der allgemeinen Vorschrift 2) werden 1 g der unter 1e) beschriebenen Verbindung und 15,1 ml einer 1,6 molaren Lösung von n-Butyllithium in Hexan sowie Propingas in absolutem Tetrahydrofuran umgesetzt. Man erhält 1,05 g 3a) als weißen Schaum, welcher ohne Reinigung in die Folgestufe eingesetzt wird.

### 3b) 9,11α-Dihydro-17β-hydroxy-17α-(1-propinyl)-6'H-benzo[10,9,11]estr-4-en-3-on

Gemäß der allgemeinen Vorschrift 1) werden 1 g der unter 3a) beschriebenen Substanz mit 2,5 ml 4 normaler wäßriger Salzsäure in Aceton umgesetzt. Man erhält nach Kristallisation des Rohproduktes aus Diisopropylether 600 mg 3b) als weiße Kristalle.
Fp= 161,6°C; [α]_{D}²⁰=-53,4°(CHCl₃; c=0,520)
¹H-NMR (CDCl₃): δ= 5,81 ppm sbr (1H,H-4); 5,60 dbr (J=10 Hz,1H, Bügel); 5,50 m (1H, Bügel); 2,68 m (1H, H-11); 1,87 s (3H, Propin); 0,90 s (3H, C-18)

### Beispiel 4

### (Z)-9,11α-Dihydro-17β-hydroxy-17α-(3-hydroxy-1-propenyl)-6'H-benzo[10,9,11]estr-4-en-3-on

### a) 9,11α-Dihydro-3,3-[2,2-dimethyl-1,3-propandiylbis(oxy)]-17α-[3-[(tetrahydro-2H-pyran-2-yl)oxy]-1-propinyl]-6'H-benzo[10,9,11]-5α-estran-5,17β-diol

Aus 17 ml 3-[(Tetrahydro-2H-pyran-2-yl)oxy]-1-propin in 600 ml absolutem Tetrahydrofuran und 75,4 ml einer 1,6 molaren Lösung von n-Butyllithium in Hexan stellt man bei 0°C unter Argon die lithiumorganische Verbindung her. Anschließend wird eine Lösung von 5 g der unter 1e) beschriebenen Substanz in 120 ml absolutem Tetrahydrofuran addiert. Man läßt eine Stunde bei 0°C nachrühren und arbeitet dann wäßrig (C,F) auf. Man erhält nach Aufreinigung 5,75 g 4a) als weißen Schaum.
¹H-NMR (CDCl₃): δ= 5,56 ppm dbr (J=10 Hz,lH, Bügel); 5,48 m (1H, Bügel); 4,84 m (1H, THP); 4,40 s (1H,OH); 4,32 m (2H, CH₂OTHP); 3,87 m (1H, THP); 3,50-3,70 m (5H, Ketal und THP); 2,50 m (1H, H-11); 0,95 m (6H, Me-Ketal); 0,89 s (3H, C-18)

### b) (Z)-9,11α-Dihydro-3,3-[2,2-dimethyl-1,3-propandiylbis(oxy)]-17α-[3-[(tetrahydro-2H-pyran-2-yl)oxy]-1-propenyl]-6'H-benzo[10,9,11]-5α-estran-5,17β-diol

Zu einer Lösung von 5,4 g der unter 4a) beschriebenen Substanz in 45 ml Tetrahydrofuran werden 549 mg Palladium auf Bariumsulfat (10%ig) addiert. Man setzt die Apparatur unter Wasserstoff und läßt 20 Minuten rühren. Anschließend wird die Reaktionsmischung über Celite filtriert und im Vakuum eingeengt. Man erhält nach Aufreinigung 4,56 g 4b) als weißen Schaum.
¹H-NMR (CDCl₃): δ= 5,73 ppm dbr (J=10 Hz,1H, Bügel); 5,55 m (2H); 5,47 m (1H, Bügel); 4,71 m (1H, THP); 4,38 m (2H, CH₂OTHP); 3,85 m (1H, THP); 3,50-3,65 m (5H, Ketal und THP); 2,45 m (1H, H-11); 0,99 s (3H, Me-Ketal); 0,98 s (3H, Me-Ketal); 0,94 s (3H, C-18)

### c) (Z)-9,11α-Dihydro-17β-hydroxy-17α-(3-hydroxy-1-propenyl)-6'H-benzo[10,9,11]-estr-4-en-3-on

Gemäß der allgemeinen Vorschrift 1) werden 1 g der unter 4b) beschriebenen Substanz mit 4 normaler Salzsäure in Aceton umgesetzt. Man erhält nach Kristallisation des Rohproduktes aus Diisopropylether 440 mg 4c) als weiße Kristalle.
Fp= 219-221°C; [α]_{D}²⁰= 21,3° (CHCl₃, c=0,535)
¹H-NMR (CDCl₃): δ= 5,81 ppm sbr (1H,H-4); 5,70 m (1H); 5,62 dbr (J=10 Hz, 1H, Bügel); 5,59 m (2H); 4,26 m (2H, CH₂OH); 2,63 m (1H, H-11); 1,00 s (3H, C-18)

### Beispiel 5

### 9,11α-Dihydro-17β-hydroxy-17α-(3-hydroxypropyl)-6'H-benzo[10,9,11]estr-4-en-3-on

### a) 9,11α-Dihydro-3,3-[2,2-dimethyl-1,3-propandiylbis(oxy)]-17α-[3-[(tetrahydro-2H-pyran-2-yl)oxy]propyl]-6'H-benzo[10,9,11]-5α-estran-5,17β-diol

Zu einer Lösung von 3,56 g der unter 4b) beschriebenen Substanz in 175 ml Ethylacetat werden 360 mg Palladium auf Aktivkohle (10%ig) addiert. Man setzt die Apparatur unter Wasserstoff und läßt 1 Stunde bei Raumtemperatur nachrühren. Anschließend wird das Reaktionsgemisch über Celite filtriert und im Vakuum eingeengt. Das erhaltene Rohprodukt (3,49 g) wird ohne Reinigung in die Folgestufe eingesetzt.

### b) 9,11α-Dihydro-17β-hydroxy-17α-(3-hydroxypropyl)-6'H-benzo[10,9,11]estr-4-en-3-on

Gemäß der allgemeinen Vorschrift 1) werden 3,23 g 5a) mit 4 normaler Salzsäure in Aceton umgesetzt. Man erhält nach Aufreinigung 1,5 g 5b) als weißen Schaum.
[α]_{D}²⁰= -23,7° (CHCl₃; c=0,510)
¹H-NMR (CDCl₃): δ= 5,81 ppm sbr (1H, H-4); 5,63 dbr (J=10 Hz, 1H, Bügel); 5,50 m (1H, Bügel); 3,60-3,75 m (2H, CH₂OH); 2,68 m (1H, H-11); 0,96 s (3H, C-18)

### Beispiel 6

### 5",4",9,11α-Tetrahydrospiro[6'H-benzo[10,9,11]estr-4-en-17β,2"(5")-furan]-3,5"-dion

Gemäß der allgemeinen Vorschrift 3) werden 700 mg der unter 5b) beschriebenen Substanz, 1,1 g Chromtrioxid und 3,62 ml Pyridin in Dichlormethan umgesetzt. Man erhält nach Aufreinigung 485 mg 6) als weißen Schaum.
[α]_{D}²⁰=-31,4°(CHCl₃;c=0,515)
¹H-NMR (CDCl₃): δ= 5,82 ppm sbr (1H, H-4); 5,58 m (2H, Bügel); 2,71 m (1H, H-11); 1,02 s (3H, C-19)

### Beispiel 7

### 5",4",9,11α-Tetrahydrospiro[6'H-benzo[10,9,11]estr-4-en-17β,2"(5")-furan]-3-on

600 mg der unter 5b) beschriebenen Verbindung werden in 40 ml Dichlormethan gelöst. Es wird mit 3,3 ml Triethylamin versetzt. Man kühlt auf 0°C und addiert 770 mg p-Toluolsulfonsäurechlorid. Anschließend läßt man eine Stunde bei 0°C und 6 Stunden bei Raumtemperatur nachrühren und arbeitet dann wäßrig auf (B,F). Man erhält nach Aufreinigung 380 mg 7) als weißen Schaum.
¹H-NMR (CDCl₃): δ= 5,82 ppm sbr (1H, H-4); 5,62 dbr (J=10 Hz, 1H, Bügel); 5,55 m (1H, Bügel); 3,70-3,81 m (2H, Spiroether); 2,63 m (1H, H-11); 0,93 s (3H, C-18)
Fp= 127,3°C, [α]_{D}²⁰= -52,3° (CHCl₃; c=0,485)

### Beispiel 8

### 9,11α-Dihydro-17β-hydroxy-17α-(1,3-pentadiinyl)-6'H-benzo[10,9,11]estr-4-en-3-on

700 mg der unter 2b) beschriebenen Substanz werden in 60 ml Triethylamin gelöst. Man sättigt die Lösung bei Raumtemperatur mit Propingas, addiert 250 mg Tetrakis(triphenylphosphin)palladium und 120 mg Kupfer(I)iodid, erwärmt auf 60°C und läßt unter Aufrechterhaltung des Propinstromes über eine Stunde bei dieser Temperatur nachrühren. Anschließend wird die Reaktionslösung über Celite filtriert und im Vakuum eingeengt Man erhält nach Aufreinigung 311 mg 8) als weißen Schaum.
¹H-NMR (CDCl₃): δ= 5,80 ppm sbr (1H, H-4); 5,58 m (2H, Bügel); 2,70 m (1H, H-11); 1,95 s (3H, Butinyl); 0,82 s (3H, Methyl)

### Beispiel 9

### 9,11α-Dihydro-17α-ethinyl-17β-hydroxy-6'H-benzo[10,9,11]estra-4,15-dien-3-on

### a) 9,11α-Dihydro-3,3-[2,2-dimethyl-1,3-propandiylbis(oxy)]-17-[(trimethylsilyl)oxy]-6'H-benzo[10,9,11]-5α-estr-16-en-5-ol

Gemäß der allgemeinen Vorschrift 4a) werden 2,7 g der unter 1e) beschriebenen Substanz mit 3,2 ml Diisopropylamin, 14,4 ml einer 1,6 molaren Lösung von n-Butyllithium in Hexan und 4 ml Trimethylchlorsilan in absolutem Tetrahydrofuran umgesetzt. Man erhält nach Umkristallisation des Rohproduktes aus Acetonitril 2,5 g 9a).

### b) 9,11α-Dihydro-3,3-[2,2-dimethyl-1,3-propandiylbis(oxy)]-5-hydroxy-6'H-benzo-[10,9,11]-5α-estr-15-en-17-on

Gemäß der allgemeinen Vorschrift 4b) werden 2,5 g der unter 9a) beschriebenen Verbindung mit 1,3 g Palladium(II)acetat in Acetonitril umgesetzt. Man erhält nach Aufreinigung 1,8 g 9b).
¹H-NMR (CDCl₃): δ= 7,52 ppm dbr (J=6 Hz, 1H, H-15); 5,98 dd (J=6, 3 Hz, 1H, H-16); 5,60 dbr (J=10 Hz, 1H, Bügel); 5,50 m (1H, Bügel); 4,47 s (3H, OH); 3,50-3,60 m (4H, Ketal); 2,60 m (1H, H-11); 1,18 s (3H, C-18); 1,00 s (3H, Me-Ketal); 0,98 s (3H, Me-Ketal)

### c) 9,11α-Dihydro-3,3-[2,2-dimethyl- 1 ,3-propandiylbis(oxy)]-17α-ethinyl-6'H-benzo-[10,9,11]-5α-estr-15-en-5,17β-diol

Gemäß der allgemeinen Vorschrift 2) werden 1,8 g 9b), 15 ml einer 1,6 molaren Lösung von n-Butyllithium in Hexan sowie Ethingas in absolutem Tetrahydrofuran umgesetzt. Man erhält 1,59 g 9c), welches ohne Reinigung in die Folgestufe eingesetzt wird.

### d) 9,11α-Dihydro-17α-ethinyl-17β-hydroxy-6'H-benzo[10,9,11]estra-4,15-dien-3-on

Gemäß der allgemeinen Vorschrift 1) werden 1,59 g 9c) mit 4 normaler Salzsäure in Aceton umgesetzt. Man erhält nach Aufreinigung 970 mg 9d).
¹H-NMR (CDCl₃): δ= 6,00 dbr (J=6 Hz, 1H, H-15); 5,72 sbr (1H, H-4); 5,21 dd (J=6, 3 Hz, 1H, H-16); 5,60 m (2H, Bügel); 2,77 m (1H, H-11); 2,60 s (1H, Ethin); 1,00 s (3H-C-18)
Fp= 198°C, [α]_{D}²⁰= -202,9°(CHCl₃ ; c=0,515)

### Beispiel 10

### 9,11α-Dihydro-17β-hydroxy-17α-(1-propinyl)-6'H-benzo[10,9,11]estra-4,15-dien-3-on

### a) 9,11α-Dihydro-3,3-[2,2-dimethyl-1,3-propandiylbis(oxy)]-17α-(1-propinyl)-6'H-benzo[10,9,11]-5α-estr-15-en-5,17β-diol

Gemäß der allgemeinen Vorschrift 2) werden 1g 9b), 15 ml einer 1,6 molaren Lösung von n-Butyllithium in Hexan sowie Propingas in absolutem Tetrahydrofuran umgesetzt.
Man erhält 1 g 10a), welches ohne Reinigung in die Folgestufe eingesetzt wird.

### b) 9,11α-Dihydro-17β-hydroxy-17α-(1-propinyl)-6'H-benzo[10,9,11]estra-4,15-dien-3-on

Gemäß der allgemeinen Vorschrift 1) wird 1 g 10a) mit 4 normaler Salzsäure in Aceton umgesetzt. Man erhält nach Aufreinigung 610 mg 10b).
[α]_{D}²⁰= -204,2° (CHCl₃; c=0,520)
¹H-NMR (CDCl₃): δ= 5,94 dbr (J=6 Hz, 1H, H-15); 5,72 sbr (1H, H-4); 5,20 dd (J=6, 3 Hz, 1H, H-16); 5,60 m (2H, Bügel); 2,73 m (1H, H-11); 1,90 s (3H, Propin); 0,99 s (3H, C-18)

### Beispiel 11

### 9,11α-Dihydro-17β-hydroxy-17α-methyl-6'H-benzo[10,9,11]estra-4,14-dien-3-on

### a) 9,11α-Dihydro-3,3-[2,2-dimethyl-1,3-propandiylbis(oxy)]-5-hydroxy-6'H-benzo-[10,9,11]-5α-estr-14-en-17-on

2,3 g der unter 9b) beschriebenen Substanz werden in 300 ml eines Gemisches aus Ethylacetat und Hexan (9:1) gelöst. Man addiert 180 g Kieselgel und 35 ml Triethylamin und läßt 2 Tage bei Raumtemperatur nachrühren. Anschließend wird über Celite filtriert und eingeengt. Man erhält 1,02 g a) neben 1,15 g Ausgangsmaterial.
¹H-NMR (CDCl₃): δ= 5,65 ppm dbr (J= 10 Hz, 1H, Bügel); 5,55 m (1H, Bügel); 5,52 m (1H, H-15); 4,40 s (1H, OH); 3,50-3,62 m (4H, Ketal); 3,00 ddd (J=20, 3, 1 Hz, 1H, H-16); 2,80 dt (J=20, 1.5 Hz, 1H, H-16'); 2,51 m (1H, H-11); 1,12 s (3H, C-18); 1,00 s (3H, Me-Ketal); 0,98 s (3H, Me-Ketal)

### b) 9,11α-Dihydro-3,3-[2,2-dimethyl-1,3-propandiylbis(oxy)]-17α-methyl-6'H-benzo-[10,9,11]-5α-estr-14-en-5,17β-diol

2,5 g wasserfreies Certrichlorid werden zu 20 ml absolutem Tetrahydrofuran addiert. Man läßt die Suspension eine Stunde nachrühren, kühlt dann auf 0°C und addiert 3,34 ml einer 3 molaren Lösung von Methylmagnesiumchlorid in absolutem Tetrahydrofuran. Man läßt weitere 1,5 Stunden bei 0°C nachrühren und addiert dann eine Lösung von 413 mg der unter 11a) beschriebenen Substanz in 5 ml absolutem Tetrahydrofuran. Anschließend wird 30 Minuten bei 0°C nachgerührt und dann wäßrig (C,F) aufgearbeitet. Man erhält nach Aufreinigung 170 mg 11b).
¹H-NMR (CDCl₃): δ= 5,60 ppm dbr (J=10 Hz, 1H, Bügel); 5,52 m (1H, Bügel); 5,18 m (1H, H-15); 4,40 s (1H, OH); 3,50-3,62 m (4H, Ketal); 2,52 m (1H, H-11); 2,42 dtr (J= 10, 1 Hz, 1H, H-16); 2,30 dtr (J=20, 1Hz, 1H, H-16'); 1,22 s (3H, 17-Methyl); 1,10 s (3H, C-18); 0,98 s (6H, Me-Ketal)

### c) 9,11α-Dihydro-17β-hydroxy-17α-methyl-6'H-benzo[10,9,11]estra-4,14-dien-3-on

Gemäß der allgemeinen Vorschrift 1 werden 170 mg der unter 11b) beschriebenen Verbindung mit 4 normaler Salzsäure in Aceton umgesetzt. Man erhält nach Aufreinigung 100 mg 11c).
[α]_{D}²⁰= -38,0° (CHCl₃; c=0,505)
¹H-NMR (CDCl₃): δ= 5,83 sbr (1H, H-4); 5,68 dbr (J=10 Hz, 1H, Bügel); 5,62 m (1H-Bügel); 5,18 m (1H, H-15); 1,23 s (3H, 17-Methyl); 1,13 s (3H, C-18)

### Beispiel 12

### 9,11α-Dihydro-17β-hydroxy-17α-(1-propinyl)-6'H-benzo[10,9,11]estra-4,14-dien-3-on

### a) 9,11α-Dihydro-3,3-[2,2-dimethyl-1,3-propandiylbis(oxy)]-17α-(1-propinyl)-6'H-benzo-[10,9,11]-5α-estr- 14-en-5,17β-diol

Gemäß der allgemeinen Vorschrift 2) wird eine gesättigte Lösung von Propingas in 30 ml absolutem Tetrahydrofuran mit 6,25 ml einer 1,6 molaren Lösung von n-Butyllithium in Hexan umgesetzt. Man addiert diese Lösung zu einer Suspension von 2,5 g wasserfreiem Certrichlorid in 20 ml absolutem Tetrahydrofuran, welche analog zu Beispiel 11b) vorbehandelt wurde. Anschließend wird analog zu 11b) mit einer Lösung von 413 mg der unter 11a) beschriebenen Substanz in absolutem Tetrahydrofuran umgesetzt. Man erhält 420 mg Rohprodukt, welches ohne Aufreinigung in die Folgestufe eingesetzt wird.

### b) 9,11α-Dihydro-17β-hydroxy-17α-(1-propinyl)-6'H-benzo[10,9,11]estra-4,14-dien-3-on

Gemäß der allgemeinen Vorschrift 1) werden 420 mg 12a) mit 4 normaler Salzsäure in Aceton umgesetzt. Man erhält nach Aufreinigung 200 mg 12b) als weißen Schaum.
¹H-NMR (CDCl₃): δ= 5,82 ppm sbr (1H, H-4); 5,69 dbr (J=10 Hz, 1H, Bügel); 5,62 m (1H, Bügel); 5,15 m (1H, H-15); 1,88 s (3H, Propin); 1,15 s (3H, C-18)

### Beispiel 13

### 17-(Acetyloxy)-9,11α-dihydro-6'H-benzo[10,9,11]-19-norpregn-4-en-3,20-dion

### a) 9,11α-Dihydro-5,17α-dihydroxy-3,3-[2,2-dimethyl-1,3-propandiylbis(oxy)]-6'H-benzo[10,9,11]-5α-estran-17β-carbonitril

10 g der unter 1e) beschriebenen Substanz werden in 50 ml 2-Hydroxy-2-methylpropannitril zusammen mit 0,74 ml 20%iger wäßriger Natiumhydroxidlösung bei 80°C gelöst. Das nach dem Abkühlen ausgefallene Produkt wird abfiltriert und aus Diisopropylether umkristallisiert. Man erhält 8,6 g 13a) als weiße Kristalle.
¹H-NMR (CDCl₃): δ= 5,58 ppm dbr (J=10 Hz, 1H, Bügel); 5,50 m (1H, Bügel); 4,60 (1H, OH); 3,50-3,62 m (4H, Ketal); 2,59 m (1H, H-11); 1,00 s (3H, Me-Ketal); 0,97 s (3H, Me-Ketal); 0,96 s (3H, C-18)

### b) 9,11α-Dihydro-17-hydroxy-6'H-benzo[10,9,11]-19-norpregn-4-en-3,20-dion

Zu einer Lösung von 4 g der unter 13a) beschriebenen Verbindung in 100 ml Diethylether werden 350 mg p-Toluolsulfonsäure und 8,8 ml Ethoxyethen addiert. Man läßt 30 Minuten bei Raumtemperatur nachrühren und arbeitet danach wäßrig (B,F) auf. Das erhaltenen Rohprodukt wird erneut in 100 ml Diethylether gelöst. Man kühlt auf 0°C und addiert 16 ml einer 1,6 molaren Lösung von Methyllithium in Diethylether. Man läßt 3 Stunden bei 0°C nachrühren und arbeitet dann wäßrig (C,F) auf. Das erhaltene Rohprodukt wird gemäß der allgemeinen Vorschrift 1) umgesetzt. Man erhält nach Kristallisation aus Diisopropylether/Methanol 2,9 g 13b) als weiße Kristalle.
¹H-NMR (CDCl₃): δ= 5,81 ppm sbr (1H, H-4); 5,55 m (2H, Bügel); 2,27 s (3H, Acetyl); 0,71 s (3H, C-18)

### 13c) 17-(Acetyloxy)-9,11α-dihydro-6'H-benzo[10,9,11]-19-norpregn-4-en-3,20-dion

Zu einer Suspension von 2,9 g der unter 13b) beschriebenen Substanz in 40 ml Eisessig werden bei 0°C 15 ml Trifluoressigsäureanhydrid gegeben. Man läßt 4 Stunden bei Raumtemperatur nachrühren und arbeitet danach wäßrig (B,F) auf. Nach Aufreinigung erhält man 2,3 g 13c).
Fp= 223°C, [α]_{D}²⁰= -29,3° (CHCl₃; c=0,515)
¹H-NMR (CDCl₃): δ= 5,82 ppm sbr (1H, H-4); 5,56 m (2H, Bügel); 2,72 m (1H, H-11); 2,15 s (3H, Acetyl); 2,10 s (3H, Acetoxy); 0,70 s (3H, C-18)

### Beispiel 14

### 17-(Acetyloxy)-9,11α-dihydro-6'H-benzo[10,9,11]-19-norpregna-4,6-dien-3,20-dion

### a) 17-(Acetyloxy)-9,11α-dihydro-3-ethoxy-6'H-benzo[10,9,11]-19-norpregna-3,5-dien-20-on

Eine Mischung von 800 mg der unter 13c) beschriebenen Substanz, 2 ml Triethylorthoformiat, 2 ml Ethanol und 40 mg p-Toluolsulfonsäure in 20 ml Tetrahydrofuran wird eine Stunde bei 40°C gerührt. Anschließend wird wäßrig (B,F) aufgearbeitet. Das erhaltene Rohprodukt (850 mg) wird ohne Reinigung in die Folgestufe eingesetzt.

### b) 17-(Acetyloxy)-6β-brom-9,11α-dihydro-6'H-benzo[10,9,11]-19-norpregn-4-en-3,20-dion

Zu einer Lösung von 850 mg der unter 14a) beschriebenen Substanz in 10 ml Dioxan werden 4 ml 10%ige wäßrige Natriumacetatlösung und anschließend 285 mg 1,3-Dibrom-5,5-dimethylhydantoin addiert. Man läßt 5 Minuten bei 0°C nachrühren und arbeitet danach wäßrig (B,F) auf. Nach Aufreinigung werden 600 mg 14b) erhalten.
¹H-NMR (CDCl₃): δ= 6,02 ppm sbr (1H, H-4); 5,60 m (1H, Bügel); 5,52 dbr (J=10 Hz, 1H, Bügel); 5,05 dbr (J=4 Hz, 1H, H-6α); 2,28 m (1H, H-11); 2,15 s (3H, Acetyl); 2,10 s (3H, Acetoxy); 0,79 s (3H, C-18)

### c) 17-(Acetyloxy)-9,11α-dihydro-6'H-benzo[10,9,11]-19-norpregna-4,6-dien-3,20-dion

Eine Mischung von 600 mg der unter 14b) beschriebenen Substanz, 550 mg Lithiumbromid und 375 mg Lithiumcarbonat in 10 ml N,N-Dimethylformamid wird eine Stunde bei 100°C gerührt. Anschließend wird wäßrig aufgearbeitet (A,F). Man erhält nach Aufreinigung 435 mg 14c).
[α]_{D}²⁰= -35,5° (CHCl₃; c=0,520)
¹H-NMR (CDCl₃): δ= 6,13 ppm m (2H, H-6, H-7); 5,73 sbr (1H, H-4); 5,60 m (2H, Bügel); 2,79 m (1H, H-11); 2,15 s (3H, Acetyl); 2,10 s (3H, Acetoxy); 0,73 s (3H, C-18)

### Beispiel 15

### 17-(Acetyloxy)-6-chlor-9,11α-dihydro-6'H-benzo[10,9,11]-19-norpregna-4,6-dien-3,20-dion

### a) 17-(Acetyloxy)-9,11α-dihydro-6α,7α-epoxy-6'H-benzo[10,9,11]-19-norpregn-4-en-3,20-dion

Zu einer Lösung von 2 g der unter 14c) beschriebenen Verbindung in 50 ml Dichlormethan werden 1,8 g m-Chlorperbenzoesäure (70%ig) gegeben. Man läßt 8 Stunden bei Raumtemperatur nachrühren. Anschließend gießt man das Reaktionsgemisch auf gesättigte wäßrige Natriumhydrogencarbonatlösung, extrahiert mit Dichlormethan und wäscht die organische Phase mit gesättigter Natriumthiosulfat-sowie gesättiger Natriumchloridlösung. Aufreinigung ergibt 832 mg 15a).
¹H-NMR (CDCl₃): δ= 6,20 s (1H, H-4); 5,55 m (2H, Bügel); 3,52 d (J=4 Hz, 1H, H-6); 3,45 dbr (J= 4 Hz, 1H, H-7); 2,70 m (1H, H-11); 2,15 s (3H, Acetyl); 2,10 s (3H, Acetoxy); 0,73 s (3H, C-18)

### b) 17-(Acetyloxy)-6β-chlor-9,11α-dihydro-7α-hydroxy-6'H-benzo[10,9,11]-19-norpregn-4-en-3,20-dion

Zu einer Lösung von 832 mg der unter 15a) beschriebenen Verbindung in 20 ml Eisessig werden 4,8 g Lithiumchlorid gegeben. Man rührt 1,5 Stunden nach und arbeitet dann wäßrig (B,F) auf. Das erhaltenen Rohprodukt (900 mg) wird ohne Aufreinigung in die Folgestufe eingesetzt.

### c) 17-(Acetyloxy)-6β-chlor-9,11α-dihydro-7α-[(methylsulfonyl)oxy]-6'H-benzo-[10,9,11]-19-norpregn-4-en-3,20-dion

Zu einer Lösung von 900 mg der unter 15b) beschriebenen Substanz in 10 ml Pyridin werden bei 0°C 1,2 ml Methansulfonsäurechlorid gegeben. Man rührt zwei Stunden bei Raumtemperatur nach, gießt dann das Reaktionsgemisch auf gesättigte wäßrige Natriumchloridlösung und extrahiert mit Dichlormethan. Die organische Phase wird mit gesättigter Natriumchloridlösung gewaschen. Das erhaltene Rohprodukt wird ohne Reinigung in die Folgestufe eingesetzt.

### d) 17-(Acetyloxy)-6-chlor-9,11α-dihydro-6'H-benzo[10,9,11]-19-norpregna-4,6-dien-3,20-dion

Zu einer Lösung von 930 mg der unter 15c) beschriebenen Substanz in 25 ml N,N-Dimethylformamid werden 3,5 g wasserfreies Natriumacetat gegeben. Man erwärmt auf 100°C und läßt 1,5 Stunden bei dieser Temperatur nachrühren. Anschließend wird das Reaktionsgemisch auf Eiswasser gegossen. Man läßt eine weitere Stunde nachrühren und filtriert danach den Niederschlag ab. Nach Aufreinigung des Rohproduktes werden 450 mg 15d) erhalten.
Fp= 225°C, [α]_{D}²⁰= -39,2° (CHCl₃; c=0,505)
¹H-NMR (CDCl₃): δ= 6,40 ppm sbr (1H, H-4); 6,35 d (J= 2 Hz, 1H, H-7); 5,60 m (2H, Bügel); 2,80 m (1H, H-11); 2,13 s (3H, Acetyl); 2,09 s (3H, Acetoxy); 0,72 s (3H, C-18)

### Beispiel 16

### 17-(Acetyloxy)-9,11α-dihydro-6-methylen-6'H-benzo[10,9,11]-19-norpregn-4-en-3,20-dion

3 g der unter Beispiel 13c) beschriebenen Substanz werden in 75 ml Tetrahydrofuran gelöst. Man addiert 7,5 ml Ethanol, 7,5 ml Triethylorthoformiat und 170 mg p-Toluolsulfonsäure. Anschließend wird eine Stunde bei 40°C gerührt. Danach werden 2,3 ml N-Methylanilin und 2,6 ml 37%ige wäßrige Formaldehydlösung addiert. Es wird weitere 30 Minuten bei 40°C nachgerührt. Anschließend läßt man auf Raumtemperatur abkühlen und setzt 7,5 ml konzentrierte Salzsäure hinzu. Man läßt weitere 3 Stunden bei Raumtemperatur rühren und arbeitet wäßrig (A,E,F) auf. Nach Aufreinigung werden 2 g 16) erhalten.
¹H-NMR (CDCl₃): δ= 6,02 ppm sbr (1H, H-4); 5,55 m (2H, Bügel); 5,15 m (1H, exo-Methylen), 5,02 m (1H, exo-Methylen); 2,76 m (1H, H-11); 2,13 s (3H, Acetyl); 2,10 s (3H, Acetoxy); 0,72 s (3H, C-18)

### Beispiel 17

### 17-(Acetyloxy)-9,11α-dihydro-6α-methyl-6'H-benzo[10,9,11]-19-norpregn-4-en-3,20-dion

2 g der unter 16) beschriebenen Substanz werden in 30 ml Ethanol gelöst. Man addiert 3 ml Cyclohexen und 250 mg Palladium auf Aktivkohle (10%). Danach wird eine Stunde unter Rückfluß gekocht. Anschließend wird die Reaktionslösung über Celite filtriert. Man engt im Vakuum ein, nimmt den Rückstand in 30 ml Aceton auf, addiert 1,4 ml 4 normale Salzsäure und rührt 2,5 Stunden bei 40°C nach. Wäßrige Aufarbeitung (B,F) und Reinigung ergibt 1,1 g 17).
Fp= 248°C, [α]_{D}²⁰= -39,4° (CHCl₃; c=0,510)
¹H-NMR (CDCl₃): δ= 5,90 ppm sbr (1H, H-4); 5,57 m (2H, Bügel); 2,73 m (1H, H-11); 2,13 s (3H, Acetyl); 2,09 s (3H, Acetoxy); 1,12 d (J=6 Hz, 3H, 6α-Methyl): 0,72 s (3H, C-18)

### Beispiel 18

### 17-(Acetyloxy)-9,11α-dihydro-6-methyl-6'H-benzo[10,9,11]-19-norpregna-4,6-dien-3,20-dion

Zu 0,25 ml Cyclohexen in 15 ml Ethanol werden 125 mg Palladium auf Aktivkohle (10%) gegeben. Man kocht eine Stunde unter Rückfluß und addiert dann eine Lösung von 500 mg der unter Beispiel 16) beschriebenen Substanz in 5 ml Ethanol. Danach wird weitere zwei Stunden unter Rückfluß gekocht. Anschließend filtriert man über Celite und engt im Vakuum ein. Nach Aufreinigung werden 400 mg 18) erhalten.
Fp= 203°C, [α]_{D}²⁰= +28,1° (CHCl₃; c=0,515)
¹H-NMR (CDCl₃): δ= 6,00 ppm sbr (1H, H-7); 5,92 sbr (1H, H-4); 5,60 m (2H, Bügel); 2,77 m (1H, H-11); 2,14 s (3H, Acetyl); 2,10 s (3H, Acetoxy); 1,88 sbr (3H, 6-Methyl); 0,73 s (3H, C-18)

### Beispiel 19

### 17β-Hydroxy-17α-methyl-4',5',9,11α-tetrahydro-6'H-benzo[10,9,11]estr-4-en-3-on

### a) 3,3-[2,2-Dimethyl-1,3-propandiylbis(oxy)]-5-hydroxy-4',5',9,11α-tetrahydro-6'H-benzo[10,9,11]-5α-estran-17-on

### Hydrierung unter Verwendung von Palladium auf Aktivkohle:

Zu einer Lösung von 1,6 g der unter 1e) beschriebenen Substanz in 65 ml Ethanol werden 165 mg Palladium auf Aktivkohle (10%ig) addiert. Man setzt die Apparatur unter 15 bar Wasserstoffdruck und läßt zwei Stunden reagieren. Anschließend wird über Celite filtriert und im Vakuum eingeengt. Man erhält 1,5 g 19a) welches ohne Reinigung weiter eingesetzt wird.

### Hydrierung unter Verwendung von Platinoxid:

Zu einer Lösung von 1 g der unter 1e) beschriebenen Substanz in einem Gemisch aus 24 ml Ethylacetat und 6 ml Tetrahydrofuran werden 100 mg Platin(IV)oxid gegeben. Man setzt sie Apparatur unter Wasserstoff und läßt eine Stunde bei Raumtemperatur nachrühren. Anschließend wird die Reaktionslösung über Celite filtriert und eingeengt. Man erhält nach Aufreinigung 600 mg 19a) und 150 mg 3,3-[2,2-Dimethyl-1,3-propandiylbis(oxy)]-4',5',9,11α-tetrahydro-6'H-benzo[10,9,11]-5α-estran-5,17β-diol.
¹H-NMR (CDCl₃): δ= 4,30 ppm s (1H, OH); 3,50-3,60 m (4H, Ketal); 2,40 dd (J=17, 9 Hz, 1H, H-16); 1,00 s (3H, C-18); 0,96 s (3H, Me-Ketal); 0,94 s (3H, Me-Ketal)

### b) 3,3-[2,2-Dimethyl-1,3-propandiylbis(oxy)]-17α-methyl-4',5',9,11α-tetrahydro-6'H-benzo[10,9,11]-5α-estran-5,17β-diol

Analog zu Beispiel 1f) werden 1,65 g der unter 19a) hergestellten Substanz in 30 ml absolutem Tetrahydrofuran mit 18,8 ml einer 1,6 molaren Lösung von Methyllithium in Diethylether umgesetzt. Man erhält 1,5 g 19b) welches ohne Reinigung in die Folgestufe eingesetzt werden.

### c) 17β-Hydroxy-17α-methyl-4',5',9,11α-tetrahydro-6'H-benzo[10,9,11]estr-4-en-3-on

Gemäß der allgemeinen Vorschrift 1 werden 1,5 g der unter 19b) beschriebenen Substanz mit 4 normaler Salzsäure in Aceton umgesetzt. Man erhält nach Aufreinigung 0,97 g 19c) als weißen Schaum.
[α]_{D}²⁰= +107,8° (CHCl₃, c=0,520)
¹H-NMR (CDCl₃): δ= 5,75 ppm sbr (1H, H-4); 1,22 s (3H, Methyl); 1,03 s (3H, C-19)

### Beispiel 20

### 17β-Hydroxy-17α-(1-propinyl)-4',5',9,11α-tetrahydro-6'H-benzo[10,9,11]estr-4-en-3-on

### a) 3,3-[2,2-Dimethyl-1,3-propandiylbis(oxy)]-17α-(1-propinyl)-4',5',9,11α-tetrahydro-6'H-benzo[10,9,11]-5α-estran-5,17β-diol

Gemäß der allgemeinen Vorschrift 2) werden 1 g der unter 19a) beschriebenen Verbindung und 15 ml einer 1,6 molaren Lösung von n-Butyllithium in Hexan sowie Propingas in absolutem Tetrahydrofuran umgesetzt. Das erhaltene Rohprodukt (1,19 g) wird ohne Reinigung in die Folgestufe eingesetzt

### b) 17β-Hydroxy-17α-(1-propinyl)-4',5',9,11α-tetrahydro-6'H-benzo[10,9,11]estr-4-en-3-on

Gemäß der allgemeinen Vorschrift 1) werden 1,19 g 20a) mit 4 normaler Salzsäure in Aceton umgesetzt. Man erhält nach Aufreinigung 720 mg 20b).
Fp= 186,7°C, [α]_{D}²⁰= +70,1° (CHCl₃; c=0,510)
¹H-NMR (CDCl₃): δ= 5,73 ppm s (1H, H-4); 1,84 s (3H, Propin); 1,01 s (3H, C-18)

### Beispiel 21

### 17β-Hydroxy-17α-(1-propinyl)-4',5',9,11α-tetrahydro-6'H-benzo[10,9,11]-estra-4,15-dien-3-on

### a) 3,3-[2,2-Dimethyl-1,3-propandiylbis(oxy)]-4',5',9,11α-tetrahydro-17-[(trimethylsilyl)oxy]-6'H-benzo[10,9,11]-estr-16-en-5-ol

Gemäß der allgemeinen Vorschrift 4a) werden 1 g der unter 19a) beschriebenen Verbindung mit 1,2 ml Diisopropylamin, 5,6 ml einer 1,6 molaren Lösung von n-Butyllithium in Hexan und 1,5 ml Trimethylchlorsilan in absolutem Tetrahydrofuran umgesetzt. Man erhält nach Umkristallisation des Rohproduktes aus Acetonitril 940 mg 21a).

### b) 3,3-[2,2-Dimethyl-1,3-propandiylbis(oxy)]-5-hydroxy-4',5',9,11α-tetrahydro-6'H-benzo[10,9,11]-5α-estr-15-en-17-on

Gemäß der allgemeinen Vorschrift 4b) werden 940 mg 21a) mit 475 mg Palladium(II)acetat in Acetonitril umgesetzt. Man erhält nach Aufreinigung 650 mg 21b).
¹H-NMR (CDCl₃): δ= 7,52 ppm dbr (J=6 Hz, 1H, H-15); 5,96 dd (J=6, 3 Hz, 1H, H-16); 4,45 s (1H, OH); 3,50-3,60 m (4H, Ketal); 1,21 s (3H, C-18); 1,00 s (3H, Me-Ketal); 0,97 s (3H, Me-Ketal)

### c) 3,3-[2,2-Dimethyl-1,3-propandiylbis(oxy)]-17α-(1-propinyl)-4',5',9,11α-tetrahydro-6'H-benzo[10,9,11]-5α-estr-15-en-5,17β-diol

Gemäß der allgemeinen Vorschrift 2) werden aus 650 mg 21b), 10 ml einer 1,6 molaren Lösung von n-Butyllithium in Hexan sowie Propingas in absolutem Tetrahydrofuran 712 mg 21c) erhalten. Das Rohprodukt wird ohne Aufreinigung in die Folgestufe eingesetzt.

### d) 17β-Hydroxy-17α-(1-propinyl)-4',5',9,11α-tetrahydro-6'H-benzo[10,9,11]-estra-4,15-dien-3-on

Gemäß der allgemeinen Vorschrift 1) werden 712 mg 21c) mit 4 normaler Salzsäure in Aceton umgesetzt. Man erhält nach Aufreinigung 440 mg 21d).
[α]_{D}²⁰= -79,6° (CHCl₃; c=0,510)
¹H-NMR (CDCl₃): δ= 5,98 ppm dbr (J=6 Hz, 1H, H-15); 5,76 sbr (1H, H-4) 5,70 dd (J=6, 3 Hz, 1H, H-16); 1,90 s (3H, Propin); 1,09 s (3H, C-18)

### Beispiel 22

### 5',6'-Dihydro-17β-hydroxy-17α-(1-propinyl)-9H-benzo[10,9,11]estr-4-en-3-on

### a) 5',6'-Dihydro-3,3-[2,2-dimethyl-1,3-propandiylbis(oxy))]-5-hydroxy-9H-benzo-[10,9,11]-5α-estran- 17-on

4 g der unter le) beschriebenen Substanz werden in 120 ml eines Gemisches aus Tetrahydrofuran und Ethanol (1:1) gelöst. Man addiert 25 ml Cyclohexen und 1 g Palladium auf Aktivkohle (10%ig) und kocht 24 Stunden unter Rückfluß. Anschließend wird über Celite filtriert und im Vakuum eingeengt. Man erhält nach Aufreinigung 1,9 g 22a).
¹H-NMR (CDCl₃): δ= 5,29 ppm m (1H, Bügel/11-exo); 4,40 s (1H, OH); 3,50-3,60 m (4H, Ketal); 2,43 dd (J= 17, 9 Hz, 1H, H-16); 1,00 s (3H, Me-Ketal); 0,98 s (3H, Me-Ketal); 0,80 s (3H, C-18)

### b) 5',6'-Dihydro-3,3-[2,2-dimethyl-1,3-propandiylbis(oxy)]-17α-(1-propinyl)-9H-benzo-[10,9,11]-5α-estran-5,17β-diol

Gemäß der allgemeinen Vorschrift 2) werden aus 1 g der unter 22a) beschriebenen Substanz und 15 ml einer 1,6 molaren Lösung von n-Butyllithium in Hexan sowie Propingas in absolutem Tetrahydrofuran 1,1 g 22b) als Rohprodukt erhalten, welches ohne Aufreinigung in die Folgestufe eingesetzt wird.

### c) 5',6'-Dihydro-17β-hydroxy-17α-(1-propinyl)-9H-benzo[10,9,11]estr-4-en-3-on

Gemäß der allgemeinen Vorschrift 1 werden 1,1 g der unter 22b) beschriebenen Substanz mit 4 normaler Salzsäure in Aceton umgesetzt. Man erhält nach Aufreinigung 640 mg 22c).
Fp= 197,3°C, [α]_{D}²⁰= +40,7° (CHCl₃; c=0,520)
¹H-NMR (CDCl₃): δ= 5,27 ppm sbr (1H, H-4); 5,48 m (1H, Bügel/11-exo); 1,86 s (3H, Propin); 0,80 s (3H, C-18)

### Beispiel 23

### 5',6'-Dihydro-17β-hydroxy-17α-(1-propinyl)-9H-benzo[10,9,11]estra-4,15-dien-3-on

### a) 5',6'-Dihydro-3,3-[2,2-dimethyl-1,3-propandiylbis(oxy)]-17-[(trimethylsilyl)oxy]-9H-benzo[10,9,11]-5α-estr-16-en-5-ol

Gemäß der allgemeinen Vorschrift 4a) werden 1 g der unter 22a) beschriebenen Substanz mit 1,2 ml Diisopropylamin, 5,6 ml einer 1,6 molaren Lösung von n-Butyllithium in Hexan und 1,5 ml Trimethylchlorsilan in absolutem Tetrahydrofuran umgesetzt. Man erhält nach Umkristallisation des Rohproduktes aus Acetonitril 920 mg 23a).

### b) 5',6'-Dihydro-3,3-[2,2-dimethyl-1,3-propandiylbis(oxy)]-5-hydroxy-9H-benzo-[10,9,11]-5α-estr-15-en-17-on

Gemäß der allgemeinen Vorschrift 4b) werden 920 mg der unter 23a) beschriebenen Verbindung mit 470 mg Palladium(II)acetat in Acetonitril umgesetzt. Man erhält nach Aufreinigung 630 mg 23b).
¹H-NMR (CDCl₃): δ= 7,55 ppm dbr (J=6 Hz, 1H, H-15); 6,02 dd (J=6, 3 Hz, 1H, H-16); 5,48 m (1H, Bügel/11-exo); 4,42 s (1H, OH); 3,50-3,60 m (4H, Ketal); 1,04 s (3H, C-18); 1,00 s (3H, Me-Ketal); 0,97 s (3H, Me-Ketal)

### c) 5',6'-Dihydro-3,3-[2,2-dimethyl-1,3-propandiylbis(oxy)]-17α-(1-propinyl)-9H-benzo-[10,9,11]-5α-estr-15-en-5,17β-diol

Gemäß der allgemeinen Vorschrift 2) werden 630 mg 23b) mit 10 ml einer 1,6 molaren Lösung von n-Butyllithium sowie Propingas in absolutem Tetrahydrofuran umgesetzt. Das Rohprodukt (685 mg) wird ohne Reinigung in die Folgestufe eingesetzt.

### d) 5',6'-Dihydro-17β-hydroxy-17α-(1-propinyl)-9H-benzo[10,9,11]estra-4,15-dien-3-on

Gemäß der allgemeinen Vorschrift 1) werden 685 mg 23b) mit 4 normaler Salzsäure in Aceton umgesetzt. Man erhält nach Aufreinigung 430 mg 23d).
[α]_{D}²⁰= -128,7° (CHCl₃; c=0,515)
¹H-NMR (CDCl₃): δ= 5,97 ppm dbr (J= 6 Hz, 1H, H-15); 5,78 sbr (1H, H4); 5,73 dd (J=6, 3 Hz, 1H, H-16); 5,40 m (1H, Bügel/11-exo); 1,90 s (3H, Propin); 0,90 s (3H, C-18)

### Beispiel 24

### 17-(Acetyloxy)-5',6'-dihydro-9H-benzo[10,9,11]-19-norpregn-4-en-3,20-dion

### a) 5',6'-Dihydro-5,17α-dihydroxy-3,3-[2,2-dimethyl-1,3-propandiylbis(oxy)]-9H-benzo-[10,9,11]-5α-estran-17β-carbonitril

Analog zu Beispiel 13a) werden aus 1,6 g der unter 22a) beschriebenen Verbindung, 8 ml 2-Hydroxy-2-methylpropannitril und 0,15 ml 20%iger wäßriger Natriumhydroxidlösung 1,3 g 24a) erhalten.
¹H-NMR (CDCl₃): δ= 5,38 ppm m (1H, Bügel/11-exo); 4,50 s (1H, OH); 3,50-3,60 m (4H, Ketal); 1,00 s (3H, Me-Ketal); 0,98 s (3H, Me-Ketal); 0,90 s (3H, C-18)

### b) 5',6'-Dihydro- 17-hydroxy-9H-benzo[10,9,11]-19-norpregn-4-en-3,20-dion

Analog zu Beispiel 13b) werden 1,3 g der unter 24a) beschriebenen Substanz mit 2,8 ml Ethoxyethen und 120 mg p-Toluolsulfonsäure in Diethylether umgesetzt. Das erhaltene Rohprodukt wird erneut in Diethylether gelöst und mit 5 ml einer 1,6 molaren Lösung von Methyllithium in Diethylether umgesetzt. Das erhaltene Rohprodukt wird sauer (gemäß der allgemeinen Vorschrift 1) mit 4 normaler Salzsäure in Aceton gespalten. Man erhält nach Umkristallisation aus Diisopropylether/Methanol 700 mg 24b).
¹H-NMR (CDCl₃): δ= 5,77 ppm sbr (1H, H-4); 5,32 m (1H, Bügel/11-exo); 2,28 s (3H, Acetyl); 0,78 s (3H, C-18)

### c) 17-(Acetyloxy)-5',6'-dihydro-9H-benzo[10,9,11]-19-norpregn-4-en-3,20-dion

700 mg der unter 24b) beschriebenen Verbindung werden analog zu 13c) mit 9 ml Eisessig und 3,5 ml Trifluoressigsäureanhydrid umgesetzt. Man erhält nach Aufreinigung 520 mg 24c).
¹H-NMR (CDCl₃): δ=5,79 ppm sbr (1H, H-4); 5,48 m (1H, Bügel/11-exo); 2,15 s (3H, Acetyl); 2,10 s (3H, Acetoxy); 0,62 s (3H, C-18)

### Beispiel 25

### 9,11α-Dihydro-17α-ethinyl-17β-hydroxy-6'H-benzo[10,9,11]-18a-homoestr-4-en-3-on

### a) 17β-Hydroxy-18a-homoestr-5(10)-en-3-on

Zu einer Suspension von 355 g 17β-Hydroxy-3-methoxy-18a-homoestra-2,5(10)-dien in 4 1 Aceton wird binnen 15 min eine Lösung von 252 g Oxalsäuredihydrat in 2 l Wasser getropft, anschließend für 3 h gerührt, dann in 3,6 l Wasser eingerührt und zweimal mit je 2 l Dichlormethan extrahiert. Die vereinigten organischen Phasen werden zweimal mit je 1,5 l gesättigter Natriumhydrogencarbonatlösung gewaschen, über Natriumsulfat getrocknet und im Vakuum eingeengt Man erhält nach Umkristallisation aus Essigester 293 g 25a).
Fp= 103-105°C, [α]_{D}²⁰= +155,0° (CHCl₃; c=0,505)
¹H-NMR (CDCl₃): δ= 3,78 ppm dd (J=14, 7.5 Hz, 1H, H-17); 1.00 t (J=7.5 Hz, 3H, 18a-CH₃)

### b)17β-Hydroxy-18a-homoestra-4,9-dien-3-on

Zu einer Lösung von 290 g der unter 25a) beschriebenen Verbindung in 4 l Pyridin werden unter Kühlung 443 g Pyridiniumhydrobromidperbromid so langsam addiert, daß die Temperatur 25°C nicht übersteigt. Anschließend wird für 2 h bei 50°C gerührt, dann im Eisbad gekühlt, in 4 l eisgekühlte halbkonzentrierte Salzsäure eingerührt und dann einmal mit 4 l und zweimal mit je 2 l Dichlormethan extrahiert. Die vereinigten organischen Phasen werden mit 4 l eiskalter halbkonzentrierter Salzsäure gewaschen, über Natriumsulfat getrocknet und im Vakuum eingeengt. Nach Digerieren mit warmem Essigester werden 147,5 g 25b) erhalten. Chromatographie der Mutterlauge ergibt weitere 41,5 g 25b).
Fp= 136-138°C, [α]_{D}²⁰= -293,9° (CHCl₃; c=0,510)
¹H-NMR (CDCl₃): δ= 5,68 ppm sbr (1H, 4-H); 3,77 dd (J=14, 7.5 Hz, 1H, H-17); 1.08 t (J=7.5 Hz, 3H, 18a-CH₃)

### c) 3,3-[2,2-Dimethyl-1,3-propandiylbis(oxy)]-18a-homoestra-5(10),9(11)-dien-17β-ol

Zu einer Lösung von 189 g der unter 25b) beschriebenen Verbindung in 1,8 l Dichlormethan werden 180 g 2,2-Dimethylpropan-1,3-diol, 84 g Trimethylorthoformiat und 1 g p-Toluolsulfonsäuremonohydrat gegeben. Nach 3 h Rühren wird mit 1 Dichlormethan verdünnt, mit 2 l gesättigter Natriumhydrogencarbonatlösung gewaschen, die wäßrige Phase zweimal mit je 400 ml Dichlormethan extrahiert und die vereinigten organischen Phasen nach Trocknung über Natriumsufat im Vakuum eingeengt. Der Rückstand wird in 200 ml Dichlormethan gelöst, mit 900 ml Methanol und 180 g Kaliumcarbonat versetzt und die Mischung für 1 h unter Rückfluß erhitzt. Die Mischung wird anschließend im Vakuum weitestgehend eingeengt, mit 2 l Wasser versetzt und dann mit 2 l und noch dreimal mit je 400 ml Dichlormethan extrahiert. Nach Trocknung der vereinigten organischen Phasen über Natriumsulfat und Abziehen des Lösungsmittels im Vakuum erhält man 252 g 25c), welches ohne Reinigung in die Folgestufe eingesetzt wird.
¹H-NMR (CDCl₃): δ= 5,55 ppm m (1H, 11-H); 3,86 dd (J=14, 7.5 Hz, 1H, H-17); 3,40-3,68 m (4H, Ketal); 1,07 s (3H, Me-Ketal); 0.94 t (J= 7.5 Hz, 3H, 18a-CH₃); 0,90 s (3H, Me-Ketal)

### d) 3,3-[2,2-Dimethyl-1,3-propandiylbis(oxy)]-5,10α-epoxy-18a-homo-5α-estr-9(11)-en-17β-ol

252 g der unter 25c) beschriebenen Verbindung werden in 1,2 l Dichlormethan gelöst und mit 59 g 2-(3-Nitrophenyl)-1,1,1-trifluorethanon und 190 ml gesättigter Natriumhydrogencarbonatlösung versetzt. Unter Kühlung im Eibad werden 240 ml 30%ige Wasserstoffsuperoxidlösung addiert und die Mischung für 18 h bei T<10°C gerührt. Anschließend werden vorsichtig 500 ml gesättigte Natriumthiosulfatlösung zugetropft, dann mit 500 ml Wasser verdünnt, die Phasen getrennt und die wäßrige Phase zweimal mit je 500 ml Dichlormethan extrahiert. Die vereinigten organischen Phasen werden mit 500 ml gesättigter Natriumchloridlösung, zweimal mit je 500 ml 5%iger Natronlauge und wiederum mit 500 ml gesättigter Natriumchloridlösung gewaschen, über Natriumsulfat getrocknet und im Vakuum eingeent. Man erhält 250 g rohes 25d), welches ohne Reinigung in die Folgestufe eingesetzt wird.
¹H-NMR (CDCl₃): δ= 6,03 ppm m (1H, 11-H); 3,82 m (1H, H-17); 3,36-3,63 m (4H, Ketal); 1,07 s (3H, Me-Ketal); 0.98 t (J= 7.5 Hz, 3H, 18a-CH₃); 0,87 s (3H, Me-Ketal)

### e) 3,3-[2,2-Dimethyl-1,3-propandiylbis(oxy)]-19-ethinyl-18a-homo-5α-androst-9(11)-en-5,17β-diol

50 g der unter 25d) beschriebenen Verbindung werden analog zu 1a) mit aus 3-Brompropin erzeugtem Grignardreagenz umgesetzt. Nach wäßriger Aufarbeitung (F) und Aufreinigung des Rohproduktes werden 33,6 g 25e) erhalten.
Fp= 112-114°C, [α]_{D}²⁰= -16,2° (CHCl₃; c=0,510)
¹H-NMR (CDCl₃): δ= 5,42 ppm m (1H, H-11); 4,38 s (1H, OH); 3,82 m (1H, H-17); 3,42-3,60 m (4H, Ketal); 1,88 t (J=1.5 Hz, 1H, Ethin); 0,99 s (3H, Me-Ketal); 0,92 s (3H, Me-Ketal); 0,92 t (J=7.5 Hz, 3H, 18a-CH₃)

### f) 19-(Bromethinyl)-3,3-[2,2-dimethyl-1,3-propandiylbis(oxy)]-18a-homo-5α-androst-9(11)-en-5,17β-diol

75,5 g der unter 25e) beschriebenen Substanz werden analog zu 1b) in 2 l Aceton mit 2,99 g Silbernitrat und 32,9 g N-Bromsuccinimid umgesetzt. Wäßrige Aufarbeitung (B,F) ergibt 96 g 25f), welches ohne Reinigung in die Folgestufe eingesetzt wird.

### g) 19-(2-Bromethenyl)-3,3-[2,2-dimethyl-1,3-propandiylbis(oxy)]-18a-homo-5α-androst-9(11)-en-5,17β-diol

96 g der unter 25f) beschriebenen Substanz werden analog zu 1c) in 1,8 1 eines Gemisches aus Tetrahydrofuran und Wasser (1:1) mit 131 g p-Toluolsulfonsäurehydrazid und 87 g Natriumacetat umgesetzt. Wäßrige Aufarbeitung (B,F) sowie Aufreinigung ergibt 66,7 g 25g) als hellgelben Schaum.
[α]_{D}²⁰= -157,2° (CHCl₃; c=0,515)
¹H-NMR (CDCl₃): δ= 6,15 ppm m (1H, Vinyl); 5,78 m (1H, Vinyl); 5,34 m (1H, H-11); 4,40 s (1H, OH); 3,83 dd (J=14, 7.5 Hz, 1H, H-17); 3,46-3,58 m (4H, Ketal); 0,98 s (3H, Me-Ketal); 0,96 s (3H, Me-Ketal); 0,94 t (J=7.5 Hz, 3H, 18a-CH₃)

### h) 9,11α-Dihydro-3,3-[2,2-dimethyl-1,3-propandiylbis(oxy)]-6'H-benzo[10,9,11]-18a-homo-5α-estran-5,17β-diol

66,3 g der unter 25g) beschriebenen Substanz werden analog zu 1d) in 1,3 l Toluol mit 40 ml Tributylzinnhydrid und 75 mg Azobisisobutyronitril umgesetzt. Nach Aufreinigung des Rohproduktes werden 31 g 25h) erhalten.
Fp= 213-215°C, [α]_{D}²⁰= -45,1° (CHCl₃; c=0,505)
¹H-NMR (CDCl₃): δ= 5,70 ppm dbr (J=10 Hz, 1H, Bügel); 5,48 m (1H, Bügel); 4,37 s (1H, OH); 3,70 m (1H, 17-H); 3,48-3,63 m (4H, Ketal); 1.07 t (J=7.5 Hz, 3H, 18a-CH₃); 0,99 s (3H, Me-Ketal); 0,97 s (3H, Me-Ketal)

### i) 9,11α-Dihydro-3,3-[2,2-dimethyl-1,3-propandiylbis(oxy)]-5-hydroxy-6'H-benzo-[10,9,11]-18a-homo-5α-estran-17-on

Gemäß der allgemeinen Vorschrift 3) werden aus 12,0 g der unter 25h) beschriebenen Substanz, 16,7 g Chromtrioxid und 57 ml Pyridin in 500 ml Dichlormethan 11,0 g 25i) als weiße Kristalle erhalten.
Fp= 200-203°C, [α]_{D}²⁰=-17,4° (CHCl₃; c=0,510)
¹H-NMR (CDCl₃): δ= 5,61 ppm dbr (J=10 Hz, 1H, Bügel); 5,52 m (1H, Bügel); 4,40 s (1H, OH); 3,48-3,63 m (4H, Ketal); 2,51 m (1H, H-11); 0,99 s (3H, Me-Ketal); 0,97 s (3H, Me-Ketal); 0,79 t (J=7.5 Hz, 3H, 18a-CH₃)

### j) 9,11α-Dihydro-3,3-[2,2-dimethyl-1,3-propandiylbis(oxy)]-17α-ethinyl-6'H-benzo-[10,9,11]-18a-homo-5α-estran-5,17β-diol

Gemäß der allgemeinen Vorschrift 2) werden 750 mg der unter 25i) beschriebenen Substanz und 11 ml einer 1,6 molaren Lösung von n-Butyllithium in Hexan sowie Ethingas in absolutem Tetrahydrofuran umgesetzt. Man erhält nach Aufreinigung 620 mg 25j) als weiße Kristalle.
Fp= 124°C, [α]_{D}²⁰= -49,6° (CHCl₃; c=0,520)
¹H-NMR (CDCl₃): δ= 5,72 ppm dbr (J=10 Hz, 1H, Bügel); 5,49 m (1H, Bügel); 4,40 s (1H, OH); 3,50-3,65 m (4H, Ketal); 2,60 s (1H, Ethin); 2,50 m (1H, H-11); 1,06 t (J=7.5 Hz, 3H, 18a-CH₃); 0,98 s (6H, Me-Ketal)

### k) 9,11α-Dihydro-17α-ethinyl-17β-hydroxy-6'H-benzo[10,9,11]-18a-homoestr-4-en-3-on

Gemäß der allgemeinen Vorschrift 1) werden 600 mg 25j) mit 1,5 ml 4 normaler Salzsäure in Aceton umgesetzt. Man erhält nach Aufreinigung des Rohproduktes 348 mg 25k) als weiße Kristalle.
Fp= 242-245°C, [α]_{D}²⁰= -60,3° (CHCl₃; c=0,520)
¹H-NMR (CDCl₃): δ= 5,81 ppm sbr (1H, H-4); 5,77 dbr (J=10 Hz, 1H, Bügel); 5,58 m (1H, Bügel); 2,67 m (1H, H-11); 2,62 s (1H, Ethin); 1,08 t (J=7.5 Hz, 3H, 18a-CH₃)

### Beispiel 26

### 9,11α-Dihydro-17β-hydroxy-17α-(1-propinyl)-6'H-benzo[10,9,11]-18a-homoestr-4-en-3-on

### a) 9,11α-Dihydro-3,3-[2,2-dimethyl-1,3-propandiylbis(oxy)]-17α-(1-propinyl)-6'H-benzo-[10,9,11]-18a-homo-5α-estran-5,17β-diol

Gemäß der allgemeinen Vorschrift 2) werden 750 mg der unter 25i) beschriebenen Verbindung und 11 ml einer 1,6 molaren Lösung von n-Butyllithium in Hexan sowie Propingas in absolutem Tetrahydrofuran umgesetzt. Man erhält 705 mg 26a) als amorphen Feststoff.
[α]_{D}²⁰= -42,4° (CHCl₃; c=0,972)
¹H-NMR (CDCl₃): δ= 5,71 ppm dbr (J=10 Hz, 1H, Bügel); 5,48 m (1H, Bügel); 4,46 s (1H, OH); 3,50-3,63 m (4H, Ketal); 2,50 m (1H, H-11); 1,88 s (3H, Propin); 1,06 t (J=7.5 Hz, 3H, 18a-CH₃); 0,98 s (3H, Me-Ketal); 0,96 s (3H, Me-Ketal)

### b) 9,11α-Dihydro-17β-hydroxy-17α-(1-propinyl)-6'H-benzo[10,9,11]-18a-homoestr-4-en-3-on

Gemäß der allgemeinen Vorschrift 1) werden 690 mg der unter 26a) beschriebenen Substanz mit 2 ml 4 normaler wäßriger Salzsäure in Aceton umgesetzt. Man erhält nach Aufreinigung des Rohproduktes 380 mg 26b) als weiße Kristalle.
Fp= 201-203°C; [α]_{D}²⁰= -57,4° (CHCl₃; c=0,510)
¹H-NMR (CDCl₃): δ= 5,81 ppm sbr (1H, H-4); 5,76 dbr (J=10 Hz,1H, Bügel); 5,56 m (1H, Bügel); 2,68 m (1H, H-11); 1,87 s (3H, Propin); 1,07 t (J=7.5 Hz, 3H, 18a-CH₃)

### Beispiel 27

### 9,11α-Dihydro-17α-(1-butinyl)-17β-hydroxy-6'H-benzo[10,9,11]-18a-homoestr-4-en-3-on

### a) 9,11α-Dihydro-3,3-[2,2-dimethyl- 1 ,3-propandiylbis(oxy)]-17α-butinyl-6'H-benzo-[10,9,11]-18a-homo-5α-estran-5,17β-diol

Gemäß der allgemeinen Vorschrift 2) werden 800 mg der unter 25i) beschriebenen Verbindung und 15 ml einer 1,6 molaren Lösung von n-Butyllithium in Hexan sowie 5 g 1-Butin in absolutem Tetrahydrofuran umgesetzt. Man erhält 545 mg 27a) als weiße Kristalle.
Fp= 186-191°C; [α]_{D}²⁰= -51,2° (CHCl₃; c=0,500)
¹H-NMR (CDCl₃): δ= 5,7 ppm dbr (J=10 Hz, 1H, Bügel); 5,48 m (1H, Bügel); 4,42 s (1H, OH); 3,46-3,62 m (4H, Ketal); 2,48 m (1H, H-11); 2,23 q (J= 7.5 Hz, 2H, Butin-CH₂); 1,12 t (J=7.5 Hz, 3H, Butin-CH₃); 1,06 t (J=7.5 Hz, 3H, 18a-CH₃); 0,98 s (3H, Me-Ketal); 0,96 s (3H, Me-Ketal)

### b) 9,11α-Dihydro-17α-(1-butinyl)-17β-hydroxy-6'H-benzo[10,9,11]-18a-homoestr-4-en-3-on

Gemäß der allgemeinen Vorschrift 1) werden 485 mg der unter 27a) beschriebenen Substanz mit 1 ml 4 normaler wäßriger Salzsäure in Aceton umgesetzt. Man erhält nach Aufreinigung des Rohproduktes 323 mg 27b) als weiße Kristalle.
Fp= 143-146°C; [α]_{D}²⁰= -52,5° (CHCl₃; c=0,500)
¹H-NMR (CDCl₃): δ= 5,81 ppm sbr (1H,H-4); 5,77 dbr (J=10 Hz, 1H, Bügel); 5,56 m (1H, Bügel); 2,68 m (1H, H-11); 2,23 q (J= 7.5 Hz, 2H, Butin-CH₂); 1,12 t (J=7.5 Hz, 3H, Butin-CH₃); 1,07 t (J=7.5 Hz, 3H, 18a-CH₃)

### Beispiel 28

### 9,11α-Dihydro-17α-ethenyl-17β-hydroxy-6'H-benzo[10,9,11]-18a-homoestr-4-en-3-on

### a) 9,11α-Dihydro-3,3-[2,2-dimethyl-1,3-propandiylbis(oxy)]-17α-ethenyl-6'H-benzo-[10,9,11]-18a-homo-5a-estran-5,17β-diol

Bei 0°C werden zu einer Lösung von 1,32 g Tetravinylzinn in 50 ml Diethylether 14,5 ml einer 1,6 molaren Lösung von n-Butyllithium in Hexan getropft und eine Stunde bei Raumtemperatur nachgerührt. Danach wird eine Lösung von 1 g der unter 25i) beschriebenen Verbindung in 25 ml Tetrahydrofuran addiert. Man läßt eine Stunde bei 0°C nachrühren und arbeitet dann wäßrig (C,F) auf. Man erhält nach Aufreinigung des Rohproduktes 660 mg 28a) als weiße Kristalle.
Fp= 117-121°C; [α]_{D}²⁰= -37,8° (CHCl₃; c=0,520)
¹H-NMR (CDCl₃): δ= 6,08 ppm dd (J=17.5, 10 Hz, 1H, Vinyl); 5,7 dbr (J=10 Hz, 1H, Bügel); 5,46 m (1H, Bügel); 5.12 dd (J=17.5, 2 Hz, 1H, Vinyl); 5,07 dd (J= 10, 2 Hz, 1H, Vinyl); 4,38 s (1H, OH); 3,48-3,60 m (4H, Ketal); 2,42 m (1H, H-11); 1,09 t (J=7.5 Hz, 3H, 18a-CH₃); 0,98 s (6H, Me-Ketal)

### b) 9,11α-Dihydro-17α-ethenyl-17β-hydroxy-6'H-benzo[10,9,11]-18a-homoestr-4-en-3-on

Gemäß der allgemeinen Vorschrift 1) werden 600 mg der unter 28a) beschriebenen Substanz mit 1,3 ml 4 normaler wäßriger Salzsäure in Aceton umgesetzt. Man erhält nach Aufreinigung des Rohproduktes 406 mg 28b) als weiße Kristalle.
Fp= 170-173°C; [α]_{D}²⁰= -24,4° (CHCl₃; c=0,515)
¹H-NMR (CDCl₃): δ= 6,06 ppm dd (J=17.5, 10 Hz, 1H, Vinyl); 5,80 sbr (1H, H-4); 5,75 dbr (J=10 Hz, 1H, Bügel); 5,56 m (1H, Bügel); 5,12 dd (J=17.5, 2 Hz, 1H, Vinyl); 5,08 dd (J= 10, 2 Hz, 1H, Vinyl); 1,12 t (J=7.5 Hz, 3H, 18a-CH₃)

### Beispiel 29

### 9,11α-Dihydro-17β-hydroxy-17α-(trifluormethyl)-6'H-benzo[10,9,11]-18a-homoestr-4-en-3-on

### a) 9,11α-Dihydro-3,3-[2,2-dimethyl-1,3-propandiylbis(oxy)]-17α-(trifluormethyl)-5-(trimethylsilyloxy)-6'H-benzo[10,9,11]-18a-homo-5α-estran-17β-ol

Zu einer Lösung von 1 g der unter 25i) beschriebenen Verbindung in 15 ml Tetrahydrofuran tropft man bei -10°C 1,7 ml Trifluormethyltrimethylsilan und anschließend 2,8 ml einer einmolaren Lösung von Tetra-n-butylammoniumfluorid in Tetrahydrofuran. Man rührt 30 Minuten nach, gießt dann auf ein Gemisch aus Ethylacetat und Wasser, extrahiert die wäßrige Phase noch zweimal mit Ethylacetat, trocknet die vereinigten organischen Phasen über Natriumsulfat und engt im Vakuum ein. Man erhält 1,39 g 29a) als weißen Schaum, der ohne Aufreinigung in die nächste Stufe eingesetzt wird.
¹H-NMR (CDCl₃): δ= 5,70 ppm dbr (J=10 Hz, 1H, Bügel); 5,50 m (1H, Bügel); 3,63 m (2H, Ketal); 3,30 m (2H, Ketal); 2,50 m (1H, H-11); 1,16 s (3H, Me-Ketal); 1,04 t (J=7.5 Hz, 3H, 18a-CH₃); 0,77 s (3H, Me-Ketal); 0,14 s (9H, Me₃Si)

### b) 9,11α-Dihydro-17β-hydroxy-17α-(trifluormethyl)-6'H-benzo[10,9,11]-18a-homoestr-4-en-3-on

Gemäß der allgemeinen Vorschrift 1) werden 1,38 g der unter 29a) beschriebenen Substanz mit 2,8 ml 4 normaler wäßriger Salzsäure in Aceton umgesetzt. Man erhält nach Aufreinigung des Rohproduktes 302 mg 29b) als weißen Schaum.
[α]_{D}²⁰= -17,6° (CHCl₃; c=0,505)
¹H-NMR (CDCl₃): δ= 5,80 ppm sbr (1H, H-4); 5,77 dbr (J=10 Hz, 1H, Bügel); 5,58 m (1H, Bügel); 2,67 m (1H, H-11); 1,08 t (J=7.5 Hz, 3H, 18a-CH₃)

### Beispiel 30

### 9,11α-Dihydro-17α-(cyanomethyl)-17β-hydroxy-6'H-benzo[10,9,11]-18a-homoestr-4-en-3-on

### a) 9,11α-Dihydro-3,3-[2,2-dimethyl-1,3-propandiylbis(oxy)]-17α-(cyanomethyl)-6'H-benzo[10,9,11]-18a-homo-5α-estran-5,17β-diol

Zu einer gemäß der allgemeinen Vorschrift 4a) aus 2,8 ml Diisopropylamin und 12,5 ml einer 1,6 molaren Lösung von n-Butyllithium erzeugten Lösung von Lithiumdiisopropylamid tropft man bei -70°C 0,82g Acetonitril, läßt 30 Minuten bei -70°C nachrühren und addiert dann eine Lösung von 1,29 g der unter 25i) beschriebenen Substanz in 10 ml Tetrahydrofuran und läßt binnen vier Stunden auf -40°C erwärmen. Nach wäßriger Aufarbeitung (C,F) und Aufreinigung erhält man 1,06 g 30a).
¹H-NMR (CDCl₃): δ= 5,70 ppm dbr (J=10 Hz, 1H, Bügel); 5,49 m (1H, Bügel); 4,40 s (1H, OH); 3,47-3,65 m (4H, Ketal); 2,65 und 2,52 AB-Signal (J_{AB}= 15 Hz, 2H, CH₂CN); 2,50 m (1H, H-11); 1,06 t (J=7.5 Hz, 3H, 18a-CH₃); 0,98 s (6H, Me-Ketal)

### b) 9,11α-Dihydro-17α-(cyanomethyl)-17β-hydroxy-6'H-benzo[10,9,11]-18a-homoestr-4-en-3-on

Gemäß der allgemeinen Vorschrift 1) werden 1,04 g der unter 30a) beschriebenen Substanz mit 1,1 ml 4 normaler wäßriger Salzsäure in Aceton umgesetzt. Man erhält nach Aufreinigung des Rohproduktes 640 mg 28b) als weiße Kristalle.
Fp= 207-212°C
¹H-NMR (CDCl₃): δ= 5,80 ppm sbr (1H, H-4); 5,76 dbr (J=10 Hz, 1H, Bügel); 5,59 m (1H, Bügel); 2,67 und 2,52 AB-Signal (J_{AB}= 15 Hz, 2H, CH₂CN); 1,10 t (J=7.5 Hz, 3H, 18a-CH₃)

### Beispiel 31

### 9,11α-Dihydro-17β-hydroxy-17α-(1,2-propadienyl)-6'H-benzo[10,9,11]-18a-homoestr-4-en-3-on

### a) 9,11α-Dihydro-3,3-[2,2-dimethyl-1,3-propandiylbis(oxy)]-17α-[3-[(tetrahydro-2H-pyran-2-yl)oxy]-1-propinyl]-6'H-benzo[10,9,11]-18a-homo-5α-estran-5,17β-diol

Aus 10,7 ml 3-[(Tetrahydro-2H-pyran-2-yl)oxy]-1-propin in 380 ml absolutem Tetrahydrofuran und 48 ml einer 1,6 molaren Lösung von n-Butyllithium in Hexan stellt man bei 0°C unter Argon die lithiumorganische Verbindung her. Anschließend wird eine Lösung von 3,3 g der unter 25i) beschriebenen Substanz in 80 ml absolutem Tetrahydrofuran addiert. Man läßt eine Stunde bei 0°C nachrühren und arbeitet dann wäßrig (C,F) auf. Man erhält nach Aufreinigung 3,7 g 31a) als weißen Schaum.
[α]_{D}²⁰= -39,4° (CHCl₃; c=0,545)
¹H-NMR (CDCl₃): δ= 5,70 ppm dbr (J=10 Hz,1H, Bügel); 5,48 m (1H, Bügel); 4,82 m (1H, THP); 4,39 s (1H, OH); 4,32 m (2H, CH₂OTHP); 3,87 m (1H, THP); 3,47-3,63 m (5H, Ketal und THP); 2,48 m (1H, H-11); 1,02 t (J=7.5 Hz, 3H, 18a-CH₃); 0,95 m (6H, Me-Ketal)

### b) 9,11α-Dihydro-3,3-[2,2-dimethyl-1,3-propandiylbis(oxy)]-17α-(1,2-propadienyl)-6'H-benzo[10,9,11]-18a-homo-5α-estran-5,17β-diol

Zu 30 ml einer einmolaren Lösung von Lithiumaluminiumhydrid in Tetrahydrofuran tropft man eine Lösung von 1,1 g der unter 31a) beschriebenen Verbindung in 25 ml Diethylether und erhitzt für sechs Stunden unter Rückfluß. Anschließend tropft man unter Eiskühlung 10 ml Aceton und danach 50 ml gesättigte Natriumsulfatlösung zu, saugt von den Feststoffen ab, wäscht mit Dichlormethan nach, trocknet die vereinigten organischen Phasen über Natriumsulfat, engt im Vakuum ein und reinigt das zurückbleibende Harz säulenchromatographisch an Kieselgel mit einem Gemisch aus Hexan/Ethylacetat. Man erhält 230 mg 31b) als weißen Schaum.
¹H-NMR (CDCl₃): δ= 5,71 ppm dbr (J=10 Hz, 1H, Bügel); 5,48 m (1H, Bügel); 5,42 dd (J=7, 7 Hz, 1H, Allen); 4,88 m (2H, Allen); 4,40 s (1H, OH); 3,48-3,63 m (4H, Ketal); 2,46 m (1H, H-11); 1,08 t (J=7.5 Hz, 3H, 18a-CH₃); 0,98 s (6H, Me-Ketal)

### c) 9,11α-Dihydro-17β-hydroxy-17α-(1,2-propadienyl)-6'H-benzo[10,9,11]-18a-homo-estr-4-en-3-on

180 mg der unter 31b) beschriebenen Substanz werden gemäß der allgemeinen Vorschrift 1) in Aceton mit 0,38 ml 4 normaler wäßriger Salzsäure umgesetzt Nach Aufreinigung des Rohproduktes erhält man 135 mg 31c) als weißen Schaum.
¹H-NMR (CDCl₃): δ= 5,80 ppm sbr (1H, H-4); 5,77 dbr (J=10 Hz, 1H, Bügel); 5,57 m (1H, Bügel); 5,41 dd (J=7, 7 Hz, 1H, Allen); 4,90 d (J=7 Hz, 2H, Allen); 2,54 m (1H, H-11); 1,12 t (J=7.5 Hz, 3H, 18a-CH₃)

### Beispiel 32

### 9,11α-Dihydro-17β-hydroxy-17α-(3-methyl-1,2-butadienyl)-6'H-benzo[10,9,11]-18a-homoestr-4-en-3-on

### a) 9,11α-Dihydro-3,3-[2,2-dimethyl-1,3-propandiylbis(oxy)]-17α-[3-methyl-3-[(tetrahydro-2H-pyran-2-yl)oxy]-1-butinyl]-6'H-benzo[10,9,11]-18a-homo-5a-estran-5,17β-diol

Aus 19,5 ml 3-Methyl-3-[(tetrahydro-2H-pyran-2-yl)oxy]-1-butin in 570 ml absolutem Tetrahydrofuran und 72,5 ml einer 1,6 molaren Lösung von n-Butyllithium in Hexan stellt man bei 0°C unter Argon die lithiumorganische Verbindung her. Anschließend wird eine Lösung von 5 g der unter 25i) beschriebenen Substanz in 120 ml absolutem Tetrahydrofuran addiert. Man läßt 30 Minuten bei 0°C nachrühren und arbeitet dann wäßrig (C,F) auf. Man erhält nach Aufreinigung 4,22 g 32a) als weißen Schaum. [α]_{D}²⁰= -47,4° (CHCl₃; c=0,535)
¹H-NMR (CDCl₃): δ= 5,70 ppm dbr (J=10 Hz,1H, Bügel); 5,48 m (1H, Bügel); 5,00 m (1H, THP); 4,30 s (1H, OH); 3,96 m (1H, THP); 3,42-3,65 m (5H, Ketal und THP); 2,48 m (1H, H-11); 1,52 s (3H, Me); 1,49 s (3H, Me); 1,05 t (J=7.5 Hz, 3H, 18a-CH₃); 0,97 s (3H, Me-Ketal); 0,95 s (3H, Me-Ketal)

### b) 9,11α-Dihydro-3,3-[2,2-dimethyl-1,3-propandiylbis(oxy)]-17α-(3-methyl-1,2-butadienyl)-6'H-benzo[10,9,11]-18a-homo-5α-estran-5,17β-diol

Eine Suspension von 3,8 g Lithiumaluminiumhydrid in 70 ml Diethylether wird eine Stunde unter Rückfluß erhitzt. Dann tropft man bei Raumtemperatur binnen 30 Minuten eine Lösung von 4 g der unter 32a) beschriebenen Verbindung in 100 ml Diethylether zu und erhitzt anschließend für zwei Stunden unter Rückfluß. In die Reaktionsmischung rührt man danach 40 g Natriumsulfat Decahydrat ein, rührt eine Stunde nach, saugt über Celite ab, wäscht mit Dichlormethan nach und zieht das Lösungsmittel im Vakuum ab. Man erhält nach Aufreinigung 1,2 g 32b) als weiße Kristalle.
Fp= 155°C, [α]_{D}²⁰= -54,0° (CHCl₃; c=0,525)
¹H-NMR (CDCl₃): δ= 5,72 ppm dbr (J=10 Hz, 1H, Bügel); 5,48 m (1H, Bügel); 5,42 m (1H, Allen); 4,38 s (1H, OH); 3,46-3,64 m (4H, Ketal); 2,45 m (1H, H-11); 1,75 d(J=2 Hz, 3H, Me-Allen); 1,74 d (J=2 Hz, 3H, Me-Allen); 1,08 t (J=7.5 Hz, 3H, 18a-CH₃); 0,99 s (3H, Me-Ketal); 0,95 s (3H, Me-Ketal)

### c) 9,11α-Dihydro-17β-hydroxy-17α-(3-methyl-1,2-butadienyl)-6' H-benzo[10,9,11]-18a-homoestr-4-en-3-on

Gemäß der allgemeinen Vorschrift 1) werden 620 mg 32b) mit 4 normaler Salzsäure in Aceton umgesetzt. Nach einstündigem Rühren bei Raumtemperatur arbeitet man auf (B,F) und erhält nach Aufreinigung des Rohproduktes 177 mg 36b) als weiße Kristalle.
Fp= 150-152°C, [α]_{D}²⁰= -68,5° (CHCl₃; c=0,460)
¹H-NMR (CDCl₃): δ= 5,78 ppm sbr (1H, H-4); 5,76 dbr (J=10 Hz, 1H, Bügel); 5,55 m (1H, Bügel); 5,20 m (1H, Allen); 2,52 m (1H, H-11); 1,75 d (J=2.5 Hz, 3H, Me-Allen); 1,72 d (J=2.5 Hz, 3H, Me-Allen); 1,12 t (J=7.5 Hz, 3H, 18a-CH₃)

### Beispiel 33

### 5",4",9,11α-Tetrahydrospiro[6'H-benzo[10,9,11]-18a-homoestr-4-en-17β,2"(5")-furan]-3,5"-dion

### a) 5",4",9,11α-Tetrahydro-3,3-[2,2-dimethyl-1,3-propandiylbis(oxy)][spiro[5-hydroxy-6'H-benzo[10,9,11]-18a-homo-5α-estran-17β,2"(5")-furan]-5-ol-5"-on

Zu einer Mischung aus 70 ml Tetrahydrofuran und 60 ml einer 1,6 molaren Lösung von n-Butyllithium in n-Hexan tropft man bei -50°C binnen 20 Minuten eine Lösung aus 8,6 ml Allyltetramethylphosphordiamidat in 40 ml Tetrahydrofuran und rührt eine Stunde bei -30°C nach. Anschließend tropft man eine Lösung aus 2 g der unter 25i) beschriebenen Verbindung in 15 ml Tetrahydrofuran zu, läßt innerhalb einer Stunde auf Raumtemperatur erwärmen und rührt vier Stunden nach. Nach wäßriger Aufarbeitung (C,F) und Aufreinigung erhält man 995 mg 33a) als weißen Schaum.
¹H-NMR (CDCl₃): δ= 5,70 ppm dbr (J=10 Hz, 1H, Bügel); 5,50 m (1H, Bügel); 4,42 s (1H, OH); 3,48-3,63 m (4H, Ketal); 1,03 t (J=7.5 Hz, 3H, 18a-CH₃); 0,98 s (6H, Me-Ketal)

### b) 5",4",9,11α-Tetrahydrospiro[6'H-benzo[10,9,11]-18a-homoestr-4-en-17β,2"(5")-furan]-3,5"-dion

Gemäß der allgemeinen Vorschrift 1) werden 818 mg der unter 33a) beschriebenen Substanz mit 2,3 ml 4 normaler wäßriger Salzsäure in Aceton umgesetzt. Man erhält nach Aufreinigung des Rohproduktes 441 mg 33b) als weiße Kristalle.
Fp= 100-102°C; [α]_{D}²⁰= +23,75° (CHCl₃; c=0,500)
¹H-NMR (CDCl₃): δ= 5,82 ppm sbr (1H, H-4); 5,75 dbr (J=10 Hz, 1H, Bügel); 5,58 m (1H, Bügel); 2,68 m (1H, 11-H); 1,05 t (J=7.5 Hz, 3H, 18a-CH₃)

### Beispiel 34

### 9,11α-Dihydro-17α-ethinyl-17β-hydroxy-6'H-benzo[10,9,11]-18a-homoestra-4,15-dien-3-on

### a) 9,11α-Dihydro-3,3-[2,2-dimethyl-1,3-propandiylbis(oxy)]-17-[(trimethylsilyl)oxy]-6'H-benzo[10,9,11]-18a-homo-5α-estr-16-en-5-ol

Gemäß der allgemeinen Vorschrift 4a) werden 2,15 g der unter 25i) beschriebenen Substanz mit 2,5 ml Diisopropylamin, 11 ml einer 1,6 molaren Lösung von n-Butyllithium in Hexan und 3,1 ml Trimethylchlorsilan in absolutem Tetrahydrofuran umgesetzt. Man erhält als Rohprodukt 2,52g 34a), das ohne weitere Reinigung in die nächste Stufe eingesetzt wird.
Fp= 172-174°C
¹H-NMR (CDCl₃): δ= 5,58 ppm dbr (J=10 Hz, 1H, Bügel); 5,44 m (1H, Bügel); 4,46 m (1H, H-16); 4,37 s (1H, OH); 3,48-3,64 m (4H, Ketal); 2,48 m (1H, H-11); 0,98 s (3H, Me-Ketal); 0,96 s (3H, Me-Ketal); 0,87 t (J=7.5 Hz, 3H, 18a-CH₃); 0,18 s (9H, Me-Si)

### b) 9,11α-Dihydro-3,3-[2,2-dimethyl-1,3-propandiylbis(oxy)]-5-hydroxy-6'H-benzo-[10,9,11]-18a-homo-5α-estr-15-en-17-on

Gemäß der allgemeinen Vorschrift 4b) werden 2,42 g der unter 34a) beschriebenen Verbindung mit 1,19 g Palladium(II)acetat in Acetonitril umgesetzt. Man erhält nach Aufreinigung 1,34 g 34b) als weiße Kristalle.
Fp= 210-212°C; [α]_{D}²⁰= -128,5° (CHCl₃; c=0,505)
¹H-NMR (CDCl₃): δ= 7,54 ppm dbr (J=6 Hz, 1H, H-15); 5,96 dd (J=6, 3 Hz, 1H, H-16); 5,63 dbr (J=10 Hz, 1H, Bügel); 5,52 m (1H, Bügel); 4,47 s (3H, OH); 3,46-3,63 m (4H, Ketal); 2,56 m (1H, H-11); 0,98 s (3H, Me-Ketal); 0,96 s (3H, Me-Ketal); 0,78 t (J=7.5 Hz, 3H, 18a-CH₃)

### c) 9,11α-Dihydro-3,3-[2,2-dimethyl-1,3-propandiylbis(oxy)]-17α-ethinyl-6'H-benzo-[10,9,11]-18a-homo-5α-estr-15-en-5,17β-diol

Gemäß der allgemeinen Vorschrift 2) werden 1,0 g 34b), 15 ml einer 1,6 molaren Lösung von n-Butyllithium in Hexan sowie Ethingas in absolutem Tetrahydrofuran umgesetzt. Man erhält 1,23 g 34c), welches ohne Reinigung in die Folgestufe eingesetzt wird.
¹H-NMR (CDCl₃): δ= 5,97 ppm dbr (J=6 Hz, 1H, H-15); 5,63 m (2H, H-16 und Bügel); 5,48 m (1H, Bügel); 4,42 s (1H, OH); 3,50-3,65 m (4H, Ketal); 2,62 s (1H, Ethin); 2,58 m (1H, H-11); 0,98 s (6H, Me-Ketal); 0,82 t (J=7.5 Hz, 3H, 18a-CH₃)

### d) 9,11 1α-Dihydro-17α-ethinyl-17β-hydroxy-6'H-benzo[10,9,11]-18a-homostra-4,15-dien-3-on

Gemäß der allgemeinen Vorschrift 1) werden 1,1 g 34c) mit 4 normaler Salzsäure in Aceton umgesetzt. Man erhält nach Aufreinigung 620 mg 34d).
Fp= 201-203°C; [α]_{D}²⁰= -198,0° (CHCl₃; c=0,510)
¹H-NMR (CDCl₃): δ= 5,97 dbr (J=6 Hz, 1H, H-15); 5,82 sbr (1H, H-4); 5,68 dd (J=6, 3 Hz, 1H, H-16); 5,66 m (1H, Bügel); 5,58 m (1H, Bügel); 2,75 m (1H, H-11); 2,66 s s (1H, Ethin); 0,86 t (J=7.5 Hz, 3H, 18a-CH₃)

### Beispiel 35

### 9,11α-Dihydro-17β-hydroxy-17α-(1-propinyl)-6'H-benzo[10,9,11]-18a-homostra-4,15-dien-3-on

### a) 9,11α-Dihydro-3,3-[2,2-dimethyl-1,3-propandiylbis(oxy)]-17α-(1-propinyl)-6'H-benzo[10,9,11]-18a-homo-5α-estr-15-en-5,17β-diol

Gemäß der allgemeinen Vorschrift 2) werden 600 mg 34b), 8,7 ml einer 1,6 molaren Lösung von n-Butyllithium in Hexan sowie Propingas in absolutem Tetrahydrofuran umgesetzt. Man erhält 715 mg 35a), welches ohne Reinigung in die Folgestufe eingesetzt wird.
¹H-NMR (CDCl₃): δ= 5,90 ppm dbr (J=6 Hz, 1H, H-15); 5,62 m (2H, H-16 und Bügel); 5,48 m (1H, Bügel); 4,46 s (1H, OH); 3,48-3,63 m (4H, Ketal); 2,56 m (1H, H-11); 1,90 s (3H, Propin); 0,98 s (3H, Me-Ketal); 0,97 s (3H, Me-Ketal); 0,82 t (J=7.5 Hz, 3H, 18a-CH₃)

### b) 9,11α-Dihydro-5,17β-dihydroxy-17α-(1-propinyl)-6'H-benzo[10,9,11]-18a-homo-5α-estr-15-en-3-on

542 mg der unter 35a) beschriebenen Verbindung werden in 10 ml Aceton gelöst, mit 1,2 ml 0,5 normaler Salzsäure versetzt und 2,5 Stunden bei Raumtemperatur gerührt. Man gießt auf gesättigte Natriumhydrogencarbonatlösung, extrahiert dreimal mit Dichlormethan und trocknet die vereinigten organischen Phasen über Natriumsulfat. Nach Abziehen des Lösungsmittels erhält man 438 mg 35b), die ohne Reinigung in der nächsten Stufe eingesetzt werden.
¹H-NMR (CDCl₃): δ= 5,90 dbr (J=6 Hz, 1H, H-15); 5,72 dbr (J=10 Hz, 1H, Bügel); 5,65 dd (J=6, 3 Hz, 1H, H-16); 5,56 m (1H, Bügel); 2,64 m (1H, H-11); 1,92 s (3H, Propin); 0,85 t (J=7.5 Hz, 3H, 18a-CH₃)

### c) 9,11α-Dihydro-17β-hydroxy-17α-(1-propinyl)-6'H-benzo[10,9,11]-18a-homostra-4,15-dien-3-on

400 mg der unter 35b) beschriebenen Verbindung werden in 10 ml Aceton gelöst, mit 0,5 ml 4 normaler Salzsäure versetzt und 5 Stunden bei Raumtemperatur gerührt. Nach wäßriger Aufarbeitung (B,F) und Aufreinigung erhält man 139 mg 35c) als weiße Kristalle.
Fp= 68-73°C; [α]_{D}²⁰= -53,2° (CHCl₃; c=0,500)
¹H-NMR (CDCl₃): δ= 5,90 dbr (J=6 Hz, 1H, H-15); 5,83 sbr (1H, H-4); 5,68 m (1H, Bügel); 5,66 m (1H, H-16); 5,58 m (1H, Bügel); 2,72 m (1H, H-11); 1,90 s (3H, Propin); 0,86 t (J=7.5 Hz, 3H, 18a-CH₃)

### Beispiel 36

### 17α-(1-Butinyl)-9,11α-dihydro-17β-hydroxy-6'H-benzo[10,9,11]-18a-homostra-4,15-dien-3-on

### a) 17α-(1-Butinyl)-9,11α-dihydro-3,3-[2,2-dimethyl-1,3-propandiylbis(oxy)]-6'H-benzo-[10,9,11]-18a-homo-5α-estr-15-en-5,17β-diol

Gemäß der allgemeinen Vorschrift 2) werden 800 mg 34b), 15 ml einer 1,6 molaren Lösung von n-Butyllithium in Hexan sowie 5 g 1-Butin in absolutem Tetrahydrofuran umgesetzt. Man erhält nach Aufreinigung 637 mg 36a) als weißen Schaum.
¹H-NMR (CDCl₃): δ= 5,90 ppm dbr (J=6 Hz, 1H, H-15); 5,63 m (2H, H-16 und Bügel); 5,48 m (1H, Bügel); 4,43 s (1H, OH); 3,46-3,63 m (4H, Ketal); 2,25 q (J= 7.5 Hz, 2H, Butin-CH₂); 1,16 t (J=7.5 Hz, 3H, Butin-CH₃); 0,98 s (3H, Me-Ketal); 0,96 s (3H, Me-Ketal); 0,81 t (J=7.5 Hz, 3H, 18a-CH₃)

### b) 17α-(1-Butinyl)-9,11α-dihydro-17β-hydroxy-6'H-benzo[10,9,11]-18a-homostra-4,15-dien-3-on

Gemäß der allgemeinen Vorschrift 1) werden 364 mg 36a) mit 4 normaler Salzsäure in Aceton umgesetzt. Nach dreistündigem Rühren bei Raumtemperatur arbeitet man auf (B,F) und erhält nach Aufreinigung des Rohproduktes 145 mg 36b) als weißen Schaum. [α]_{D}²⁰=-195,2°(CHCl₃; c=0,500)
¹H-NMR (CDCl₃): δ= 5,87 dbr (J=6 Hz, 1H, H-15); 5,82 sbr (1H, H-4); 5,68 m (2H, H-16 und Bügel); 5,56 m (1H, Bügel); 2,72 m (1H, H-11); 2,26 q (J= 7.5 Hz, 2H, Butin-CH₂); 1,14 t (J=7.5 Hz, 3H, Butin-CH₃); 0,85 t (J=7.5 Hz, 3H, 18a-CH₃)

### Beispiel 37

### 9,11α-Dihydro-17β-hydroxy-17α-(1,3-pentadiinyl)-6'H-benzo[10,9,11]-18a-homostra-4, 15-dien-3-on

300 mg der unter 34d) beschriebenen Substanz werden in 40 ml Triethylamin gelöst. Man sättigt die Lösung bei Raumtemperatur mit Propingas, addiert 90 mg Tetrakis(triphenylphosphin)palladium und 45 mg Kupfer(I)iodid, erwärmt auf 60°C und läßt unter Aufrechterhaltung des Propinstromes eine Stunde bei dieser Temperatur nachrühren. Anschließend wird die Reaktionslösung über Celite filtriert und im Vakuum eingeengt. Nach Aufreinigung erhält man 66 mg 37) als amorphen Feststoff.
¹H-NMR (CDCl₃): δ= 5,95 dbr (J=6 Hz, 1H, H-15); 5,82 sbr (1H, H-4); 5,68 m (1H, Bügel); 5,63 dd (J=6, 3 Hz, 1H, H-16); 5,56 m (1H, Bügel); 2,74 m (1H, H-11); 1,96 s (3H, Pentadiin-Me); 0,82 t (J=7.5 Hz, 3H, 18a-CH₃)

### Beispiel 38

### 9,11α,15α,16α-Tetrahydro-17α-ethinyl-17β-hydroxy-6'H-benzo[10,9,11][3"H]cyclopropa[15,16]-18a-homoestr-4-en-3-on

### a) 9,11α,15α,16α-Tetrahydro-3,3-[2,2-dimethyl-1,3-propandiylbis(oxy)]-5-hydroxy-6'H-benzo[10,9,11][3"H]cyclopropa[15,16]-18a-homo-5α-estran-17-on

Zu einer Suspension von 3,68 g Trimethylsulfoxoniumjodid in 50 ml Dimethylsulfoxid gibt man 430 mg Natriumhydrid, rührt 90 Minuten, addiert dann 2 g der unter 34b) beschriebenen Substanz und läßt sechs Stunden rühren. Das Gemisch wird in Eiswasser eingerührt; das ausgefallene Material wird abgesaugt, in Dichlormethan aufgenommen, über Natriumsulfat getrocknet und das Lösungsmittel im Vakuum abgezogen. Man erhält 1,67 g 38a) als weiße Kristalle.
Fp= 226-230°C, [α]_{D}²⁰= -70,0° (CHCl₃; c=0,500)
¹H-NMR (CDCl₃): δ= 5,56 ppm dbr (J=10 Hz, 1H, Bügel); 5,48 m (1H, Bügel); 4,45 s (1H, OH); 3,46-3,64 m (4H, Ketal); 2,54 s (1H, H-11); 1,00 s (3H, Me-Ketal); 0,97 s (3H, Me-Ketal); 0,76 t (J=7.5 Hz, 3H, 18a-CH₃)

### b) 9,11α,15α,16α-Tetrahydro-3,3-[2,2-dimethyl-1,3-propandiylbis(oxy)]-17α-ethinyl-6'H-benzo[10,9,11][3"H]cyclopropa[15,16]-18a-homo-5α-estran-5,17β-diol

Gemäß der allgemeinen Vorschrift 2) werden 1,0 g der unter 38a) beschriebenen Substanz und 14 ml einer 1,6 molaren Lösung von n-Butyllithium in Hexan sowie Ethingas in absolutem Tetrahydrofuran umgesetzt. Man erhält nach Aufreinigung 949 mg 38b) als weiße Kristalle.
Fp= 197-202°C, [α]_{D}²⁰= -86,8° (CHCl₃; c=0,530)
¹H-NMR (CDCl₃): δ= 5,72 ppm dbr (J=10 Hz, 1H, Bügel); 5,47 m (1H, Bügel); 4,40 s (1H, OH); 3,48-3,65 m (4H, Ketal); 2,70 s (1H, Ethin); 2,50 m (1H, H-11); 0,99 t (J=7.5 Hz, 3H, 18a-CH₃); 0,97 s (6H, Me-Ketal); 0,81 m (1H, Cyclopropyl-H)

### c) 9,11α,15α,16α-Tetrahydro-17α-ethinyl-17β-hydroxy-6'H-benzo[10,9,11][3"H]cyclopropa[15,16]-18a-homoestr-4-en-3-on

Gemäß der allgemeinen Vorschrift 1) werden 650 mg 38b) mit 4 normaler Salzsäure in Aceton umgesetzt. Man erhält nach Aufreinigung 348 mg 38c).
Fp= 227-230°C; [α]_{D}²⁰= -82,9° (CHCl₃; c=0,505)
¹H-NMR (CDCl₃): δ= 5,83 sbr (1H, H-4); 5,78 dbr (J=10 Hz, 1H, Bügel); 5,56 m (1H, Bügel); 2,72 s (1H, Ethin); 1,02 t (J=7.5 Hz, 3H, 18a-CH₃); 0,48 m (1H, Cyclopropyl-H)

### Beispiel 39

### 17-(Acetyloxy)-9,11α-dihydro-6'H-benzo[10,9,11]-18a-homo-19-norpregn-4-en-3,20-di-on

### a) [17(21)S]-21-Phenylsufinyl)-9,11α-dihydro-3,3-[2,2-dimethyl-1,3-propandiylbis-(oxy)]-5-hydroxy-6'H-benzo[10,9,11]-18a-homo- 19-nor-5α-pregna- 17(20),20-dien

Zu einer Lösung von 500 mg der unter 25i) beschriebenen Substanz in 7 ml Dichlormethan und 1 ml Triethylamin tropft man bei -70°C eine Lösung von 285 mg Phenylsulfenylchlorid in 0,7 ml Dichlormethan, läßt anschließend auf -30°C erwärmen und rührt drei Stunden bei -30°C. Man versetzt die Reaktionsmischung mit Wasser, extrahiert zweimal mit Ethylacetat, wäscht die vereinigten organischen Phasen mit 1 normaler wäßriger Salzsäure und Wasser, trocknet über Natriumsulfat und engt im Vakuum ein. Nach Aufreinigung erhält man 445 mg 39a).
¹H-NMR (CDCl₃): δ= 7,68 ppm m (2H, Aromat); 7,50 m (3H, Aromat); 6,06 t (J=3.5 Hz, 1H, Allen); 5,60 dbr (J=10 Hz, 1H, Bügel); 5,50 m (1H, Bügel); 4,37 s (1H, OH); 3,48-3,63 m (4H, Ketal); 0,97 s (3H, Me-Ketal); 0,95 s (3H, Me-Ketal); 0,93 t (J=7.5 Hz, 3H, 18a-CH₃)

### b) 9,11α-Dihydro-3,3-[2,2-dimethyl-1,3-propandiylbis(oxy)]-5,17-dihydroxy-20-(methyloxy)-6'H-benzo[10,9,11]-18a-homo- 19-nor-5α-pregn-20-en

Zu einer Lösung von 3,3 g der unter 39a) beschriebenen Verbindung in 30 ml Tetrahydrofuran tropft man 24 ml einer 1 molaren Lösung von Natriummethanolat in Methanol, läßt 48 Stunden bei Raumtemperatur rühren, gießt anschließend auf Wasser, extrahiert dreimal mit Dichlormethan, trocknet die vereinigten organischen Phasen über Natriumsulfat, filtriert und zieht das Lösungsmittel im Vakuum ab. Den Rückstand löst man in 65 ml Methanol, setzt 10 ml Triethylphosphit zu, erhitzt für 45 Minuten unter Rückfluß und engt dann im Vakuum ein. Nach Aufreinigung erhält man 2,1 g 39b).
¹H-NMR (CDCl₃): δ= 5,67 ppm dbr (J=10 Hz, 1H, Bügel); 5,43 m (1H, Bügel); 4,30 d (J=4 Hz, 1H, H-21); 4,30 s (1H, OH); 3,98 d (J=4 Hz, 1H, H-21); 3,52 s (3H, MeO); 3,45-3,64 m (4H, Ketal); 0,97 s (3H, Me-Ketal); 0,95 s (3H, Me-Ketal); 0,72 t (J=7.5 Hz, 3H, 18a-CH₃)

### c) 9,11α-Dihydro-5-hydroxy-6'H-benzo-[10,9,11]-18a-homo-19-nor-5α-pregnan-3,20-dion

Zu einer Lösung von 2,0 g der unter 39b) beschriebenen Verbindung in 25 ml Aceton gibt man 2 ml 4 normale wäßrige Salzsäure, rührt eine Stunde bei Raumtemperatur und saugt die ausgefallene Substanz ab. Man erhält 1,31 g 39c), das ohne Reinigung in der nächsten Stufe eingesetzt wird.
¹H-NMR (D6-DMSO): δ= 5,76 ppm dbr (J=10 Hz, 1H, Bügel); 5,62 m (1H, Bügel); 5,17 s (1H, OH); 4,40 s (1H, OH); 2,25 s (3H, Acetyl); 0,70 t (J=7.5 Hz, 3H, 18a-CH₃)

### d) 17-(Acetyloxy)-9,11α-dihydro-6'H-benzo[10,9,11]-18a-homo-19-norpregn-4-en-3,20-dion

Analog zu Beispiel 13c) werden 1,3 g der unter 39c) beschriebenen Verbindung in 25 ml Eisessig mit 6,6 ml Trifluoressigsäureanhydrid umgesetzt. Nach wäßriger Aufarbeitung (B,F) und Aufreinigung erhält man 1,0 g 39d).
Fp= 225-229°C
¹H-NMR (CDCl₃): δ= 5,82 ppm sbr (1H, H-4); 5,70 dbr (J=10 Hz, 1H, Bügel); 5,58 m (1H, Bügel); 2,72 m (1H, H-11); 2,12 s (3H, Acetyl); 2,10 s (3H, Acetoxy); 0,72 t (J=7.5 Hz, 3H, 18a-CH₃)

### Beispiel 40

### 17-(Acetyloxy)-9,11α-dihydro-6-methylen-6'H-benzo[10,9,11]-18a-homo-19-norpregn-4-en-3,20-dion

1,41 g der unter Beispiel 39d) beschriebenen Substanz werden analog zu der in Beispiel 16) angegebenen Vorschrift umgesetzt. Man erhält 1,03 g 40) als weiße Kristalle.
Fp= 215-219°C, [α]_{D}²⁰= +126,4° (CHCl₃; c=0,500)
¹H-NMR (CDCl₃): δ= 6,01 ppm sbr (1H, H-4); 5,70 dbr (J=10 Hz, 1H, Bügel); 5,55 m (1H, Bügel); 5,15 m (1H, exo-Methylen), 5,00 m (1H, exo-Methylen); 2,74 m (1H, H-11); 2,14 s (3H, Acetyl); 2,12 s (3H, Acetoxy); 0,72 t (J=7.5 Hz, 3H, 18a-CH₃)

### Beispiel 41

### 17-(Acetyloxy)-9,11α-dihydro-6-methyl-6'H-benzo[10,9,11]-18a-homo-19-norpregna-4,6-dien-3,20-dion

423 mg der unter Beispiel 40) beschriebenen Substanz werden analog zu der in Beispiel 18) angegebenen Vorschrift umgesetzt. Man erhält 303 g 41) als weiße Kristalle. Fp= 260-264°C, [α]_{D}²⁰= +43,4° (CHCl₃; c=0,500)
¹H-NMR (CDCl₃): δ= 6,00 ppm sbr (1H, H-7); 5,92 sbr (1H, H-4); 5,72 dbr (J=10 Hz, 1H, Bügel); 5,62 m (1H, Bügel); 2,75 m (1H, H-11); 2,15 s (3H, Acetyl); 2,11 s (3H, Acetoxy); 1,86 sbr (3H, 6-Methyl); 0,73 t (J=7.5 Hz, 3H, 18a-CH₃)

### Beispiel 42

### 5',6'-Dihydro-17β-hydroxy-17α-(1-propinyl)-9H-benzo[10,9,11]-18a-homoestr-4-en-3-on

### a) 5',6'-Dihydro-3,3-[2,2-dimethyl-1,3-propandiylbis(oxy)]-9H-benzo[10,9,11]-18a-homo-5α-estran-5,17β-diol

2 g der unter 25h) beschriebenen Verbindung werden in einer Schüttelente in einer Mischung aus 5 ml Tetrahydrofuran und 50 ml Ethylacetat gelöst. Man addiert 550 mg Palladium auf Aktivkohle (10%ig), setzt die Apparatur unter Wasserstoff und schüttelt für drei Stunden. Anschließend wird über Celite filtriert und im Vakuum eingeengt. Man erhält nach Aufreinigung 1,69 g 42a).
¹H-NMR (CDCl₃): δ= 5,30 ppm m (1H, Bügel); 4,38 s (1H, OH); 3,85 dd (J=14, 7.5 Hz, 1H, 17-H); 3,50-3,65 m (4H, Ketal); 1,04 t (J=7.5 Hz, 3H, 18a-CH₃); 0,98 s (6H, Me-Ketal)

### b) 5',6'-Dihydro-3,3-[2,2-dimethyl-1,3-propandiylbis(oxy)]-5-hydroxy-9H-benzo-[10,9,11]-18a-homo-5α-estran-17-on

Gemäß der allgemeinen Vorschrift 3) werden aus 1,68 g der unter 42a) beschriebenen Substanz, 2,35 g Chromtrioxid und 8 ml Pyridin in 70 ml Dichlormethan 1,50 g 42b) erhalten.
¹H-NMR (CDCl₃): δ= 5,28 ppm m (1H, Bügel); 4,40 s (1H, OH); 3,48-3,60 m (4H, Ketal); 1,00 s (3H, Me-Ketal); 0,96 s (3H, Me-Ketal); 0,76 t (J=7.5 Hz, 3H, 18a-CH₃)

### c) 5',6'-Dihydro-3,3-[2,2-dimethyl-1,3-propandiylbis(oxy)]-17α-(1-propinyl)-9H-benzo-[10,9,11]-18a-homo-5α-estran-5,17β-diol

Gemäß der allgemeine Vorschrift 2) werden aus 1,35 g der unter 42b) beschriebenen Substanz und 19,5 ml einer 1,6 molaren Lösung von n-Butyllithium in Hexan sowie Propingas 1,02 g 42c) erhalten.
[α]_{D}²⁰= -78,8° (CHCl₃; c=0,510)
¹H-NMR (CDCl₃): δ= 5,31 ppm m (1H, Bügel); 4,44 s (1H, OH); 3,45-3,62 m (4H, Ketal); 1,87 s (3H, Propin); 1,02 t (J=7.5 Hz, 3H, 18a-CH₃); 0,98 s (3H, Me-Ketal); 0,95 s (3H, Me-Ketal)

### d) 5',6'-Dihydro-17β-hydroxy-17α-(1-propinyl)-9H-benzo[10,9,11]-18a-homoestr-4-en-3-on

Gemäß der allgemeinen Vorschrift 1) werden 0,99 g 42c) mit 4 normaler Salzsäure in Aceton umgesetzt. Man erhält nach Aufreinigung des Rohproduktes 421 mg 42d).
Fp= 188-190°C, [α]_{D}²⁰= +20,3° (CHCl₃; c=0,515)
¹H-NMR (CDCl₃): δ= 5,78 ppm sbr (1H, H-4); 5,48 m (1H, Bügel); 1,88 s (3H, Propin); 1,05 t (J=7.5 Hz, 3H, 18a-CH₃)

### Beispiel 43

### 17β-Hydroxy-17α-(1-propinyl)-4',5',9,11α-tetrahydro-6'H-benzo[10,9,11]-18a-homoestr-4-en-3-on

### a) 3,3-[2,2-Dimethyl-1,3-propandiylbis(oxy)]-4',5',9,11α-tetrahydro-6'H-benzo-[10,9,11]-18a-homo-5α-estran-5,17β-diol

2 g der unter 25h) beschriebenen Verbindung werden in einer Schüttelente in einer Mischung aus 5 ml Tetrahydrofuran und 50 ml Ethylacetat gelöst Man addiert 400 mg Platin(IV)oxid, setzt die Apparatur unter Wasserstoff und schüttelt für acht Stunden. Anschließend wird über Celite filtriert und im Vakuum eingeengt. Man erhält nach Aufreinigung 1,61 g 43a).
¹H-NMR (CDCl₃): δ= 4,28 ppm s (1H, OH); 3,68 ddbr (J=14, 7.5 Hz, 1H, 17-H); 3,46-3,62 m (4H, Ketal); 1,02 t (J=7.5 Hz, 3H, 18a-CH₃); 0,95 s (3H, Me-Ketal); 0,95 s (3H, Me-Ketal)

### b) 3,3-[2,2-Dimethyl-1,3-propandiylbis(oxy)]-5-hydroxy-4',5',9,11α-tetrahydro-6'H-benzo[10,9,11]-18a-homo-5α-estran-17-on

Gemäß der allgemeinen Vorschrift 3) werden aus 1,4 g der unter 43a) beschriebenen Substanz, 1,95 g Chromtrioxid und 6,7 ml Pyridin in 60 ml Dichlormethan 1,26 g 43b) erhalten.
¹H-NMR (CDCl₃): δ= 4,30 ppm s (1H, OH); 3,45-3,63 m (4H, Ketal); 0,98 s (3H, Me-Ketal); 0,96 s (3H, Me-Ketal); 0,84 t (J=7.5 Hz, 3H, 18a-CH₃)

### c) 3,3-[2,2-Dimethyl-1,3-propandiylbis(oxy)]-17α-(1-propinyl)-4',5',9,11α-tetrahydro-6'H-benzo[10,9,11]-18a-homo-5α-estran-5,17β-diol

Gemäß der allgemeine Vorschrift 2) werden aus 1,2 g der unter 43b) beschriebenen Substanz und 29 ml einer 1,6 molaren Lösung von n-Butyllithium in Hexan sowie Propingas 761 mg 43c) erhalten.
[α]_{D}²⁰= -5,1° (CHCl₃; c=0,505)
¹H-NMR (CDCl₃): δ= 4,34 ppm s (1H, OH); 3,45-3,62 m (4H, Ketal); 1,87 s (3H, Propin); 1,02 t (J=7.5 Hz, 3H, 18a-CH₃); 0,98 s (3H, Me-Ketal); 0,95 s (3H, Me-Ketal)

### d) 17β-Hydroxy-17α-(1-propinyl)-4',5',9,11α-tetrahydro-6'H-benzo[10,9,11]-18a-homoestr-4-en-3-on

Gemäß der allgemeinen Vorschrift 1) werden 700 mg 43c) mit 4 normaler Salzsäure in Aceton umgesetzt. Man erhält nach Aufreinigung des Rohproduktes 463 mg 43d).
[α]_{D}²⁰= +71,7° (CHCl₃; c=0,510)
¹H-NMR (CDCl₃): δ= 5,73 ppm sbr (1H, H-4); 1,88 s (3H, Propin); 1,06 t (J=7.5 Hz, 3H, 18a-CH₃)

### Beispiel 44

### 5',6'-Dihydro-17α-ethinyl-17β-hydroxy-9H-benzo[10,9,11]-18a-homoestra-4,15-dien-3-on

### a) 5',6'-Dihydro-3,3-[2,2-dimethyl-1,3-propandiylbis(oxy)]-17-[(trimethylsilyl)oxy]-9H-benzo[10,9,11]-18a-homo-5α-estr-16-en-5-ol

Gemäß der allgemeinen Vorschrift 4a) werden 4,1 g der unter 42b) beschriebenen Substanz mit 47 ml Diisopropylamin, 20 ml einer 1,6 molaren Lösung von n-Butyllithium in Hexan und 5,7 ml Trimethylchlorsilan in absolutem Tetrahydrofuran umgesetzt. Man erhält als Rohprodukt 5,0 g 44a).
¹H-NMR (CDCl₃): δ= 5,21 ppm m (1H, Bügel); 4,52 m (1H, H-16); 4,35 s (1H, OH); 3,45-3,64 m (4H, Ketal); 0,95 s (6H, Me-Ketal); 0,83 t (J=7.5 Hz, 3H, 18a-CH₃); 0,18 s (9H, Me-Si)

### b) 5',6'-Dihydro-3,3-[2,2-dimethyl-1,3-propandiylbis(oxy)]-5-hydroxy-9H-benzo-[10,9,11]-18a-homo-5α-estr-15-en-17-on

Gemäß der allgemeinen Vorschrift 4b) werden 4,99 g der unter 44a) beschriebenen Verbindung mit 2,5 g Palladium(II)acetat in Acetonitril umgesetzt. Man erhält nach Aufreinigung 3,15 g 44b).
¹H-NMR (CDCl₃): δ= 7,55 ppm dbr (J=6 Hz, 1H, H-15); 5,99 dd (J=6, 3 Hz, 1H, H-16); 5,35 m (1H, Bügel); 4,42 s (1H, OH); 3,45-3,62 m (4H, Ketal); 1,00 s (3H, Me-Ketal); 1,00 s (3H, Me-Ketal); 0,75 t (J= 7.5 Hz, 3H, 18a-CH₃)

### c) 5',6'-Dihydro-3,3-[2,2-dimethyl-1,3-propandiylbis(oxy)]-17α-ethinyl-9H-benzo-[10,9,11]-18a-homo-5α-estr-15-en-5,17β-diol

Gemäß der allgemeinen Vorschrift 2) werden 1,2 g 44b) mit 29 ml einer 1,6 molaren Lösung von n-Butyllithium sowie Ethingas in absolutem Tetrahydrofuran umgesetzt. Man erhält 1,2 g 44c).
¹H-NMR (CDCl₃): δ= 5,98 ppm dbr (J=6 Hz, 1H, H-15); 5,65 dd (J=6, 3 Hz, 1H, H-16); 5,32 m (1H, Bügel); 4,42 s (1H, OH); 3,45-3,65 m (4H, Ketal); 2,62 s (1H, Ethin); 0,98 s (3H, Me-Ketal); 0,95 s (3H, Me-Ketal); 0,82 t (J= 7.5 Hz, 3H, 18a-CH₃)

### d) 5',6'-Dihydro-17α-ethinyl-17β-hydroxy-9H-benzo[10,9,11]-18a-homoestra-4,15-dien-3-on

Gemäß der allgemeinen Vorschrift 1) werden 1,1 g 44c) mit 4 normaler Salzsäure in Aceton umgesetzt. Man erhält nach Aufreinigung des Rohproduktes 432 mg 44d).
Fp= 203-204°C, [α]_{D}²⁰= -89,9° (CHCl₃; c=0,500)
¹H-NMR (CDCl₃): δ= 5,98 ppm dbr (J=6 Hz, 1H, H-15); 5,78 sbr (1H, H-4); 5,73 dd (J=6, 3 Hz, 1H, H-16); 5,47 m (1H, Bügel); 2,65 s (1H, Ethin); 0,85 t (J=7.5 Hz, 3H, 18a-CH₃)

### Beispiel 45

### 5',6'-Dihydro-17β-hydroxy-17α-(1-propinyl)-9H-benzo[10,9,11]-18a-homoestra-4,15-dien-3-on

### a) 5',6'-Dihydro-3,3-[2,2-dimethyl- 1 ,3-propandiylbis(oxy)]-17α-(1-propinyl)-9H-benzo[10,9,11]-18a-homo-5α-estr-15-en-5,17β-diol

Gemäß der allgemeinen Vorschrift 2) werden 1,2 g 44b) mit 29 ml einer 1,6 molaren Lösung von n-Butyllithium sowie Propingas in absolutem Tetrahydrofuran umgesetzt. Man erhält 1,3 g 45a).
¹H-NMR (CDCl₃): δ= 5,92 ppm dbr (J=6 Hz, 1H, H-15); 5,62 dd (J=6, 3 Hz, 1H, H-16); 5,32 m (1H, Bügel); 4,46 s (1H, OH); 3,48-3,64 m (4H, Ketal); 1,88 s (3H, Propin); 0,97 s (3H, Me-Ketal); 0,95 s (3H, Me-Ketal); 0,82 t (J= 7.5 Hz, 3H, 18a-CH₃)

### b) 5',6'-Dihydro-17β-hydroxy-17α-(1-propinyl)-9H-benzo[10,9,11]-18a-homoestra-4,15-dien-3-on

Gemäß der allgemeinen Vorschrift 1) werden 1,1 g 45a) mit 4 normaler Salzsäure in Aceton umgesetzt. Man erhält nach Aufreinigung des Rohproduktes durch Säulenchromatographie an Kieselgel und nachfolgende HPLC 155 mg 45b).
Fp=210-217°C, [α]_{D}²⁰= -117,6° (CHCl₃; c=0,500)
¹H-NMR (CDCl₃): δ= 5,92 ppm dbr (J= 6 Hz, 1H, H-15); 5,78 sbr (1H, H-4); 5,70 dd (J=6, 3 Hz, 1H, H-16); 5,46 m (1H, Bügel); 1,91 s (3H, Propin); 0,84 t (J=7.5 Hz, 3H, 18a-CH₃)

### Beispiel 46

### 3",16β,9,11α-Tetrahydro-6'H-benzo[10,9,11]cyclopropa[16,17]-18a-homo-19-norpregn-4-en-3,20-dion

### a) 9,11α-Dihydro-17β-hydroxy-6'H-benzo[10,9,11]-18a-homoestr-4-en-3-on

Gemäß der allgemeinen Vorschrift 1) werden 7 g 25h) mit 4 normaler Salzsäure in Aceton umgesetzt. Man erhält nach Umkristallisation aus Ethylacetat 4 g 46a) als weiße Kristalle.
Fp= 172-175°C
¹H-NMR (CDCl₃): δ= 5,80 ppm sbr (1H, H-4); 5,75 dbr (J=10 Hz, 1H, Bügel); 5,58 m (1H, Bügel); 3,75 ddbr (J=14, 7.5 Hz, 1H, 17-H); 1,08 t (J=7.5 Hz, 3H, 18a-CH₃)

### b) 9,11α-Dihydro-3,3-[2,2-dimethyl-1,3-propandiylbis(oxy)]-6'H-benzo[10,9,11]-18a-homoestr-5-en-17β-ol

3,98 g 46a) werden analog zu der in Beispiel 25c) angegebenen Vorschrift umgesetzt. Man erhält nach Aufreinigung 2,59 g 46b) als weißen Schaum.
[α]^{D}₂₀= +7,4° (CHCl₃; c=0,500)
¹H-NMR (CDCl₃): δ= 5,70 ppm dbr (J=10 Hz, 1H, Bügel); 5,47 m (1H, Bügel); 5,32 m (1H, H-6); 3,68 m (1H, 17-H); 3,42-3,60 m (4H, Ketal); 1,06 t (J=7.5 Hz, 3H, 18a-CH₃); 1,02 s (3H, Me-Ketal); 0,92 s (3H, Me-Ketal)

### c) 9,11α-Dihydro-3,3-[2,2-dimethyl- 1 ,3-propandiylbis(oxy)]-6'H-benzo[10,9,11]-18a-homoestr-5-en-17-on

Gemäß der allgemeinen Vorschrift 3) werden aus 2,57 g der unter 46b) beschriebenen Substanz, 3,74 g Chromtrioxid und 13 ml Pyridin in 110 ml Dichlormethan 2,55 g 46c) als weiße Kristalle erhalten.
Fp= 190°C
¹H-NMR (CDCl₃): δ= 5,62 ppm dbr (J=10 Hz, 1H, Bügel); 5,48 m (1H, Bügel); 5,35 m (1H, H-6); 3,42-3,62 m (4H, Ketal); 1,04 s (3H, Me-Ketal); 0,92 s (3H, Me-Ketal); 0,78 t (J=7.5 Hz, 3H, 18a-CH₃)

### d) 9,11α-Dihydro-3,3-[2,2-dimethyl-1,3-propandiylbis(oxy)]-17-[[(1,1,2,2,3,3,4,4,4-nonafluorbutyl)sulfonyl]oxy]-6'H-benzo[10,9,11]-18a-homoestra-5,16-dien

2,54 g der unter 46c) beschriebenen Substanz werden in 30 ml absolutem Tetrahydrofuran gelöst. Man addiert eine Lösung von 3,11 g Kaliumbis(trimethylsilyl)-amid in 15 ml absolutem 1,2-Dimethoxyethan und 1,45 ml Perfluorbutansulfonylfluorid. Anschließend läßt man 5 Stunden bei Raumtemperatur nachrühren und arbeitet dann wäßrig (B,F) auf. Man erhält nach Aufreinigung 1,73 g 46d).
¹H-NMR (CDCl₃): δ= 5,60 ppm m (2H, H-16 und Bügel); 5,50 m (1H, Bügel); 5,35 m (1H, H-6); 3,42-3,60 m (4H, Ketal); 1,02 s (3H, Me-Ketal); 0,92 s (3H, Me-Ketal); 0,89 t (J=7.5 Hz, 3H, 18a-CH₃)

### e) 9,11α-Dihydro-3,3-[2,2-dimethyl-1,3-propandiylbis(oxy)]-6'H-benzo[10,9,11]-18a-homo-19-norpregna-5,16-dien-20-on

1,7 g der unter 46d) beschriebenen Verbindung werden in 32 ml absolutem Dimethylformamid gelöst und mit 1,25 ml (1-Ethoxyvinyl)tributylzinn, 320 mg wasserfreien Lithiumchlorid, 27 mg Palladium(II)acetat und 65 mg Triphenylphosphin versetzt. Man erhitzt für 5 Stunden auf 60°C und anschließend für 20 Minuten auf 100°C. Nach wäßriger Aufarbeitung (B,F) und Aufreinigung erhält man 682 mg 46e).
Fp= 165-168°C
¹H-NMR (CDCl₃): δ= 6,81 ppm dd (J=3, 2 Hz, 1H, H-16); 5,68 m (1H, Bügel); 5,46 m (1H, Bügel); 5,36 m (1H, H-6); 3,40-3,63 m (4H, Ketal); 2,28 s (3H, Acetyl); 1,02 s (3H, Me-Ketal); 0,90 s (3H, Me-Ketal); 0,73 t (J=7.5 Hz, 3H, 18a-CH₃)

### f) 3",16β,9,11α-Tetrahydro-3,3-[2,2-dimethyl-1,3-propandiylbis(oxy)]-6'H-benzo-[10,9,11]cyclopropa[16,17]-18a-homo-19-norpregn-5-en-20-on

Zu einer Suspension von 670 mg Trimethylsulfoxoniumiodid in 8,5 ml Dimethylsulfoxid werden 78 mg einer 80%igen Suspension von Natriumhydrid in Mineralöl addiert Man läßt 90 Minuten bei Raumtemperatur nachrühren und addiert dann eine Suspension von 672 mg der unter 46e) beschriebenen Verbindung in 2,8 ml Dimethylsulfoxid. Anschließend läßt man 10 Stunden bei Raumtemperatur nachrühren und arbeitet dann wäßrig (A,F) auf. Nach Aufreinigung erhält man 168 mg 46f).
¹H-NMR (CDCl₃): δ= 5,64 ppm dbr (J=10 Hz, 1H, Bügel); 5,45 m (1H, Bügel); 5,33 m (1H, H-6); 3,40-3,61 m (4H, Ketal); 2,03 s (3H, Acetyl); 1,00 s (3H, Me-Ketal); 0,92 s (3H, Me-Ketal); 0,73 t (J=7.5 Hz, 3H, 18a-CH₃)

### g) 3",16β,9,11α-Tetrahydro-6'H-benzo[10,9,11]cyclopropa[16,17]-18a-homo-19-norpregn-4-en-3,20-dion

154 mg 46f) werden in 8 ml Aceton gelöst, mit 0,18 ml 2 normaler Salzsäure versetzt und die Mischung für 5,5 Stunden bei Raumtemperatur gerührt. Nach wäßriger Aufarbeitung (B,F) reinigt man das Rohprodukt durch Säulenchromatographie an Kieselgel und nachfolgende HPLC und erhält 36 mg 46g).
¹H-NMR (CDCl₃): δ= 5,82 ppm sbr (1H, H-4); 5,68 dbr (J=10 Hz, 1H, Bügel); 5,56 m (1H, Bügel); 2,54 m (1H, H-11); 2,03 s (3H, Acetyl); 0,78 t (J=7.5 Hz, 3H, 18a-CH₃)

## Patentansprüche

1. 19,11-überbrückte 4-Estrene der allgemeinen Formel I 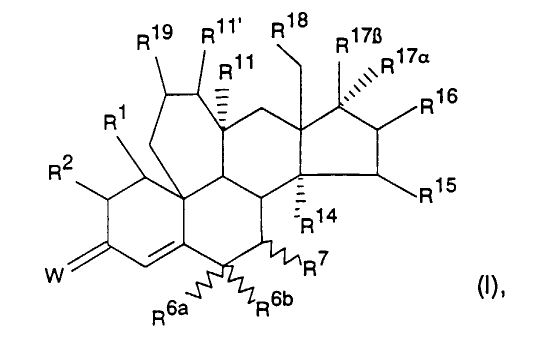 worin
W für ein Sauerstoffatom, die Hydroxyiminogruppe >N~OH oder zwei Wasserstoffatome steht,
R¹ und R² je für ein Wasserstoffatom oder gemeinsam für eine zusätzliche Bindung oder für eine α-ständige Methylenbrücke stehen,
R^{6a} und R^{6b} je für ein Wasserstoffatom oder gemeinsam für eine Methylengruppe oder gemeinsam mit dem Kohlenstoffatom 6 für einen Dreiring stehen, wobei R⁷ in diesen Fällen ein Wasserstoffatom bedeutet oder
R^{6a} für ein Wasserstoffatom oder ein Fluor-, Chlor-, Brom- oder Iodatom oder für einen gerad- oder verzweigtkettigen gesättigten α- oder β-ständigen Alkylrest mit bis zu 4 Kohlenstoffatomen, wobei dann R^{6b} und R⁷ je ein Wasserstoffatom oder gemeinsam eine zusätzliche Bindung darstellen, steht, oder
R^{6b} und R⁷ gemeinsam für eine α- oder β-ständige Methylenbrücke, wobei R^{6a} dann ein Wasserstoffatom ist, stehen,
R⁷ für einen gerad- oder verzweigtkettigen gesättigten α- oder β-ständigen Alkylrest mit bis zu 4 Kohlenstoffatomen oder für eine Thiogruppe -SR²⁰, worin R²⁰ ein Wasserstoffatom oder eine Alkanoylgruppe mit 1 bis 4 Kohlenstoffatomen darstellt, steht, wobei R^{6a} und R^{6b} dann je ein Wasserstoffatom bedeuten,
R¹⁴, R¹⁵ und R¹⁶ je für ein Wasserstoffatom oder
R¹⁴ für ein α-ständiges Wasserstoffatom sowie R¹⁵ und R¹⁶ gemeinsam für eine zusätzliche Bindung oder für eine α- oder β-ständige Methylenbrücke, oder R¹⁴ und R¹⁵ je für ein Wasserstoffatom sowie R¹⁶ für eine α- oder β-ständige C₁-C₄-Alkylgruppe oder R¹⁶ gemeinsam mit R^{17α} für eine α-ständige Methylenbrücke und R^{17β} für eine Gruppe 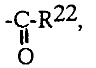
R¹⁶ für ein Wasserstoffatom sowie R¹⁴ und R¹⁵ gemeinsam für eine zusätzliche Bindung,
R¹¹, R^{11'} und R¹⁹ je für ein Wasserstoffatom oder R¹¹ für ein α-ständiges Wasserstoffatom und R^{11'} und R¹⁹ gemeinsam für eine zusätzliche Bindung oder R¹⁹ für ein Wasserstoffatom und R¹¹ und R^{11'} gemeinsam für eine zusätzliche Bindung stehen,
R^{17β}/R^{17α} -OR²¹ / -(CH₂)ₙ-A
-OR²¹ / -(CH₂)ₘ-C≡C-B
-OR²¹ / -(CH₂)ₚ-CH=CH-(CH₂)ₖ-D
-OR²¹ / -HC=C=CEG
-OR²¹/ -CF₃ 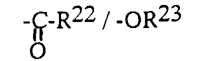 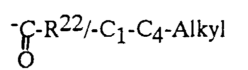 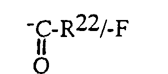 oder R^{17β}/R^{17α} gemeinsam 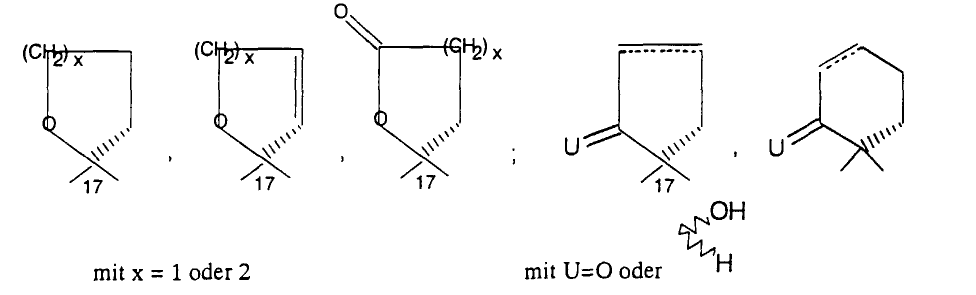 bedeuten, mit
R²¹ und R²³ in der Bedeutung eines Wasserstoffatoms, einer C₁-C₄-Alkyl- oder einer C₁-C₄-Alkanoylgruppe,
R²² in der Bedeutung einer C₁-C₃-Alkylgruppe,
A in der Bedeutung eines Wasserstoffatoms, der Cyanogruppe, von -COOR²⁴ oder -OR²⁵ wobei R²⁴ für C₁-C₄-Alkyl und R²⁵ für Wasserstoff, C₁-C₄-Alkyl- oder C₁-C₄-Alkanoyl stehen,
B in der Bedeutung eines Wasserstoffatoms, einer C₁-C₄-Alkylgruppe, einer C₂- oder C₃-Alkinylgruppe, eines Fluor-, Chlor-, Brom- oder Jodatoms, einer Hydroxyalkyl-, Alkoxyalkyl- oder Alkanoyloxyalkylgruppe mit jeweils 1 bis 4 Kohlenstoffatomen im Alkyl-, Alkoxy- oder Alkanoyloxyteil,
D in der Bedeutung eines Wasserstoffatoms, einer Hydroxy-, C₁-C₄-Alkoxy- oder C₁-C₄-Alkanoyloxygruppe,
E und G in der Bedeutung von Wasserstoff oder C₁-C₃-Alkyl,
n in der Bedeutung 0, 1, 2, 3 oder 4,
m in der Bedeutung 0, 1 oder 2,
p in der Bedeutung 0 oder 1,
k in der Bedeutung 0, 1, 2 oder 3 und
R¹⁸ für ein Wasserstoffatom oder eine Methylgruppe
steht.

2. 19,11-überbrückte 4-Estrene nach Anspruch 1, dadurch gekennzeichnet, daß W für ein Sauerstoffatom oder zwei Wasserstoffatome steht.

3. 19,11-überbrückte 4-Estrene nach Anspruch 1, dadurch gekennzeichnet, daß R^{6a} und R^{6b} je für ein Wasserstoffatom oder gemeinsam mit dem Kohlenstoffatom 6 für einen Dreiring stehen.

4. 19,11-überbrückte 4-Estrene nach Anspruch 1, dadurch gekennzeichnet, daß R^{6a} für ein Chlor- oder Bromatom oder für einen gerakettigen, gesättigten α- oder β-ständigen C₁-C₄-Alkylrest steht.

5. 19,11-überbrückte 4-Estrene nach Anspruch 1, dadurch gekennzeichnet, daß R^{6b} und R⁷ gemeinsam für eine α-oder β-ständige Methylenbrücke oder eine zusätzliche Doppelbindung stehen.

6. 19,11-überbrückte 4-Estrene nach Anspruch 1, dadurch gekennzeichnet, daß R⁷ für einen gerad- oder verzweigtkettigen gesättigten α-oder β-ständigen Alkylrest mit bis zu 4 Kohlenstoffatomen steht.

7. 19,11-überbrückte 4-Estrene nach Anspruch 1, dadurch gekennzeichnet, daß R¹⁴, R¹⁵ und R¹⁶ je für ein Wasserstoffatom stehen.

8. 19,11-überbrückte 4-Estrene nach Anspruch 1, dadurch gekennzeichnet, daß R¹⁴ für ein α-ständiges Wasserstoffatom sowie R¹⁵ und R¹⁶ gemeinsam für eine zusätzliche Bindung oder für eine β-ständige Methylenbrücke stehen.

9. 19,11-überbrückte 4-Estrene nach Anspruch 1, dadurch gekennzeichnet, daß
R^{17β}/R^{17α} -OH / -CH₃,
-OC(O)CH₃ / -CH₃;
-OH / -C≡CH,
-OC(O)CH₃ / -C≡CH;
-OH / -C≡C-CH_{3,}
-OC(O)CH₃ / -C≡C-CH₃;
-C(O)CH₃ / -OC(O)CH₃ 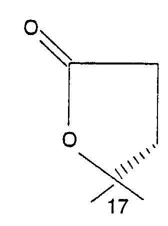 bedeuten.

10. Verbindungen nach Anspruch 1, nämlich
17-(Acetyloxy)-9,11α-dihydro-6'H-benzo[10,9,11]-19-norpregn-4-en-3,20-dion;
17-(Acetyloxy)-9,11α-dihydro-6'H-benzo[10,9,11]-19-norpregna-4,6-dien-3,20-dion;
17-(Acetyloxy)-9,11α-dihydro-6-methyl-6'H-benzo[10,9,11]-19-norpregna-4,6-dien-3,20-dion;
17-(Acetyloxy)-9,11α-dihydro-6α-methyl-6'H-benzo[10,9,11]-19-norpregn-4-en-3,20-dion;
17-(Acetyloxy)-6-chlor-9,11α-dihydro-6'H-benzo[10,9,11]-19-norpregna-4,6-dien-3,20-dion;
17-(Acetyloxy)-6-chlor-1β,2β,9,11α-tetrahydro-3'H-cyclopropa[1,2][6"H]benzo-[10,9,11]-19-norpregna-4,6-dien-3,20-dion;
17-(Acetyloxy)-4',5',9,11α-tetrahydro-6'H-benzo[10,9,11]-19-norpregn-4-en-3,20-dion;
17-(Acetyloxy)-6-methyl-4',5',9,11α-tetrahydro-6'H-benzo[10,9,11]-19-norpregna-4,6-dien-3,20-dion;
17-(Acetyloxy)-6-chlor-1β,2β,4",5",9,11α-hexahydro-3'H-cyclopropa[1,2][6"H]benzo-[10,9,11]-19-norpregna-4,6-dien-3,20-dion;
17-(Acetyloxy)-5',6'-dihydro-9H-benzo[10,9,11]- 19-norpregn-4-en-3,20-dion;
17-(Acetyloxy)-5',6'-dihydro-6-methyl-9H-benzo[10,9,11]-19-norpregna-4,6-dien-3,20-dion;
17-(Acetyloxy)-9,11α-dihydro-6'H-benzo[10,9,11]-18a-homo-19-norpregn-4-en-3,20-dion;
17-(Acetyloxy)-9,11α-dihydro-6-methyl-6'H-benzo[10,9,11]-18a-homo-19-norpregna-4,6-dien-3,20-dion;
9,11α-Dihydro-17-methyl-6'H-benzo[10,9,11]-19-norpregn-4-en-3,20-dion;
3',9,11α,16β-Tetrahydrocyclopropa[16,17][6H]benzo[10,9,11]-19-norpregn-4-en-3,20-dion;
9,11α-Dihydro- 17-methyl-6'H-benzo[10,9,11]-18a-homo-19-norpregn-4-en-3,20-dion;
3',9,11α,16β-Tetrahydrocyclopropa[16,17][6H]benzo[10,9,11]-18a-homo-19-norpregn-4-en-3,20-dion;
9,11α-Dihydro-17β-hydroxy-17α-methyl-6'H-benzo[10,9,11]estr-4-en-3-on;
9,11α-Dihydro-17α-ethinyl-17β-hydroxy-6'H-benzo[10,9,11]estr-4-en-3-on;
9,11α-Dihydro-17β-hydroxy-17α-(1-propinyl)-6'H-benzo[10,9,11]estr-4-en-3-on;
9,11α-Dihydro-17β-hydroxy-17α-(1,3-pentadiinyl)-6'H-benzo[10,9,11]estr-4-en-3-on;
(Z)-9,11α-Dihydro-17β-hydroxy-17α-(3-hydroxy-1-propenyl)-6'H-benzo[10,9,11]estr-4-en-3-on;
9,11α-Dihydro-17β-hydroxy-17α-(3-hydroxypropyl)-6'H-benzo[10,9,11]estr-4-en-3-on;
17β-Hydroxy-17α-methyl-4',5',9,11α-tetrahydro-6'H-benzo[10,9,11]estr-4-en-3-on;
17α-Ethinyl-17β-hydroxy-4',5',9,11α-tetrahydro-6'H-benzo[10,9,11]estr-4-en-3-on;
17β-Hydroxy-17α-(1-propinyl)-4',5',9,11α-tetrahydro-6'H-benzo[10,9,11]estr-4-en-3-on
5',6'-Dihydro-17α-ethinyl-17β-hydroxy-9H-benzo[10,9,11]estr-4-en-3-on;
5',6'-Dihydro-17β-hydroxy- 17α-(1-propinyl)-9H-benzo[10,9,11]estr-4-en-3-on;
9,11α-Dihydro-17α-ethinyl-17β-hydroxy-6'H-benzo[10,9,11]-18a-homoestr-4-en-3-on;
9,11α-Dihydro-17β-hydroxy-17α-(1-propinyl)-6'H-benzo[10,9,11]-18a-homoestr-4-en-3-on;
17α-(1-Butinyl)-9,11α-dihydro-17β-hydroxy-6'H-benzo[10,9,11]-18a-homoestr-4-en-3-on;
9,11α-Dihydro- 17α-(1,2-propadienyl)-17β-hydroxy-6'H-benzo[10,9,11]-18a-homoestr-4-en-3-on;
17α-Ethinyl-17β-hydroxy-4',5',9,11α-tetrahydro-6'H-benzo[10,9,11]18a-homoestr-4-en-3-on;
17β-Hydroxy-17α-(1-propinyl)-4',5',9,11α-tetrahydro-6'H-benzo[10,9,11]-18a-homoestr-4-en-3-on;
5',6'-Dihydro-17α-ethinyl-17β-hydroxy-9H-benzo[10,9,11]-18a-homoestr-4-en-3-on;
5',6'-Dihydro-17β-hydroxy-17α-(1-propinyl)-9H-benzo[10,9,11]-18a-homoestr-4-en-3-on;
9,11α-Dihydro-17α-ethinyl-17β-hydroxy-6'H-benzo[10,9,11]estra-4,15-dien-3-on;
9,11α-Dihydro-17β-hydroxy-17α-(1-propinyl)-6'H-benzo[10,9,11]estra-4,15-dien-3-on;
17α-Ethinyl-17β-hydroxy-4',5',9,11α-tetrahydro-6'H-benzo[10,9,11]estra-4,15-dien-3-on;
5',6'-Dihydro-17α-ethinyl-17β-hydroxy-9H-benzo[10,9,11]estra-4,15-dien-3-on;
5',6'-Dihydro-17β-hydroxy- 17α-(1-propinyl)-9H-benzo[10,9,11]estra-4,15-dien-3-on;
9,11α-Dihydro-17α-ethinyl-17β-hydroxy-6'H-benzo[10,9,11]-18a-homoestra-4,15-dien-3-on;
9,11α-Dihydro-17β-hydroxy-17α-(1-propinyl)-6'H-benzo[10,9,11]-18a-homoestra-4,15-dien-3-on;
17α-Ethinyl-17β-hydroxy-4',5',9,11α-tetrahydro-6'H-benzo[10,9,11]-18a-homoestra-4,15-dien-3-on;
5',6'-Dihydro-17α-ethinyl-17β-hydroxy-9H-benzo[10,9,11]-18a-homoestra-4,15-dien-3-on;
5',6'-Dihydro-17β-hydroxy-17α-(1-propinyl)-9H-benzo[10,9,11]-18a-homoestra-4,15-dien-3-on;
4",5",9,11α-Tetrahydrospiro[6'H-benzo[10,9,11]estr-4-en-17β,2"(3"H)-furan]-3-on;
3",4",9,11α-Tetrahydrospiro[6'H-benzo[10,9,11]estr-4-en-17β,2"(5"H)-furan]-3,5"-dion;
3"",4"",6α,7α,9,11α,15α,16α-Octahydrospiro[3'H,3"H-dicyclopropa[6,7;15,16]-[6H]benzo[10,9,11]estr-4-en-17β,2""(5""H)-furan]-3,5""-dion;
3"",4"",9',11'α,15'α,16'α-Hexahydrospiro[cyclopropan-1,6'-[3H]cyclopropa[15,16][6H]benzo[10,9,11]estr-4-en-17'β,2""(5""H)-furan]-3',5""-dion;
3"",4"",9',11'α,15'α,16'α-Hexahydrospiro[cyclopropan-1,6'-[3H]cyclopropa[15,16]-[6H]benzo[10,9,11]estra-1,4-dien-17'β,2""(5""H)-furan]-3',5""-dion;
3"",4"',4"",5"',6α,7α,9,11α,15α,16α-Decahydrospiro[3'H,3"H-dicyclopropa-[6,7:15,16][6H]benzo[10,9,11]estr-4-en-17β,2""(5""H)-furan]-3,5""-dion;
3",4",9,11α-Tetrahydrospiro[6'H-benzo[10,9,11]-18a-homoestr-4-en-17β,2"(5")-furan]-3,5"-dion;
3"",4"",6α,7α,9,11α,15α,16α-Octahydrospiro[3'H,3"H-dicyclopropa[6,7:15,16][6H]-benzo[10,9,11]-18a-homoestr-4-en-17β,2""(5""H)-furan]-3,5""-dion;
9,11α-Dihydro-17α-ethinyl-6'H-benzo[10,9,11]estr-4-en-17β-ol;
17α-Ethinyl-4',5',9,11α-tetrahydro-6'H-benzo[10,9,11]estr-4-en-17β-ol;
5',6'-Dihydro-17α-ethinyl-9H-benzo[10,9,11]estr-4-en-17β-ol;
9,11α-Dihydro-17α-ethinyl-6'H-benzo[10,9,11]estra-4,15-dien-17β-ol;
9,11α-Dihydro-17α-ethinyl-6'H-benzo[10,9,11]-18a-homoestr-4-en-17β-ol;
9,11α-Dihydro-17α-ethinyl-6'H-benzo[10,9,11]-18a-homoestra-4,15-dien-17β-ol.

11. Verfahren zur Herstellung der Verbindungen der allgemeinen Formel I gemäß Anspruch 1, dadurch gekennzeichnet, daß eine Verbindung der allgemeinen Formel II 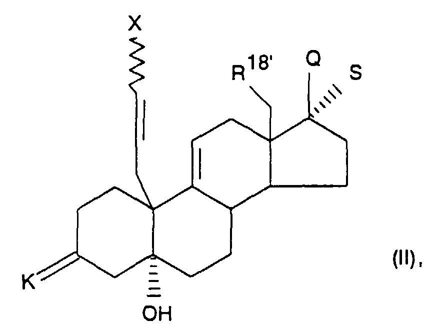 worin
K für eine Ketoschutzgruppe oder eine geschützte Hydroxygruppe und ein Wasserstoffatom,
X für ein syn- oder antiständiges Chlor- oder Bromatom,
R¹⁸ für ein Wasserstoffatom oder eine Methylgruppe sowie
Q für eine β-ständige Hydroxygruppe und S für ein α-ständiges Wasserstoffatom oder Q und S gemeinsam für ein Ketosauerstoffatom oder außerdem
Q und S für eine der in Formel I genannten R^{17β}/R^{17α} -Substituentenkombinationen einschließlich der Spiroverbindungen, wobei darin vorhandene Hydroxygruppen und/oder Ketogruppen gegebenenfalls geschützt sind,
stehen,
a) durch radikalische Cyclisierung in eine Verbindung der allgemeinen Formel III 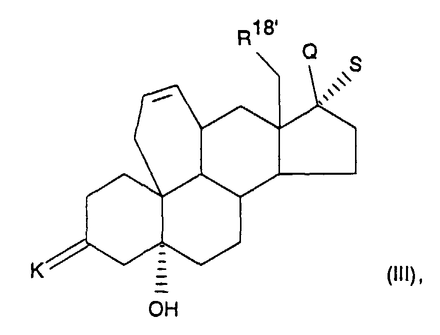 worin K, R¹⁸ sowie Q und S die in Formel II angegebene Bedeutung haben, überführt,
b) anschließend, wenn Q für eine Hydroxygruppe steht, diese gewünschtenfalls oxidiert,
c) wenn R¹¹ und R¹⁹ letztendlich Wasserstoffatome sein sollen, die 19,11β-Ethenobrücke hydriert,
d) wenn R¹⁹ letzendlich ein Wasserstoffatom sowie R¹¹ und R^{11'} gemeinsam für eine zusätzliche Bindung stehen sollen, die Doppelbindung in der 19,11β-Ethenobrücke in die 11-Position (exoständig) isomerisiert,
e) gewünschtenfalls in den D-Ring eine 15,16-Doppelbindung eingeführt und diese
f) gewünschtenfalls in die 14,15-Position isomerisiert oder
g) durch Methylenierung in die entsprechende 15β,16β-Methylenverbindung sowie
h) wenn Q und S gemeinsam ein Ketosauerstoffatom bedeuten, durch nucleophile Addition des Substituenten R^{17α} oder eines reaktiven Vorläufers von R^{17α} und gegebenenfalls Veretherung oder Veresterung der 17β-Hydroxygruppe mit einem den entsprechenden Rest R²¹ liefernden Reagenz oder das 17α-Hydroxy-17β-alkanoylsubstitutionsmuster aufgebaut und die 17α-Hydroxygruppe gegebenenfalls mit einem den entsprechenden Rest R²³ liefernden Reagenz verethert oder verestert,
i) gegebenenfalls durch teilweises oder vollständiges Hydrieren einer ungesättigten C₁₇-Seitenkette und
j) gewünschtenfalls durch Oxidation der entsprechenden 17-(3-Hydroxypropyl)- bzw. 17-(4-Hydroxybutyl)-Verbindung zur Bildung des 17-Spirolaktons oder
k) gewünschtenfalls durch Ringschlußreaktion der entsprechenden (Z)-17α-(3-Hydroxyprop-1-enyl)- bzw. (Z)-17α-(4-Hydroxybut-1-enyl)-17β-hydroxyverbindung oder der entsprechenden in der Seitenkette gesättigten Verbindungen zur Bildung des Spiroethers und
l) durch Säurebehandlung in einem mit Wasser mischbaren Lösungsmittel in das Δ⁴-3-Keto-System, wobei weitere vorhandene Schutzgruppen ebenfalls abgespalten werden, und somit in eine Verbindung der allgemeinen Formel I überführt, und diese Verbindung der allgemeinen Formel I gegebenenfalls
m) durch Einführung einer 1,2- und/oder 6,7-Doppelbindung und gegebenenfalls Methylenierung einer oder beider Doppelbindung(en),
n) durch Einführung eines gerad- oder verzweigtkettigen α- oder β-ständigen Alkylrestes oder einer Thiogruppe -SR²⁰ in 7-Position,
o) durch Epoxidierung der 6,7-Doppelbindung und Öffnung des Epoxides mit Halogenwasserstoff (Hal = F, Cl, Br, I) und Eliminierung der gebildeten 7α-Hydroxygruppe,
p) durch 6α-Hydroxymethylierung und anschließende Wasserabspaltung zur 6-Methylenverbindung,
q) durch Isomerisierung der exo-ständigen Doppelbindung der 6-Methylengruppe oder durch direkte Einführung einer 6-Alkylgruppe (6-Alkyl-4,6-dien-3-on),
r) durch Hydrierung der 6-Methylengruppe
in eine Verbindung der allgemeinen Formel I
worin
R¹, R², R¹⁴, R¹⁵, R¹⁶, R^{17α}, R^{17β}, R¹⁸, R¹¹ und R¹⁹ die letztendlich gewünschte Bedeutung haben,
und R^{6a} eine α-Methylgruppe und R^{6b} und R⁷ je ein Wasserstoffatom oder gemeinsam eine zusätzliche Bindung darstellen,
umgewandelt,
oder,
s) wenn R^{6a} letztendlich ein gerad- oder verzweigtkettiger gesättigter α- oder β-ständiger Alkylrest mit bis zu 4 Kohlenstoffatomen sein soll,
durch Ketalisierung unter gleichzeitiger Isomerisierung der 4(5)-Doppelbindung nach 5(6), Epoxidierung der 5(6)-Doppelbindung und nucleophile Öffnung des 5,6α-Epoxids mit geschützter 3-Ketogruppe mit einem/einer gerad- oder verzweigtkettigen gesättigten Alkylmagnesiumhalogenid oder Alkyllithiumverbindung mit bis zu 4 Kohlenstoffatomen im Alkylrest und Spaltung der 3-Ketoschutzgruppe in der gebildeten 5α-Hydroxy-6β-Alkylverbindung unter milden sauren Bedingungen zur entsprechenden 3-Keto-5α-hydroxy-6β-alkyl-Verbindung und basische Eliminierung der 5α-Hydroxygruppe in die entsprechende 3-Keto-4-en-Verbindung der allgemeinen Formel I mit β-ständiger 6-Alkylgruppe oder durch Abspaltung der 3-Ketoschutzgruppe unter drastischeren Bedingungen in die entsprechende 3-Keto-4-en-Verbindung der allgemeinen Formel I mit α-ständiger 6-Alkylgruppe überführt und
t) gegebenenfalls eine der vorstehend erhaltenen 3-Keto-Verbindungen mit Hydroxylaminhydrochlorid in Gegenwart von tertiären Aminen bei einer Temperatur zwischen -20° und +40°C in die 3-Hydroxyiminoverbindung (W = 〉N~OH;~ bedeutet syn- oder antiständiges OH) oder
u) gegebenenfalls in das 3-Thioketal, vorzugsweise das 3-(1',3'-Ethylendithio)-Ketal, umgewandelt und dieses reduktiv zur Verbindung der allgemeinen Formel I, worin W für zwei Wasserstoffatome steht,
gespalten wird.

12. Pharmazeutische Präparate, dadurch gekennzeichnet, daß sie mindestens eine Verbindung der allgemeinen Formel I gemäß Anspruch 1 sowie einen pharmazeutisch verträglichen Träger enthalten.

13. Verwendung der Verbindungen der allgemeinen Formel I gemäß Anspruch 1 zur Herstellung von Arzneimitteln.

14. Zwischenverbindungen der allgemeinen Formel III' 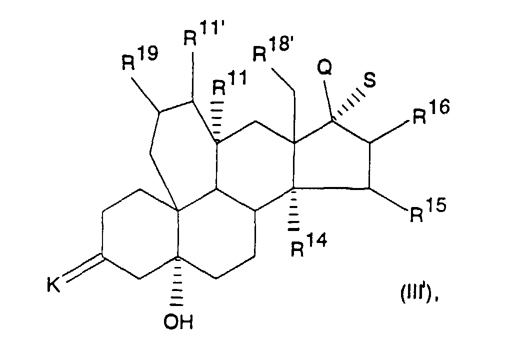 worin
K für eine Ketoschutzgruppe oder eine geschützte Hydroxygruppe und ein Wasserstoffatom,
R^{11'} und R¹⁹ gemeinsam für eine zusätzliche Bindung und R¹¹ für ein α-ständiges Wasserstoffatom oder R¹⁹ für ein Wasserstoffatom und R^{11'} und R¹¹ gemeinsam für eine zusätzliche Bindung oder R¹¹, R^{11'} und R¹⁹ je für ein Wasserstoffatom,
R¹⁴, R¹⁵ und R¹⁶ für die hierfür in Formel I angegebenen Substituenten,
R¹⁸ für ein Wasserstoffatom oder eine Methylgruppe sowie
Q für eine β-ständige Hydroxygruppe und S für ein α-ständiges Wasserstofftom oder Q und S gemeinsam für ein Ketosauerstoffatom oder außerdem
Q und S für eine der in Formel I genannten R^{17β}/R^{17α}-Substituentenkombinationen einschließlich der Spiroverbindungen, wobei darin vorhandene Hydroxy- und /oder Ketogruppen gegebenenfalls geschützt sind,
stehen.

## Claims

1. 19,11-bridged 4-oestrenes of the general formula I 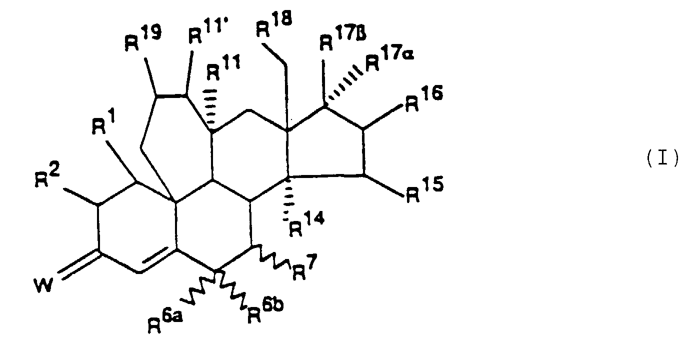 in which
W represents an oxygen atom, the hydroxyimino group >N~OH or two hydrogen atoms;
R¹ and R² each represents a hydrogen atom or together represent an additional bond or a methylene bridge having the α-configuration;
R^{6a} and R^{6b} each represents a hydrogen atom or together represent a methylene group or, together with the 6 carbon atom, a three-membered ring, in which cases R⁷ represents a hydrogen atom; or
R^{6a} represents a hydrogen atom or a fluorine, chlorine, bromine or iodine atom or a straight- or branched-chained saturated alkyl radical that contains up to 4 carbon atoms and has the α- or β-configuration, in which cases R^{6b} and R⁷ each represents a hydrogen atom or together represent an additional bond; or
R^{6b} and R⁷ together represent a methylene bridge having the α- or β-configuration, in which case R^{6a} is a hydrogen atom;
R⁷ represents a straight- or branched-chained saturated alkyl radical that contains up to 4 carbon atoms and has the α- or β-configuration, or represents a thio group -SR²⁰, wherein R²⁰ represents a hydrogen atom or an alkanoyl group having from 1 to 4 carbon atoms, in which cases R^{6a} and R^{6b} each represents a hydrogen atom;
R¹⁴, R¹⁵ and R¹⁶ each represents a hydrogen atom; or R¹⁴ represents a hydrogen atom having the α-configuration and R¹⁵ and R¹⁶ together represent an additional bond or a methylene bridge having the α- or β-configuration, or R¹⁴ and R¹⁵ each represents a hydrogen atom and R¹⁶ represents a C₁-C₄alkyl group having the α- or β-configuration, or R¹⁶ together with R^{17α} represents a methylene bridge having the α-configuration and R^{17β} represents a group 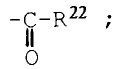 or
R¹⁶ represents a hydrogen atom and R¹⁴ and R¹⁵ together represent an additional bond;
R¹¹, R^{11'} and R¹⁹ each represents a hydrogen atom, or R¹¹ represents a hydrogen atom having the α-configuration and
R^{11'} and R¹⁹ together represent an additional bond, or R¹⁹ represents a hydrogen atom and R¹¹ and R^{11'} together represent an additional bond;
R^{17β}/R^{17α} represents -OR²¹/- (CH₂)ₙ-A,
-OR²¹/-(CH₂)ₘ-C≡C-B,
-OR²¹/-(CH₂)ₚ-CH=CH- (CH₂)ₖ-D,
-OR²¹/-HC=C=CEG,
-OR²¹/-CF₃, 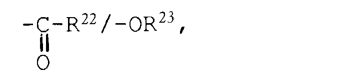 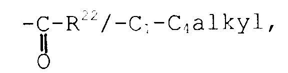 or 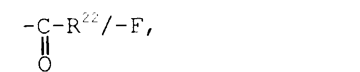
or R^{17β}/R^{17α} together represent 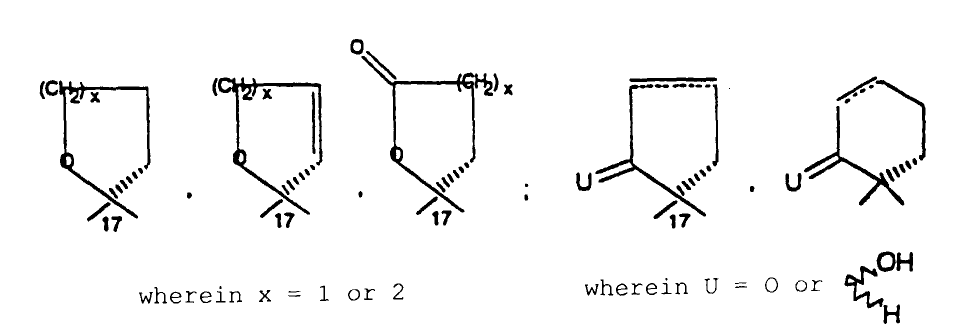 wherein
R²¹ and R²³ represent a hydrogen atom, a C₁-C₄alkyl group or a C₁-C₄alkanoyl group,
R²² represents a C₁-C₃alkyl group,
A represents a hydrogen atom, a cyano group, -COOR²⁴ or -OR²⁵, wherein R²⁴ represents C₁-C₄alkyl and R²⁵ represents hydrogen, C₁-C₄alkyl or C₁-C₄alkanoyl,
B represents a hydrogen atom, a C₁-C₄alkyl group, a C₂- or C₃-alkynyl group, a fluorine, chlorine, bromine or iodine atom, or a hydroxyalkyl, alkoxyalkyl or alkanoyloxyalkyl group each having from 1 to 4 carbon atoms in the alkyl, alkoxy or alkanoyloxy moiety,
D represents a hydrogen atom or a hydroxy, C₁-C₄alkoxy or C₁-C₄alkanoyloxy group,
E and G represent hydrogen or C₁-C₃alkyl,
n represents 0, 1, 2, 3 or 4,
m represents 0, 1 or 2,
p represents 0 or 1, and
k represents 0, 1, 2 or 3;
and
R¹⁸ represents a hydrogen atom or a methyl group.

2. 19,11-bridged 4-oestrenes according to claim 1,
characterised in that W represents an oxygen atom or two hydrogen atoms.

3. 19,11-bridged 4-oestrenes according to claim 1,
characterised in that R^{6a} and R^{6b} each represents a hydrogen atom or together with the 6 carbon atom represent a three-membered ring.

4. 19,11-bridged 4-oestrenes according to claim 1,
characterised in that R^{6a} represents a chlorine or bromine atom or a straight-chained saturated C₁-C₄alkyl radical having the α- or β-configuration.

5. 19,11-bridged 4-oestrenes according to claim 1,
characterised in that R^{6b} and R⁷ together represent a methylene bridge having the α- or β-configuration or an additional double bond.

6. 19,11-bridged 4-oestrenes according to claim 1,
characterised in that R⁷ represents a straight- or branched-chained saturated alkyl radical that contains up to 4 carbon atoms and has the α- or β-configuration.

7. 19,11-bridged 4-oestrenes according to claim 1,
characterised in that R¹⁴, R¹⁵ and R¹⁶ each represents a hydrogen atom.

8. 19,11-bridged 4-oestrenes according to claim 1,
characterised in that R¹⁴ represents a hydrogen atom having the α-configuration and R¹⁵ and R¹⁶ together represent an additional bond or a methylene bridge having the β-configuration.

9. 19,11-bridged 4-oestrenes according to claim 1,
characterised in that R^{17β} /R^{17α} represents
-OH/-CH₃,
-OC (O) CH₃/-CH₃,
-OH/-C≡CH,
-OC(O)CH₃/-C≡CH,
-OH/-C≡C-CH₃,
-OC(O)CH₃/-C≡C-CH₃,
-C(O)CH₃/-OC(O)CH₃, or

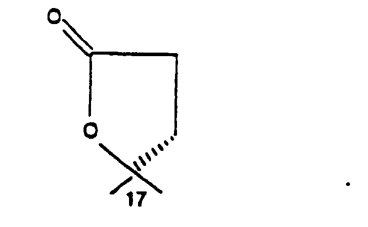

10. Compounds according to claim 1, namely
17-(acetoxy)-9,11α-dihydro-6'H-benzo[10,9,11]-19-norpregn-4-ene-3,20-dione;
17-(acetoxy)-9,11α-dihydro-6'H-benzo[10,9,11]-19-norpregna-4,6-diene-3,20-dione;
17-(acetoxy)-9,11α-dihydro-6-methyl-6'H-benzo[10,9,11]-19-norpregna-4,6-diene-3,20-dione;
17-(acetoxy)-9,11α-dihydro-6α-methyl-6'H-benzo[10,9,11]-19-norpregn-4-ene-3,20-dione;
17-(acetoxy)-6-chloro-9,11α-dihydro-6'H-benzo[10,9,11]-19-norpregna-4,6-diene-3,20-dione;
17-(acetoxy)-6-chloro-1β,2β,9,11α-tetrahydro-3'H-cyclopropa[1,2][6"H]benzo[10,9,11]-19-norpregna-4,6-diene-3,20-dione;
17-(acetoxy)-4',5',9,11α-tetrahydro-6'H-benzo[10,9,11]-19-norpregn-4-ene-3,20-dione;
17-(acetoxy)-6-methyl-4',5',9,11α-tetrahydro-6'H-benzo-[10,9,11]-19-norpregna-4,6-diene-3,20-dione;
17-(acetoxy)-6-chloro-1β,2β,4",5",9,11α-hexahydro-3'H-cyclopropa[1,2][6"H]benzo[10,9,11]-19-norpregna-4,6-diene-3,20-dione;
17-(acetoxy)-5',6'-dihydro-9H-benzo[10,9,11]-19-norpregn-4-ene-3,20-dione;
17-(acetoxy)-5',6'-dihydro-6-methyl-9H-benzo[10,9,11]-19-norpregna-4,6-diene-3,20-dione;
17-(acetoxy)-9,11α-dihydro-6'H-benzo[10,9,11]-18a-homo-19-norpregn-4-ene-3,20-dione;
17-(acetoxy)-9,11α-dihydro-6-methyl-6'H-benzo[10,9,11]-18a-homo-19-norpregna-4,6-diene-3,20-dione;
9,11α-dihydro-17-methyl-6'H-benzo[10,9,11]-19-norpregn-4-ene-3,20-dione;
3',9,11α,16β-tetrahydrocyclopropa[16,17][6H]benzo-[10,9,11]-19-norpregn-4-ene-3,20-dione;
9,11α-dihydro-17-methyl-6'H-benzo[10,9,11]-18a-homo-19-norpregn-4-ene-3,20-dione;
3',9,11α,16β-tetrahydrocyclopropa[16,17][6H]benzo-[10,9,11]-18a-homo-19-norpregn-4-ene-3,20-dione;
9,11α-dihydro-17β-hydroxy-17α-methyl-6'H-benzo[10,9,11]-oestr-4-en-3-one;
9,11α-dihydro-17α-ethynyl-17β-hydroxy-6'H-benzo[10,9,11]-oestr-4-en-3-one;
9,11α-dihydro-17β-hydroxy-17α-(1-propynyl)-6'H-benzo-[10,9,11]oestr-4-en-3-one;
9,11α-dihydro-17β-hydroxy-17α-(1,3-pentadiynyl)-6'H-benzo[10,9,11]oestr-4-en-3-one;
(Z)-9,11α-dihydro-17β-hydroxy-17α-(3-hydroxy-1-propenyl)-6'H-benzo[10,9,11]oestr-4-en-3-one;
9,11α-dihydro-17β-hydroxy-17α-(3-hydroxypropyl)-6'H-benzo[10,9,11]oestr-4-en-3-one;
17β-hydroxy-17α-methyl-4',5',9,11α-tetrahydro-6'H-benzo-[10,9,11]oestr-4-en-3-one;
17α-ethynyl-17β-hydroxy-4',5',9,11α-tetrahydro-6'H-benzo-[10,9,11]oestr-4-en-3-one;
17β-hydroxy-17α-(1-propynyl)-4',5',9,11α-tetrahydro-6'H-benzo[10,9,11]oestr-4-en-3-one;
5',6'-dihydro-17α-ethynyl-17β-hydroxy-9H-benzo[10,9,11]-oestr-4-en-3-one;
5',6'-dihydro-17β-hydroxy-17α-(1-propynyl)-9H-benzo-[10,9,11]oestr-4-en-3-one;
9,11α-dihydro-17α-ethynyl-17β-hydroxy-6'H-benzo[10,9,11]-18a-homooestr-4-en-3-one;
9,11α-dihydro-17β-hydroxy-17α-(1-propynyl)-6'H-benzo-[10,9,11]-18a-homooestr-4-en-3-one;
17α-(1-butynyl)-9,11α-dihydro-17β-hydroxy-6'H-benzo-[10,9,11]-18a-homooestr-4-en-3-one;
9,11α-dihydro-17α-(1,2-propadienyl)-17β-hydroxy-6'H-benzo[10,9,11]-18a-homooestr-4-en-3-one;
17α-ethynyl-17β-hydroxy-4',5',9,11α-tetrahydro-6'H-benzo-[10,9,11]-18a-homooestr-4-en-3-one;
17β-hydroxy-17α-(1-propynyl)-4',5',9,11α-tetrahydro-6'H-benzo[10,9,11]-18a-homooestr-4-en-3-one;
5',6'-dihydro-17α-ethynyl-17β-hydroxy-9H-benzo[10,9,11]-18a-homooestr-4-en-3-one;
5',6'-dihydro-17β-hydroxy-17α-(1-propynyl)-9H-benzo-[10,9,11]-18a-homooestr-4-en-3-one;
9,11α-dihydro-17α-ethynyl-17β-hydroxy-6'H-benzo-[10,9,11]oestra-4,15-dien-3-one;
9,11α-dihydro-17β-hydroxy-17α-(1-propynyl)-6'H-benzo-[10,9,11]oestra-4,15-dien-3-one;
17α-ethynyl-17β-hydroxy-4',5',9,11α-tetrahydro-6'H-benzo-[10,9,11]oestra-4,15-dien-3-one;
5',6'-dihydro-17α-ethynyl-17β-hydroxy-9H-benzo[10,9,11]-oestra-4,15-dien-3-one;
5',6'-dihydro-17β-hydroxy-17α-(1-propynyl)-9H-benzo-[10,9,11]oestra-4,15-dien-3-one;
9,11α-dihydro-17α-ethynyl-17β-hydroxy-6'H-benzo[10,9,11]-18a-homooestra-4,15-dien-3-one;
9,11α-dihydro-17β-hydroxy-17α-(1-propynyl)-6'H-benzo-[10,9,11]-18a-homooestra-4,15-dien-3-one;
17α-ethynyl-17β-hydroxy-4',5',9,11α-tetrahydro-6'H-benzo-[10,9,11]-18a-homooestra-4,15-dien-3-one;
5',6'-dihydro-17α-ethynyl-17β-hydroxy-9H-benzo[10,9,11]-18a-homooestra-4,15-dien-3-one;
5',6'-dihydro-17β-hydroxy-17α-(1-propynyl)-9H-benzo-[10,9,11]-18a-homooestra-4,15-dien-3-one;
4",5",9,11α-tetrahydrospiro[6'H-benzo[10,9,11]oestr-4-ene-17β,2"(3"H)-furan]-3-one;
3",4",9,11α-tetrahydrospiro[6'H-benzo[10,9,11]oestr-4-ene-17β,2"(5"H)-furan]-3,5"-dione;
3"",4"",6α,7α,9,11α,15α,16α-octahydrospiro[3'H,3"H-dicyclopropa[6,7:15,16][6H]benzo[10,9,11]oestr-4-ene-17β,2""(5""H)-furan]-3,5""-dione;
3"",4"",9',11'α,15'α,16'α-hexahydrospiro[cyclopropan-1,6'-[3H]cyclopropa[15,16][6H]benzo[10,9,11]oestr-4-ene-17'β,2""(5""H)-furan]-3',5""-dione;
3"",4"",9',11'α,15'α,16'α-hexahydrospiro[cyclopropan-1,6'-[3H]cyclopropa[15,16][6H]benzo[10,9,11]oestra-1,4-diene-17'β,2"" (5""H)-furan]-3',5""-dione;
3"", 4"',4"", 5"', 6α,7α,9,11α,15α,16α-decahydrospiro-[3'H,3"H-dicyclopropa[6,7:15,16] [6H]benzo[10,9,11]oestr-4-ene-17β,2""(5""H)-furan]-3,5""-dione;
3",4",9,11α-tetrahydrospiro[6'H-benzo[10,9,11]-18a-homooestr-4-ene-17β,2"(5")-furan]-3,5"-dione;
3"",4"",6α,7α,9,11α,15α,16α-octahydrospiro[3'H,3"H-dicyclopropa[6,7:15,16][6H]benzo[10,9,11]-18a-homooestr-4-ene-17β,2""(5""H)-furan]-3,5""-dione;
9,11α-dihydro-17α-ethynyl-6'H-benzo[10,9,11]oestr-4-en-17β-ol;
17α-ethynyl-4',5',9,11α-tetrahydro-6'H-benzo[10,9,11]-oestr-4-en-17β-ol;
5',6'-dihydro-17α-ethynyl-9H-benzo[10,9,11]oestr-4-en-17β-ol;
9,11α-dihydro-17α-ethynyl-6'H-benzo[10,9,11]oestra-4,15-dien-17β-ol;
9,11α-dihydro-17α-ethynyl-6'H-benzo[10,9,11]-18a-homooestr-4-en-17β-ol;
9,11α-dihydro-17α-ethynyl-6'H-benzo[10,9,11]-18a-homooestra-4,15-dien-17β-ol.

11. Process for the preparation of compounds of the general formula I according to claim 1, characterised in that a compound of the general formula II 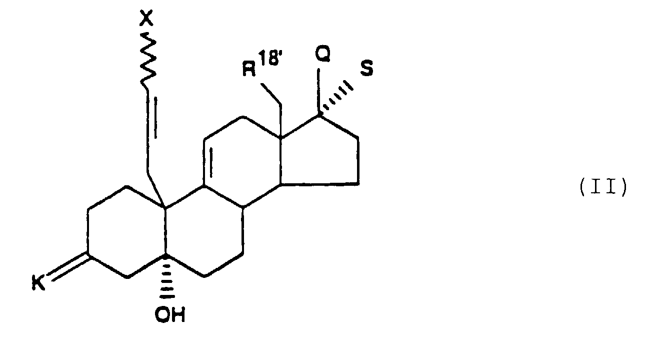 in which
K represents a keto-protecting group, or a protected hydroxy group and a hydrogen atom,
X represents a chlorine or bromine atom having the syn or anti configuration,
R¹⁸ represents a hydrogen atom or a methyl group, and
Q represents a hydroxy group having the β-configuration and S represents a hydrogen atom having the α-configuration, or Q and S together represent a keto oxygen atom, or
Q and S represent one of the R^{17β}/R^{17α} substituent combinations mentioned in formula I, including the spiro compounds, any hydroxy groups and/or keto groups present therein being protected, where appropriate,
a) is converted by means of radical cyclisation into a compound of the general formula III 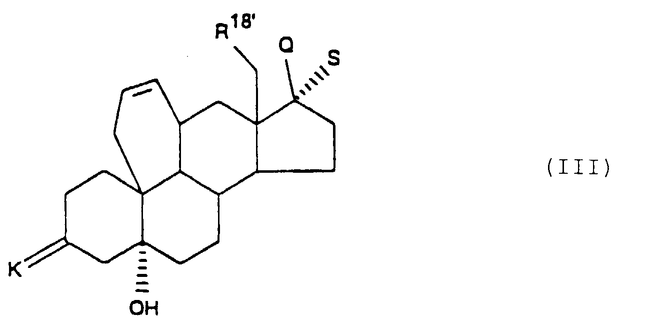 in which K, R¹⁸ and Q and S are as defined in formula II,
b) then, when Q represents a hydroxy group, that compound is, if desired, oxidised,
c) when R¹¹, R^{11'} and R¹⁹ are ultimately to be hydrogen atoms, the 19,11β-etheno bridge is hydrogenated,
d) when R¹⁹ is ultimately to be a hydrogen atom and R¹¹ and R^{11'} together are to represent an additional bond, the double bond in the 19,11β-etheno bridge is isomerised into the 11-position (exo configuration),
e) if desired, a 15,16-double bond is introduced into the D-ring and that bond
f) is, if desired, isomerised into the 14,15-position, or
g) is converted by methylenation into the corresponding 15β,16β-methylene compound and
h) when Q and S together represent a keto oxygen atom, is converted by nucleophilic addition of the substituent R^{17α} or of a reactive precursor of R^{17α} and optional etherification or esterification of the 17β-hydroxy group by a reagent supplying the corresponding radical R²¹, or the 17α-hydroxy-17β-alkanoyl substitution pattern is built up and the 17α-hydroxy group is optionally etherified or esterified by a reagent supplying the corresponding radical R²³,
i) optionally is converted by partial or complete hydrogenation of an unsaturated C₁₇ side chain and
j) if desired is converted by oxidation of the corresponding 17-(3-hydroxypropyl) or 17-(4-hydroxybutyl) compound to form the 17-spirolactone or
k) if desired is converted by ring closure reaction of the corresponding (Z)-17α-(3-hydroxyprop-1-enyl)- or (Z)-17α-(4-hydroxybut-1-enyl)-17β-hydroxy compound or of the corresponding compounds saturated in the side chain to form the spiro ether and
l) by acid treatment in a water-miscible solvent is converted into the Δ⁴-3-keto system, further protecting groups that are present likewise being removed, and hence is converted into a compound of the general formula I, and that compound of the general formula I is optionally converted
m) by introduction of a 1,2- and/or 6,7-double bond and optional methylenation of one or both double bond(s),
n) by introduction of a straight- or branched-chained alkyl radical having the α- or β-configuration or of a thio group -SR²⁰ into the 7-position,
o) by epoxidation of the 6,7-double bond and opening of the epoxide with hydrogen halide (Hal = F, Cl, Br, I) and removal of the resulting 7α-hydroxy group,
p) by 6α-hydroxymethylation and the subsequent removal of water to form the 6-methylene compound,
q) by isomerisation of the double bond of the 6-methylene group having the exo configuration or by the direct introduction of a 6-alkyl group (6-alkyl-4,6-dien-3-one),
r) by hydrogenation of the 6-methylene group into a compound of the general formula I
in which
R¹, R², R¹⁴, R¹⁵, R¹⁶, R^{17α} , R^{17β} , R¹⁸, R¹¹ and R¹⁹ have the meaning ultimately desired,
and R^{6a} represents an α-methyl group and R^{6b} and R⁷ each represents a hydrogen atom or together represent an additional bond,
or
s) when R^{6a} is ultimately to be a straight- or branched-chained saturated alkyl radical that contains up to 4 carbon atoms and has the α- or β-configuration, by ketalisation with simultaneous isomerisation of the 4(5)-double bond after 5(6) epoxidation of the 5(6)-double bond and nucleophilic opening of the 5,6α-epoxide having a protected 3-keto group with a straight- or branched-chained saturated alkylmagnesium halide or alkyllithium compound having up to 4 carbon atoms in the alkyl radical and removal of the 3-keto-protecting group in the resulting 5α-hydroxy-6β-alkyl compound under mild acidic conditions to form the corresponding 3-keto-5α-hydroxy-6β-alkyl compound and basic removal of the 5α-hydroxy group, that compound of the general formula I is converted into the corresponding 3-keto-4-ene compound of the general formula I having a 6-alkyl group in the β-configuration or, by removal of the 3-keto-protecting group under more drastic conditions, is converted into the corresponding 3-keto-4-ene compound of the general formula I in which the 6-alkyl group is in the α-configuration, and
t) optionally one of the 3-keto compounds obtained above is converted by means of hydroxylamine hydrochloride, in the presence of tertiary amines, at a temperature of from -20°C to +40°C, into the 3-hydroxyimino compound (W = >N~OH; ~ represents OH having the syn or anti configuration) or
u) optionally is converted into the 3-thioketal, preferably the 3-(1',3'-ethylenedithio)ketal, and the latter is cleaved reductively to form the compound of the general formula I in which W represents two hydrogen atoms.

12. Pharmaceutical preparations, characterised in that they comprise at least one compound of the general formula I according to claim 1 and a pharmaceutically acceptable carrier.

13. Use of the compounds of the general formula I according to claim 1 in the preparation of medicaments.

14. Intermediates of the general formula III' 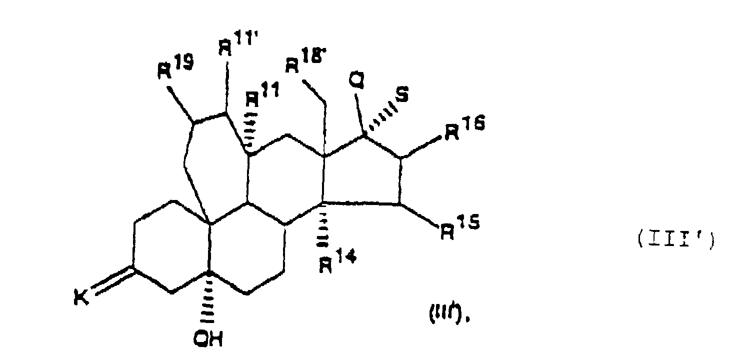 in which
K represents a keto-protecting group, or a protected hydroxy group and a hydrogen atom,
R^{11'} and R¹⁹ together represent an additional bond and R¹¹ represents a hydrogen atom having the α-configuration, or
R¹⁹ represents a hydrogen atom and R^{11'} and R¹¹ together represent an additional bond, or R¹¹, R^{11'} and R¹⁹ each represents a hydrogen atom,
R¹⁴, R¹⁵ and R¹⁶ represent the substituents mentioned therefor in formula I,
R¹⁸ represents a hydrogen atom or a methyl group, and
Q represents a hydroxy group having the β-configuration and S represents a hydrogen atom having the α-configuration, or Q and S together represent a keto oxygen atom, or
Q and S represent one of the R^{17β}/R^{17α} substituent combinations mentioned in formula I, including the spiro compounds, any hydroxy and/or keto groups present therein being protected, where appropriate.

## Revendications

1. 4-estrènes pontés en 19,11 de formule I 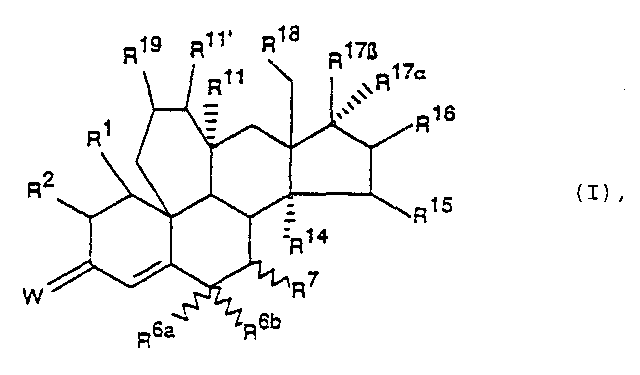 dans laquelle
W représente un atome d'oxygène, le groupe hydroxyimino >N-OH ou deux atomes d'hydrogène,
R¹ et R² représentent chacun un atome d'hydrogène ou ensemble représentent une liaison supplémentaire ou un pont méthylène en position α,
R^{6a} et R^{6b} représentent chacun un atome d'hydrogène ou ensemble représentent un groupe méthylène ou un système tricyclique formé conjointement avec l'atome de carbone 6, R⁷ dans ces cas étant un atome d'hydrogène ou
R^{6a} représente un atome d'hydrogène ou un atome de fluor, de chlore, de brome ou d'iode, ou un radical alkyle en position α ou *β,* linéaire ou ramifié, saturé, avec jusqu'à 4 atomes de carbone, R^{6b} et R⁷ étant chacun un atome d'hydrogène ou ensemble représentent une liaison supplémentaire, ou
R^{6b} et R⁷ représentent ensemble un pont méthylène en position α ou β, R^{6a} étant un atome d'hydrogène,
R⁷ représente un radical alkyle en position α ou *β,* linéaire ou ramifié, saturé, avec jusqu'à 4 atomes de carbone ou un groupe thio -SR²⁰, dans lequel R²⁰ représente un atome d'hydrogène ou un groupe alcanoyle avec 1 à 4 atomes de carbone, R^{6a} et R^{6b} représentant alors chacun un atome d'hydrogène,
R¹⁴, R¹⁵ et R¹⁶ représentent chacun un atome d'hydrogène ou
R¹⁴ représente un atome d'hydrogène en position α ainsi que R¹⁵ et R¹⁶ représentent ensemble une liaison supplémentaire ou un pont méthylène en position α ou β, ou R¹⁴ et R¹⁵ représentent chacun un atome d'hydrogène ainsi que R¹⁶ représente un groupe alkyle en C₁-C₄ en position α ou β ou R¹⁶, conjointement avec R^{17α}, représente un pont méthylène en α et R^{17β} représente un groupe 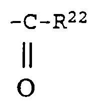
R¹⁶ représente un atome d'hydrogène ainsi que R¹⁴ et R¹⁵ ensemble forment une liaison supplémentaire,
R¹¹, R^{11'} et R¹⁹ représentent chacun un atome d'hydrogène ou R¹¹ représente un atome d'hydrogène en position αet R^{11'} et R¹⁹ représentent ensemble une liaison supplémentaire ou R¹⁹ représente un atome d'hydrogène et R¹¹ et R^{11'} ensemble forment une liaison supplémentaire,
R^{17β}/R^{17α} -OR²¹/- (CH₂)ₙ-A
-OR²¹/-(CH₂)ₘ-C≡C-B
-OR²¹/-(CH₂)ₚ-CH=CH-(CH₂)ₖ-D
-OR²¹/-HC=C=CEG
-OR²¹/ -CF₃ 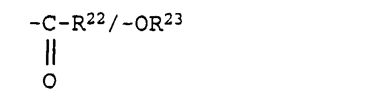 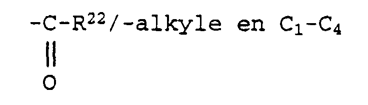 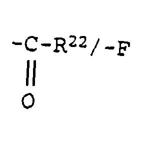
ou R^{17β}/R^{17α} ensemble représentent 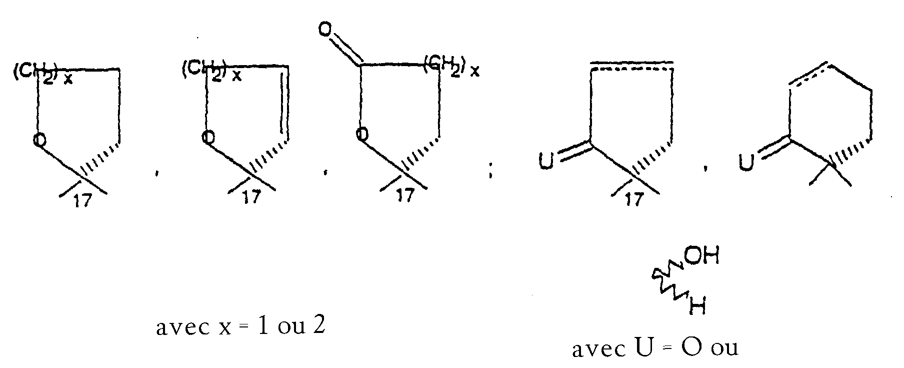 avec
R²¹ et R²³ étant un atome d'hydrogène, un groupe alkyle en C₁-C₄ ou un groupe alcanoyle en C₁-C₄,
R²² étant un groupe alkyle en C₁-C₃,
A ayant la signification d'un atome d'hydrogène, du groupe cyano, de -COOR²⁴ ou -OR²⁵, R²⁴ représentant alkyle en C₁-C₄ et R²⁵ l'hydrogène, alkyle en C₁-C₄ ou alcanoyle en C₁-C₄,
B étant un atome d'hydrogène, un groupe alkyle en C₁-C₄, un groupe alcynyle en C₂ ou en C₃, un atome de fluor, de chlore, de brome ou d'iode, un groupe hydroxyalkyle, alcoxyalkyle ou alcanoyloxyalkyle avec chacun 1 à 4 atomes de carbone dans la partie alkyle, alcoxy ou alcanoyloxy,
D ayant la signification d'un atome d'hydrogène, d'un groupe hydroxy, alcoxy en C₁-C₄ ou alcanoyloxy en C₁-C₄,
E et G ayant la signification d'hydrogène ou d'alkyle en C₁-C₃,
n valant 0, 1, 2, 3 ou 4,
m valant 0, 1 ou 2,
p valant 0 ou 1,
k valant 0, 1, 2 ou 3, et
R¹⁸ représente un atome d'hydrogène ou un groupe méthylène.

2. 4-estrènes pontés en 19,11 selon la revendication 1, caractérisés en ce que W représente un atome d'oxygène ou deux atomes d'hydrogène.

3. 4-estrènes pontés en 19,11 selon la revendication 1, caractérisés en ce que R^{6a} et R^{6b} représentent chacun un atome d'hydrogène ou un système à trois cycles formé conjointement avec l'atome de carbone 6.

4. 4-estrènes pontés en 19,11 selon la revendication 1, caractérisés en ce que R^{6a} représente un atome de chlore ou de brome ou un radical alkyle en C₁-C₄ en position α ou β, linéaire, saturé.

5. 4-estrènes pontés en 19,11 selon la revendication 1, caractérisés en ce que R^{6b} et R⁷ ensemble représentent un pont méthylène en α ou en β ou une double liaison supplémentaire.

6. 4-estrènes pontés en 19,11 selon la revendication 1, caractérisés en ce que R⁷ représente un radical alkyle en position α ou β, linéaire ou ramifié saturé, avec jusqu'à 4 atomes de carbone.

7. 4-estrènes pontés en 19,11 selon la revendication 1, caractérisés en ce que R¹⁴, R¹⁵ et R¹⁶ représentent chacun un atome d'hydrogène.

8. 4-estrènes pontés en 19,11 selon la revendication 1, caractérisés en ce que R¹⁴ représente un atome d'hydrogène en position α ainsi que R¹⁵ et R¹⁶ ensemble représentent une liaison supplémentaire ou un pont méthylène en position β.

9. 4-estrènes pontés en 19,11 selon la revendication 1, caractérisés en ce que R^{17β}/R^{16α}
-OH/-CH₃,
-OC (O) CH₃/-CH₃,
-OH/-C≡CH,
-OC (O) CH₃/-C≡CH,
-OH/-C≡CH-CH₃,
-OC (O)CH₃/-C≡C-CH₃,
-OC(O)CH₃/-OC(O)CH₃

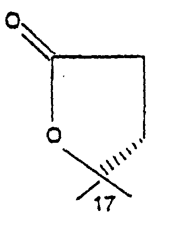

10. Composé selon la revendication 1, notamment
- 17-(Acétyloxy)-9,11α-dihydro-6'H-benzo[10,9,11]-19-norpregn-4-ène-3,20-dione ;
- 17-(Acétyloxy)-9,11α-dihydro-6'H-benzo[10,9,11]-19-norpregna-4,6-diène-3,20-dione ;
- 17-(Acétyloxy)-9,11α-dihydro-6-méthyl-6'H-benzo-[10,9,11]-19-norpregna-4,6-diène-3,20-dione ;
- 17-(Acétyloxy)-9,11α-dihydro-6α-méthyl-6'H-benzo-[10,9,11]-19-norpregn-4-ène-3,20-dione ;
- 17-(Acétyloxy)-6-chloro-9,11α-dihydro-6'H-benzo-[10,9,11]-19-norpregna-4,6-diène-3,20-dione ;
- 17-(Acétyloxy)-6-chloro-1β,2β,9,11α-tétrahydro-3'H-cyclopropa[1,2][6"H]benzo[10,9,11]-19-norpregna-4,6-diène-3,20-dione ;
- 17-(Acétyloxy)-4',5',9,11α-tétrahydro-6'H-benzo-[10,9,11]-19-norpregn-4-ène-3,20-dione ;
- 17-(Acétyloxy)-6-méthyl-4',5',9,11α-tétrahydro-6'H-benzo-[10,9,11]-19-norpregna-4,6-diène-3,20-dione ;
- 17-(Acétyloxy)-6-chloro-1β,2β,4",5",9,11α-hexahydro-3'H-cyclopropa[1,2][6"H]benzo[10,9,11]-19-norpregna-4,6-diène-3,20-dione ;
- 17-(Acétyloxy)-5',6'-dihydro-9H-benzo[10,9,11]-19-norpregn-4-ène-3,20-dione ;
- 17-(Acétyloxy)-5',6'-dihydro-6-méthyl-9H-benzo[10,9,11]-19-norpregna-4,6-diène-3,20-dione ;
- 17-(Acétyloxy)-9,11α-dihydro-6'H-benzo[10,9,11]-18a-homo-19-norpregn-4-ène-3,20-dione ;
- 17-(Acétyloxy)-9,11α-dihydro-6-méthyl-6'H-benzo-[10,9,11]-18a-homo-19-norpregna-4,6-diène-3,20-dione ;
- 9,11α-Dihydro-17-méthyl-6'H-benzo[10,9,11]-19-norpregn-4-ène-3,20-dione ;
- 3',9,11α-Tétrahydrocyclopropa[16,17][6H]benzo-[10,9,11]-19-norpregn-4-ène-3,20-dione ;
- 9,11α-Dihydro-17-méthyl-6'H-benzo[10,9,11]-18a-homo-19-norpregn-4-ène-3,20-dione ;
- 3',9,11α,16β-Tétrahydrocyclopropa[16,17][6H]benzo-[10,9,11]-18a-homo-19-norpregn-4-ène-3,20-dione ;
- 9,11α-Dihydro-17β-hydroxy-17α-méthyl-6'H-benzo-[10,9,11]estr-4-èn-3-one ;
- 9,11α-Dihydro-17α-éthynyl-17β-hydroxy-6'H-benzo-[10,9,11]estr-4-èn-3-one ;
- 9,11α-Dihydro-17β-hydroxy-17α-(1-propynyl)-6'H-benzo-[10,9,11]estr-4-èn-3-one ;
- 9,11α-Dihydro-17β-hydroxy-17α-(1,3-pentadiynyl)-6'H-benzo[10,9,11]estr-4-èn-3-one ;
- (Z)-9,11α-Dihydro-17β-hydroxy-17α-(3-hydroxy-1-propényl)-6'H-benzo[10,9,11]estr-4-èn-3-one ;
- 9,11α-Dihydro-17β-hydroxy-17α-(3-hydroxypropyl)-6'H-benzo[10,9,11]estr-4-èn-3-one ;
- 17β-Hydroxy-17α-méthyl-4',5',9,11α-tétrahydro-6'H-benzo[10,9,11]estr-4-èn-3-one;
- 17α-Ethynyl-17β-hydroxy-4',5',9,11α-tétrahydro-6'H-benzo[10,9,11]estr-4-èn-3-one;
- 17β-Hydroxy-17α-(1-propynyl)-4',5',9,11α-tétrahydro-6'H-benzo[10,9,11]estr-4-èn-3-one;
- 5',6'-Dihydro-17α-éthynyl-17β-hydroxy-9H-benzo-[10,9,11]estr-4-èn-3-one ;
- 5',6'-Dihydro-17β-hydroxy-17α-(1-propynyl)-9H-benzo-[10,9,11]estr-4-èn-3-one ;
- 9,11α-Dihydro-17α-éthynyl-17β-hydroxy-6'H-benzo-[10,9,11]-18a-homoestr-4-èn-3-one;
- 9,11α-Dihydro-17β-hydroxy-17α-(1-propynyl)-6'H-benzo-[10,9,11]-18a-homoestr-4-èn-3-one;
- 17α-(1-Butynyl)-9,11α-dihydro-17β-hydroxy-6'H-benzo-[10,9,11]-18a-homoestr-4-èn-3-one ;
- 9,11α-Dihydro-17α-(1,2-propadiényl)-17β-hydroxy-6'H-benzo[10,9,11]-18a-homoestr-4-èn-3-one ;
- 17α-Ethynyl-17β-hydroxy-4',5',9,11α-tétrahydro-6'H-benzo[10,9,11]-18a-homoestr-4-èn-3-one ;
- 17β-Hydroxy-17α-(1-propynyl)-4',5',9,11α-tétrahydro-6'H-benzo[10,9,11]-18a-homoestr-4-èn-3-one ;
- 5',6'-Dihydro-17α-éthynyl-17β-hydroxy-9H-benzo-[10,9,11]-18a-homoestr-4-èn-3-one ;
- 5',6'-Dihydro-17β-hydroxy-17α-(1-propynyl)-9H-benzo-[10,9,11]-18a-homoestr-4-èn-3-one ;
- 9,11α-Dihydro-17α-éthynyl-17β-hydroxy-6'H-benzo-[10,9,11]estra-4,15-dièn-3-one;
- 9,11α-Dihydro-17β-hydroxy-17α-(1-propynyl)-6'H-benzo-[10,9,11]estra-4,15-dièn-3-one ;
- 17α-Ethynyl-17β-hydroxy-4',5',9,11α-tétrahydro-6'H-benzo[10,9,11]estra-4,15-dièn-3-one;
- 5',6'-Dihydro-17α-éthynyl-17β-hydroxy-9H-benzo-[10,9,11]estra-4,15-dièn-3-one ;
- 5',6'-Dihydro-17β-hydroxy-17α-(1-propynyl)-9H-benzo-[10,9,11]estra-4,15-dièn-3-one ;
- 9,11α-Dihydro-17α-éthynyl-17β-hydroxy-6'H-benzo-[10,9,11]-18a-homoestra-4,15-dièn-3-one ;
- 9,11α-Dihydro-17β-hydroxy-17α-(1-propynyl)-6'H-benzo-[10,9,11]-18a-homoestra-4,15-dièn-3-one ;
- 17α-Ethynyl-17β-hydroxy-4',5',9,11α-tétrahydro-6'H-benzo[10,9,11]-18a-homoestra-4,15-dièn-3-one ;
- 5',6'-Dihydro-17α-éthynyl-17β-hydroxy-9H-benzo-[10,9,11]-18a-homoestra-4,15-dièn-3-one ;
- 5',6'-Dihydro-17β-hydroxy-17α-(1-propynyl)-9H-benzo-[10,9,11]-18a-homoestra-4,15-dièn-3-one ;
- 4",5",9,11α-Tétrahydrospiro[6'H-benzo[10,9,11]estr-4-ène-17β,2"(3"H)-furanne]-3-one ;
- 3",4",9,11α-Tétrahydrospiro[6"H-benzo[10,9,11]estr-4-ène-17β,2"(5"H)-furanne]-3,5"-dione ;
- 3"",4"",6α,7α,9,11α,15α,16α-Octahydrospiro[3'H,3"H-dicyclopropa[6,7:15,16]-[6H]benzo[10,9,11]estr-4-ène-17β,2""(5""H)-furanne]-3,5""-dione ;
- 3"",4"",9',11'α,15'α,16'α-Hexahydrospiro[cyclopropan-1,6'-[3H]cyclopropa[15,16][6H]benzo[10,9,11]estr-4-ène-17'β,2""(5""H)-furanne]-3',5""-dione ;
- 3"",4"",9',11'α,15'α,16'α-Hexahydrospiro[cyclopropan-1,6'-[3H]cyclopropa[15,16][6H]benzo[10,9,11]estra-1,4-diène-17'β,2""(5""H)-furanne]-3',5""-dione ;
- 3"",4"',4"",5"',6α,7α,9,11α,15α,16α-Décahydrospiro-[3'H,3"H]dicyclopropa[6,7:15,16][6H]benzo-[10,9,11]estr-4-ène-17β,2""(5""H)-furanne]-3,5""-dione ;
- 3",4",9,11α-Tétrahydrospiro[6'H-benzo[10,9,11]-18a-homoestr-4-ène-17β,2"(5")-furanne]-3,5"-dione ;
- 3"",4"",6α,7α,9,11α,15α,16α-Octahydrospiro[3'H,3"H-dicyclopropa[6,7:15,16][6H]benzo[10,9,11]-18a-homoestr-4-ène-17β,2""(5""H)-furanne]-3,5""-dione ;
- 9,11α-Dihydro-17α-éthynyl-6'H-benzo[10,9,11]estr-4-èn-17β-ol ;
- 17α-Ethynyl-4',5',9,11α-tétrahydro-6'H-benzo[10,9,11]-estr-4-èn-17β-ol ;
- 5',6'-Dihydro-17α-éthynyl-9H-benzo[10,9,11]-estr-4-èn-17β-ol ;
- 9,11α-Dihydro-17α-éthynyl-6'H-benzo[10,9,11]estra-4,15-dièn-17β-ol ;
- 9,11α-Dihydro-17α-éthynyl-6'H-benzo[10,9,11]-18a-homoestr-4-èn-17β-ol ;
- 9,11α-Dihydro-17α-éthynyl-6'H-benzo[10,9,11]-18a-homoestra-4,15-dièn-17β-ol.

11. Procédé pour la préparation des composés de formule générale I selon la revendication 1, caractérisé en ce que l'on transforme un composé de formule générale II 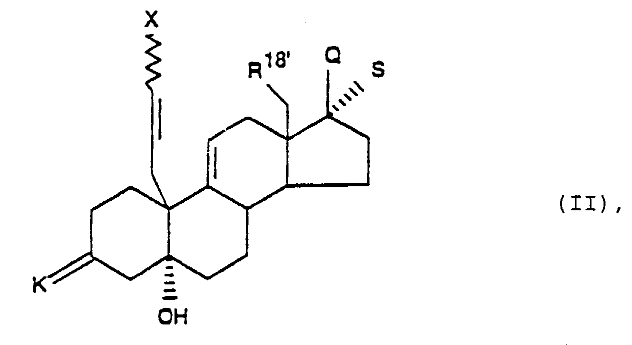 dans laquelle
K représente un groupe protecteur de céto ou un groupe hydroxy protégé et un atome d'hydrogène,
X représente un atome de chlore ou de brome en position syn ou anti,
R¹⁸ représente un atome d'hydrogène ou un groupe méthyle ainsi que
Q représente un groupe hydroxy en position β et S représente un atome d'hydrogène en position α, ou Q et S ensemble représentent un atome d'oxygène de céto ou, en outre,
Q et S représentent une combinaison de substituants R^{17β}/R^{17α} cités dans la formule I, y compris les composés spiro, les groupes hydroxy et/ou les groupes céto qui se trouvant dans ces composés sont éventuellement protégés,
a) par cyclisation radicalaire en un composé de formule générale III 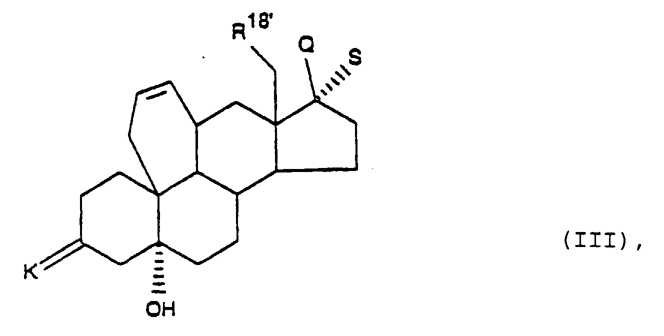 dans laquelle
K, R¹⁸ ainsi que Q et S ont les significations données dans la formule II,
b) ensuite, lorsque Q représente un groupe hydroxy, on oxyde éventuellement celui-ci,
c) lorsque R^{11,} R^{11'} et R¹⁹ doivent être finalement des atomes d'hydrogène, on hydrogène le pont 19,11β-éthéno,
d) lorsque R¹⁹ doit être finalement un atome d'hydrogène ainsi que R¹¹ et R^{11'} ensemble doivent représenter une liaison supplémentaire, on isomérise la double liaison dans le pont 19,11β-éthéno en la position 11 (en position exo),
e) si on le souhaite, on introduit dans le cycle D une double liaison 15,16 et
f) si on le souhaite, on isomérise celle-ci en la position 14,15 ou
g) par méthylénation, en la liaison 15β-16β méthylène correspondante, ainsi que
h) lorsque Q et S ensemble représentent un atome d'oxygène cétonique, par addition nucléophile du substituant R^{17α} ou d'un précurseur réactif de R^{17α} et éventuellement par éthérification ou estérification du groupe 17β-hydroxy avec un réactif correspondant donnant le radical R²¹ ou en édifiant la configuration de substitution 17α-hydroxy-17β-alcanoyle et éventuellement en éthérifiant ou en estérifiant le groupe 17α-hydroxy par un réactif donnant le radical R²³ correspondant,
i) éventuellement par hydrogénation partielle ou complète d'une chaîne latérale en C₁₇ insaturée et
j) si on le souhaite, par oxydation du composé de 17-(3-hydroxypropyle), respectivement de 17-(4-hydroxybutyle) correspondant pour former la 17-spirolactone ou
k) si on le souhaite par cyclisation du composé (Z)-17α-(3-hydroxyprop-1-ényl)-, respectivement (Z)-17α-(4-hydroxybut-1-ényl)-17β-hydroxy ou des composés saturés dans la chaîne latérale correspondante, pour former le spiroéther et
l) par traitement par l'acide dans un solvant miscible à l'eau en le système Δ⁴-3-céto en séparant éventuellement également d'autres groupes protecteurs présents et ainsi en transformant en un composé de formule générale I et éventuellement en transformant ce composé de formule générale I,
m) par introduction d'une double liaison en 1,2 et/ou en 6,7 et éventuellement par méthylénation de l'une ou des deux doubles liaisons,
n) par introduction d'un radical alkyle en position α ou β, linéaire ou ramifié, ou d'un groupe thio -SR²⁰ en position 7,
o) par époxydation de la double liaison 6,7 et par ouverture de l'époxyde par un halogénure d'hydrogène (Hal = F, Cl, Br, I) et par élimination du groupe 7α-hydroxy formé,
p) par 6α-hydroxyméthylation et ensuite par séparation de l'hydrogène en composé de 6-méthylène,
q) par isomérisation de la double liaison en position exo du groupe 6-méthylène ou par introduction directe d'un groupe 6-alkyle (6-alkyl-4,6-dién-3-one),
r) par hydrogénation du groupe 6-méthylène en un composé de formule générale I dans lequel
R¹, R², R¹⁴, R¹⁵, R¹⁶, R^{17α} , R^{17β}, R¹⁸, R¹¹ et R¹⁹ ont les significations souhaitées finalement,
et R^{6a} représente un groupe α-méthyle et R^{6b} et R⁷ chacun représentent un atome d'hydrogène ou ensemble forment une liaison supplémentaire,
ou
s) lorsque R^{6a} doit finalement être un radical alkyle en position α ou β, linéaire ou ramifié, saturé, comportant jusqu'à 4 atomes de carbone,
on transforme par cétalisation en isomérisant simultanément la double liaison 4(5) après 5(6), époxydation de la double liaison 5(6) et ouverture nucléophile de l'époxyde 5,6α avec le groupe 3-céto protégé avec un composé d'alkyllithium ou un halogénure d'alkylmagnésium linéaire ou ramifié saturé comportant jusqu'à 4 atomes de carbone dans le radical alkyle et clivage du groupe 3-céto dans le composé de 5α-hydroxy-6β-alkyle sous conditions modérément acides pour obtenir le composé 3-céto-5α-hydroxy-6β-alkyle correspondant et élimination en milieu basique du groupe 5α-hydroxy en le composé correspondant 3-céto-4-ène de formule générale I avec le groupe 6-alkyle en position β ou par séparation du groupe protecteur de 3-céto sous conditions drastiques en le composé 3-céto-4-ène correspondant de formule générale I avec le groupe 6-alkyle en position α et
t) on transforme éventuellement un des composés 3-céto obtenus ci-dessus par le chlorhydrate d'hydroxylamine en présence d'amines tertiaires, à une température comprise entre -20 °C et +40 °C, en le composé 3-hydroxyimino (W = >N~OH;~ représente un groupe OH en position syn ou anti) ou
u) éventuellement en le composé 3-thiocétal, de préférence le 3-(1',3'-éthylènedithio)-cétal et celui-ci par réduction en le composé de formule générale I dans laquelle W représente deux atomes d'hydrogène.

12. Préparations pharmaceutiques caractérisées en ce qu'elles contiennent au moins un composé de formule générale I selon la revendication 1, ainsi qu'un véhicule pharmaceutiquement toléré.

13. Utilisation des composés de formule générale I selon la revendication 1 pour la préparation de médicaments.

14. Composés intermédiaires de formule générale III' 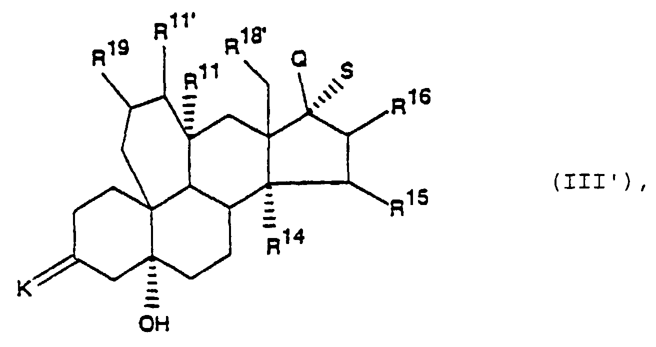 dans laquelle
K représente un groupe protecteur de céto ou un groupe hydroxy protégé et un atome d'hydrogène,
R^{11'} et R¹⁹ ensemble représentent une liaison supplémentaire et R¹¹ représente un atome d'hydrogène en position α ou R¹⁹ représente un atome d'hydrogène et R^{11'} et R¹¹ ensemble représentent une liaison supplémentaire ou R¹¹, R^{11'} et R¹⁹ représentent chacun un atome d'hydrogène,
R¹⁴, R¹⁵ et R¹⁶ ont la signification des substituants donnés dans la formule générale I,
R¹⁸ représente un atome d'hydrogène ou un groupe méthyle ainsi que
Q représente un groupe hydroxy en position β et S représente un atome d'hydrogène en position α, ou Q et S ensemble représentent l'atome d'oxygène cétonique ou en outre
Q et S représentent des combinaisons de substituants R^{17β}/R^{17α} citées dans la formule I, y compris les composés spiro, les groupes hydroxy et/ou les groupes céto présents dans ces composés étant éventuellement protégés.
